# EUROPEAN PATENT SPECIFICATION

(11) **EP 2 137 535 B1**
(45) Date of publication and mention of the grant of the patent: **03.06.2015**
(21) Application number: 08742858.7
(22) Date of filing: 11.04.2008
(51) Int. Cl.: G01N 33/574

(54) **RECEPTOR TYROSINE KINASE PROFILING**
PROFILERSTELLUNG VON REZEPTOR-TYROSINKINASEN
ÉTABLISSEMENT DU PROFIL DE RÉCEPTEURS TYROSINE KINASES

(30) Priority: 13.04.2007 US 923455 P; 13.09.2007 US 993773 P
(43) Date of publication of application: 30.12.2009
(73) Proprietor: Dana-Farber Cancer Institute, Inc., Boston, MA 02215 (US)
(72) Inventor: DEPINHO, Ronald A., Houston, TX 77019 (US); STOMMEL, Jayne M., Rockville, MD 20852 (US)
(74) Representative: Vossius & Partner Patentanwälte Rechtsanwälte mbB
(86) International application number: PCT/US2008/004801
(87) International publication number: WO 2008/127707

(56) References cited:
- FAIVRE SANDRINE ET AL: "New paradigms in anticancer therapy: Targeting multiple signaling pathways with kinase inhibitors" SEMINARS IN ONCOLOGY, vol. 33, no. 4, August 2006 (2006-08), pages 407-420, XP009101633 ISSN: 0093-7754
- PATYNA SHEM ET AL: "SU14813: a novel multiple receptor tyrosine kinase inhibitor with potent antiangiogenic and antitumor activity" MOLECULAR CANCER THERAPEUTICS, vol. 5, no. 7, July 2006 (2006-07), pages 1774-1782, XP002484745 ISSN: 1535-7163
- POTAPOVA OLGA ET AL: "Contribution of individual targets to the antitumor efficacy of the multitargeted receptor tyrosine kinase inhibitor SU11248" MOLECULAR CANCER THERAPEUTICS, vol. 5, no. 5, May 2006 (2006-05), pages 1280-1289, XP002484744 ISSN: 1535-7163
- STOMMEL JAYNE M ET AL: "Coactivation of receptor tyrosine kinases affects the response of tumor cells to targeted therapies" SCIENCE (WASHINGTON D C), vol. 318, no. 5848, October 2007 (2007-10), pages 287-290, XP002484746 ISSN: 0036-8075

## Description

### RELATED APPLICATIONS

This application claims the benefit of U.S. Provisional Application Nos. 60/923,455, filed April 13, 2007, and 60/993,773, filed September 13, 2007.

### STATEMENT REGARDING FEDERALLY-SPONSORED RESEARCH OR DEVELOPMENT

Work described herein was funded, in whole or in part, by National Institutes of Health/NCI Grant Number P01CA95616. The United States government has certain rights in the invention.

### BACKGROUND OF THE INVENTION

The PI3K/AKT signaling pathway is frequently hyperactivated by a variety of mechanisms in a wide range of human cancers, including melanoma, breast, gliomal, lung, prostate, and ovarian tumors (see Vivanco I and Sawyers CL (2002) Nat Rev Cancer. 2(7):489-501; Scheid M P and Woodgett J R (2001) J Mammary Gland Biol Neoplasia. 6(1):83-99). In normal phosphoinositide metabolism, phosphatidylinositol (3, 4) bisphosphate (PIP₂) is phosphorylated by phosphatidylinositol 3-kinase (PI3K) to generate PIP₃, and PIP₃ is dephosphorylated back to PIP₂ by the lipid phosphatase PTEN (Phosphatase and Tensin homolog). In tumor cells, PIP³ levels may be elevated e.g., by mutation or deletion of PTEN, by amplification or overexpression of PI3K, or by activation of receptor tyrosine kinases which in turn activate PI3K. Increased production of (PIP₃) activates AKT (protein kinase B) by recruitment to the plasma membrane. The AKT pathway promotes tumor progression by enhancing cell proliferation, growth, survival, and motility, and by suppressing apoptosis. As activation of receptor tyrosine kinases (RTKs) and the downstream phosphatidylinositol 3-kinase (PI3K) signaling pathway is central to cancer development, their inhibition has emerged as an effective treatment strategy for certain human malignancies.

RTKs have a conserved domain structure including an extracellular domain, a membrane-spanning (transmembrane) domain and an intracellular tyrosine kinase domain. The extracellular domain can bind to a ligand, such as to a polypeptide growth factor or to a cell membrane-associated molecule. Typically, dimerization of RTKs activates the intracellular catalytic tyrosine kinase domain of the receptor and subsequent signal transduction. RTKs can be homodimers or heterodimers. Many RTKs are capable of autophosphorylation when dimerized, such as by transphosphorylation between subunits. Autophosphorylation in the kinase domain maintains the tyrosine kinase domain in an activated state. Autophosphorylation in other regions of the RTK can influence its interaction with other cellular proteins.

Examples of RTKs include, but are not limited to ERBB receptors (e.g., EGFR, ERBB2, ERBB3, ERBB4), erythropoietin-producing hepatocellular (EPH) receptors (e.g., EphA1, EphA2, EphA3, EphA4, EphA5, EphA6, EphA7, EphA8, EphB1, EphB2, EphB3, EphB4, EphB5, EphB6), fibroblast growth factor (FGF) receptors (e.g., FGFR1, FGFR2, FGFR3, FGFR4, FGFR5), platelet-derived growth factor (PDGF) receptors (e.g., PDGFR-A, PDGFR-B), vascular endothelial growth factor (VEGF) receptors (e.g., VEGFR1/FLT1, VEGFR2/FLK1, VEGF3), tyrosine kinase with immunoglobulin-like and EGF-like domains (TIE) receptors (e.g., TIE-1, TIE-2/TEK), insulin-like growth factor (IGF) receptors (e.g., INS-R, IGF-1R, IR-R), Discoidin Domain (DD) receptors (e.g., DDR1, DDR2), receptor for c-Met (MET), recepteur d'origine nantais (RON); also known as macrophage stimulating 1 receptor, Flt3 fins-related tyrosine kinase 3 (Flt3), colony stimulating factor 1(CSF1) receptor, adhesion related kinase receptor (e.g., Axl), receptor for c-kit (KIT) and insulin receptor related (IRR) receptors.

The phosphatidyl inositol 3'-kinases (PI3K, PI3 kinase) act as downstream effectors of RTKs, are recruited upon receptor stimulation and mediate the activation of second messenger signaling pathways through the production of phosphorylated derivatives of inositol (reviewed in Fry, Biochim. Biophys. Acta., 1226:237-268, 1994).

The PI3K heterodimers consist of a 110 kD (p110) catalytic subunit associated with an 85 kD (p85) regulatory subunit, and it is through the SH2 domains of the p85 regulatory subunit that the enzyme associates with membrane-bound receptors (Escobedo et al., Cell 65:75-82, 1991; Skolnik et al., Cell 65:83-90, 1991). The p85 adapter subunit has two SH2 domains that allow PI3K to associate with RTKs, and are thereby critical to activate the enzyme. Once PI3K is activated, it generates lipid products that act to stimulate many different cellular pathways. The cellular effects observed upon PI3K activation are the result of downstream targets of this enzyme. For example, AKT, and the related kinases protein kinases A and C (PKA and PKC) are activated by two phosphorylation events catalyzed by the phosphoinositide dependent kinase PDK1, an enzyme that is activated by PI3K.

Activation of PTEN sensitizes cells to p53 mediated cell death through the control of p53 induced apoptosis, while mutations in PTEN are associated with malignant and invasive tumor progression. PTEN mutations have been isolated from several cancerous solid tumors and cell lines including brain, breast, prostate, ovary, skin, thyroid, lung, bladder and colon (Teng et al., Cancer Res., 1997, 57, 5221-5225) and have led to the classification of PTEN as a tumor suppressor gene.

Although greater than 80% of glioblastomas express high levels of phosphorylated AKT (H. Wang et al., Lab Invest 84, 941 (2004)), indicating PI3K activation, targeted therapies against RTK have elicited only modest and non-durable responses. In gliobastoma multiforme (GBM), EGFR activation is a critical pathogenetic event with amplification, mutation and rearrangement observed in more than 40% of the cases, making it a compelling molecular target for therapeutic inhibition (A. El-Obeid et al., Cancer Res 57, 5598 (1997) and M. Nagane et al., Cancer Res 56, 5079 (1996)). A small proportion of the remaining GBMs are shown to harbor PDGFRα, PDGFRβ and/or MET alterations. The observation that co-expression of EGFR activating mutants and PTEN in GBM cells is correlated statistically with clinical response to EGFR kinase inhibitors indicates that PTEN is a response biomarker for anti-EGFR therapy, and that its loss (which occurs minimally in 40-50% of GBM cases (J. Li et al., Science 275, 1943 (1997)) dissociates the inhibition of EGFR from downstream PI3K pathway inhibition, rendering targeted EGFR therapy ineffective (I. K. Mellinghoff et al., N Engl J Med 353, 2012 (2005)). Treatment of GBM patients with anti-PDGFR therapy has previously been shown to fail (P. Y. Wen et al., Clin Cancer Res 12, 4899 (2006)) and only 10-20% of patients appear to benefit from EGFR targeted inhibition (J. N. Rich et al., J Clin Oncol 22, 133 (2004)).

Clearly, there exists a need of developing improved cancer treatment methods, in particular for cancers with increased PI3K signaling. The application provides such improved methods to profile aberrant signaling in cancers, enabling the rational design of therapeutic agents.

### SUMMARY OF THE INVENTION

The invention relates to the embodiments as characterized in the claims.

One aspect of the invention provides for a method of designing a first-line therapeutic treatment for a subject afflicted with cancer, the method comprising i) determining the presence of 10 or more indicators of receptor tyrosine kinase (RTK) activation in a biological sample from the subject; and ii) selecting a group of one or more RTK inhibitors, wherein the group can inhibit the activity of at least two RTKs that display one or more of the indicators.

Also described herein is a method of designing a first-line therapeutic treatment for a subject afflicted with cancer, the method comprising i) obtaining a biological sample of a subject; ii) determining the presence of one or more indicators of receptor tyrosine kinase (RTK) activation in a biological sample; iii) selecting a group of one or more RTK inhibitors, wherein the group can inhibit the activity of at least two RTKs that display one or more of the indicators; and iv) transmitting a descriptor of the group of one or more RTK inhibitors, thereby designing a therapeutic treatment for the subject. In certain embodiments, a description of the therapy is transmitted across a network.

The group of one or more RTK inhibitors can inhibit the activity of at least 3, 4, 5, 6, 7, 8, 9, 10, or more RTKs that display one or more of the indicators.

Further described is a method of treating a subject afflicted with cancer is provided, the method comprising i) determining the presence of one or more indicators of receptor tyrosine kinase (RTK) activation in a biological sample from the subject; and ii) administering a therapeutically-effective amount of a group of one or more RTK inhibitors to the subject, wherein the group inhibits the activity of at least two RTKs that display one or more of the indicators. One or more DNA damaging agents may be conjointly administered with one or more RTK inhibitors.

One or more DNA damaging agents may be selected from radiation, a chemotherapeutic, cyclophosphamide, melphalan, busulfan, chlorambucil, mitomycin, cisplatin, bleomycin, irinotecan, mitoxantrone, dactinomycin, temozolomide, or a combination thereof. The one or more RTK inhibitors and the one or more DNA damaging agents can be administered simultaneously or sequentially, wherein the one or more RTK inhibitors are administered first, followed by the one or more DNA damaging agents.

Further described are methods for evaluating a candidate receptor tyrosine kinase (RTK) inhibitor, the methods comprising i) determining the presence of one or more indicators of receptor tyrosine kinase (RTK) activation in a biological sample in a population of subjects; ii) selecting subjects having similar RTK activation profiles; and iii) administering to at least one of the selected subjects (a) the candidate RTK inhibitor ; and (b) at least one additional RTK inhibitor that targets one or more RTKs that display one or more of the indicators in the selected population. The subjects in the population may be afflicted with cancer. The subjects in the population may be afflicted with the same type of cancer.

The method may comprises i) determining the presence of one or more indicators of receptor tyrosine kinase (RTK) activation in a biological sample in a population of subjects; ii) selecting subjects that are more likely to be sensitive to the candidate RTK inhibitor based on the RTK activation profile; and iii) administering the candidate RTK inhibitor to one or more of the selected subjects in combination with at least one additional RTK inhibitor that targets one or more RTKs that display one or more of the indicators in the selected subjects.

The method may comprises i) determining the presence of one or more indicators of receptor tyrosine kinase (RTK) activation in a biological sample in a population of subjects; ii) administering the candidate RTK inhibitor to one of more subjects from the population of subjects; and iii) correlating the effectiveness of the candidate RTK inhibitor with an RTK activation profile for the one of more subjects from the population of subjects.

The group of one or more RTK inhibitors may inhibits the activity of at least 3, 4, 5, 6, 7, 8, 9, 10, or more RTKs that display one or more of the indicators.

In certain embodiments of methods of the invention, the subject is a human.

In certain embodiments of methods of the invention, subject is afflicted with at least one of the following types of cancer: acral lentiginous melanoma, actinic keratoses, adenocarcinoma, adenoid cycstic carcinoma, adenomas, adenosarcoma, adenosquamous carcinoma, adrenocortical carcinoma, AIDS-related lymphoma, anal cancer, anaplastic glioma, astrocytic tumors, astrocytomas, bartholin gland carcinoma, basal cell carcinoma, biliary tract cancer, bone cancer, bile duct cancer, bladder cancer, brain stem glioma, brain tumors, breast cancer, bronchial gland carcinomas, capillary carcinoma, carcinoids, carcinoma, carcinosarcoma, cavernous, central nervous system lymphoma, cerebral astrocytoma, cervical cancer, connective tissue cancer, cholangiocarcinoma, chondosarcoma, choriod plexus papilloma/carcinoma, clear cell carcinoma, colon cancer, colorectal cancer, cutaneous T-cell lymphoma, cystadenoma, endodermal sinus tumor, endometrial hyperplasia, endometrial stromal sarcoma, endometrioid adenocarcinoma, ependymal, ependymoma, epitheloid, esophageal cancer, Ewing's sarcoma, extragonadal germ cell tumor, eye cancer, fibrolamellar, focal nodular hyperplasia, gallbladder cancer, gangliogliomas , gastric cancer, gastrinoma, germ cell tumors, gestational trophoblastic tumor, glioblastoma multiforme, glioma, glucagonoma, head and neck cancer, hemangiblastomas, hemangioendothelioma, hemangiomas, hepatic adenoma, hepatic adenomatosis, hepatocellular carcinoma, Hodgkin's lymphoma, hypopharyngeal cancer, hypothalamic and visual pathway glioma, childhood, insulinoma, intaepithelial neoplasia, interepithelial squamous cell neoplasia, intraocular melanoma, intra-epithelial neoplasm, invasive squamous cell carcinoma, large cell carcinoma, islet cell carcinoma, Kaposi's sarcoma, kidney cancer, laryngeal cancer, leiomyosarcoma, lentigo maligna melanomas, leukemia-related disorders, lip and oral cavity cancer, liver cancer, lung cancer, lymphoma, malignant mesothelial tumors, malignant thymoma, medulloblastoma, medulloepithelioma, melanoma, meningeal, merkel cell carcinoma, mesothelial, metastatic carcinoma, mucoepidermoid carcinoma, multiple myeloma/plasma cell neoplasm, mycosis fungoides, myelodysplastic syndrome, myeloproliferative disorders, nasal cavity and paranasal sinus cancer, nasopharyngeal cancer, neuroblastoma, neurofibromatosis, neuroepithelial adenocarcinoma nodular melanoma, non-Hodgkin's lymphoma, non-small cell lung cancer, oat cell carcinoma, oligodendroglial, oligoastrocytomas, oral cancer, oropharyngeal cancer, osteosarcoma, pancreatic polypeptide, ovarian cancer, ovarian germ cell tumor, pancreatic cancer, papillary serous adenocarcinoma, pineal cell, pituitary tumors, plasmacytoma, pseudosarcoma, pulmonary blastoma, parathyroid cancer, penile cancer, pheochromocytoma, pineal and supratentorial primitive neuroectodermal tumors, pituitary tumor, plasma cell neoplasm, pleuropulmonary blastoma, prostate cancer, rectal cancer, renal cell carcinoma, cancer of the respiratory system, retinoblastoma, rhabdomyosarcoma, sarcoma, serous carcinoma, skin cancer, small cell carcinoma, small intestine cancer, soft tissue carcinomas, somatostatin-secreting tumor, squamous carcinoma, squamous cell carcinoma, stomach cancer, stromal tumors, submesothelial, superficial spreading melanoma, supratentorial primitive neuroectodermal tumors, testicular cancer, thyroid cancer, undifferentiatied carcinoma, urethral cancer, uterine sarcoma, uveal melanoma, verrucous carcinoma, vaginal cancer, vipoma, vulvar cancer, Waldenstrom's macroglobulinemia, well differentiated carcinoma, or Wilm's tumor. In certain embodiments, the subject is afflicted with a glioblastoma.

Also described herein is a method for reducing PI3K-mediated signaling in a cancer cell, the method comprising the steps of i) determining the presence of one or more indicators of receptor tyrosine kinase (RTK) activation in the cancer cell; and ii) contacting the cancer cell with a group of one or more RTK inhibitors, wherein the group inhibits the activity of at least two RTKs that display one or more of the indicators.

Described herein is also a method for reducing AKT phosphorylation in a cancer cell comprising the steps of i) determining the presence of one or more indicators of receptor tyrosine kinase (RTK) activation in the cancer cell; and ii) contacting the cancer cell with a group of one or more RTK inhibitors, wherein the group inhibits the activity of at least two RTKs that display one or more of the indicators.

Further described herein is a method for reducing cancer cell proliferation comprising the steps of i) determining the presence of one or more indicators of receptor tyrosine kinase (RTK) activation in the cancer cell; and ii) contacting the cancer cell with a group of one or more RTK inhibitors, wherein the group inhibits the activity of at least two RTKs that display one or more of the indicators.

Also described is a method for increasing cell death in a cancer cell comprising the steps of i) determining the presence of one or more indicators of receptor tyrosine kinase (RTK) activation in the cancer cell; and ii) contacting the cancer cell with a group of one or more RTK inhibitors, wherein the group inhibits the activity of at least two RTKs that display one or more of the indicators.

Also described is a method for reducing tumor maintenance or progression comprising the steps of i) determining the presence of one or more indicators of receptor tyrosine kinase (RTK) activation in the cancer cell; and ii) contacting the cancer cell with a group of one or more RTK inhibitors, wherein the group inhibits the activity of at least two RTKs that display one or more of the indicators.

Further described is a method for identifying an RTK inhibitor comprising the steps of i) contacting a cancer cell with one or more known RTK inhibitors and with a candidate RTK inhibitor, and ii) detecting one or more of the following: cell death, reduced cell growth, reduced RTK phosphorylation, reduced AKT phosphorylation, or reduced S6 phosphorylation in said cell compared to in a cancer cell contacted with only the one or more known RTK inhibitors.

The cancer cell may be a mammalian cell, in particular a human cancer cell.

The cancer cell may be a cell line. The cancer cell may be from a primary tissue sample.

The cancer cell may be contacted ex vivo with candidate or known RTK inhibitors. The cancer cell may be contacted in vivo with candidate or known RTK inhibitors.

The cancer cell may be selected from a lung cancer cell, a brain cancer cell, a breast cancer cell, a head and neck cancer cell, a colon cancer cell, prostate cancer cell, colon cancer cell, pancreatic cancer cell, hepatic cancer cell, testicular cancer cell, ovarian cancer cell, cervical cancer cell, rectal cancer cell, thyroid cancer cell, uterine cancer cell, vaginal cancer cell, glioma cancer cell and skin cancer cell.

The cancer cell may comprises an activating mutation in an EGFR gene. The activating mutation may be an EGFRvIII mutation. The EGFR gene may be amplified in the cancer cell.

EGFR gene amplification may be at least 2-, 3-, 4-, 5-, 6-, 7-, 8-, 9-, 10-fold or more.

PTEN may be disabled in said cancer cell. In certain

No detectable levels of PTEN protein may be present in said cancer cell.

One or more RTK inhibitors may be delivered to the cancer cell via a virus. The virus may be an adeno-associated virus (AAV).

In certain embodiments, the group of one or more RTK inhibitors inhibits the activity of at least 3, 4, 5, 6, 7, 8, 9, 10, or more RTKs that display one or more of the indicators.

In certain embodiments of the methods, the presence of one or more indicators of RTK activation is determined for two or more of the following RTKs: ALK, AXL, CSF1 R, DDR1, DDR2, EGFR, EPHA1, EPHA10, EPHA2, EPHA3, EPHA4, EPHA5, EPHA6, EPHA7, EPHA8, EPHB1, EPHB2, EPHB3, EPHB4, EPHB6, ERBB2, ERBB3, ERBB4, FGFR1, FGFR2, FGFR3, FGFR4, FLT1, FLT3, FLT4, IGF1R, INSR, INSRR, KDR, LTK, MERTK, MET, MUSK, NTRK1, NTRK2, NTRK3, PDGFRA, PDGFRB, RET, RON, ROR1, ROR2, ROS1, RYK, TIE 1, TYRO3 or KIT.

In certain embodiments of the methods, one or more indicators of MET activation and one or more indicators of EGFR activation are determined.

In certain embodiments of the methods, the presence of one or more indicators of RTK activation is determined using an array, real-time PCR, fluorescence in situ hybridization, RT-PCR, nuclease protection assay, northern blot, nucleotide sequencing, immunohistochemistry or immunocytochemistry with phosphorylation state-specific or total-protein detection antibodies, or a combination thereof. In certain embodiments, the array is an anti-phospho-RTK antibody array. In certain embodiments, the array is an anti-RTK array and an anti-phosphotyrosine antibody is used to detect RTK phosphorylation.

In certain embodiments of the methods, at least one of the RTK inhibitors is a small molecule therapeutic, a protein therapeutic, or a nucleic acid therapeutic.

In certain embodiments of the methods, one or more RTK inhibitors is an anti-RTK antibody. In certain embodiments, one or more RTK inhibitors in panitumumab, cetuximab, bevacizumab, or trastuzumab. In certain embodiments, one or more RTK inhibitors is an antisense nucleic acid that targets an RTK or an RTK ligand. In certain embodiments, the antisense nucleic acid is an siRNA, LNA, or a ribozyme. In certain embodiments, one or more RTK inhibitors decrease binding between the RTK and a ligand, wherein binding of the ligand to the RTK activates the RTK. In certain embodiments, one or more RTK inhibitors is an anti-RTK ligand antibody.

### BRIEF DESCRIPTION OF THE DRAWINGS

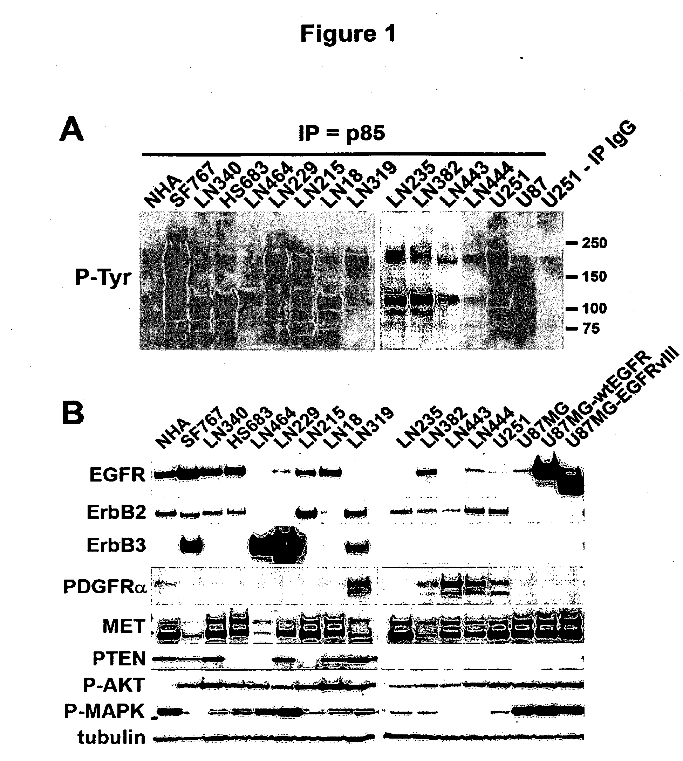

**Figures 1A-1B****:** PI 3-kinase is bound to multiple phosphoproteins and stimulates downstream signaling in the majority of glioma cell lines. A. Whole cell extracts were immunoprecipitated with an antibody to the p85 α subunit of PI 3-kinase and bound proteins were eluted and separated on Tris-Acetate gradient gels. Immunoblots were probed with anti-phosphotyrosine (P-Tyr), revealing the presence of multiple p85α-associated phosphoproteins. B. Whole cell extracts were separated on Bis-Tris gradient gels and immunoblots were probed with the indicated antibodies. Multiple RTKs, such as EGFR, ErbB2, ErbB3, PDGFR α, and Met, are simultaneously expressed in the majority of the cell lines. AKT phosphorylation is increased in every cell line relative to an immortalized normal human astrocyte control (NHA) irrespective of PTEN status, indicating enhanced PI 3-kinase activity in every cell line examined.

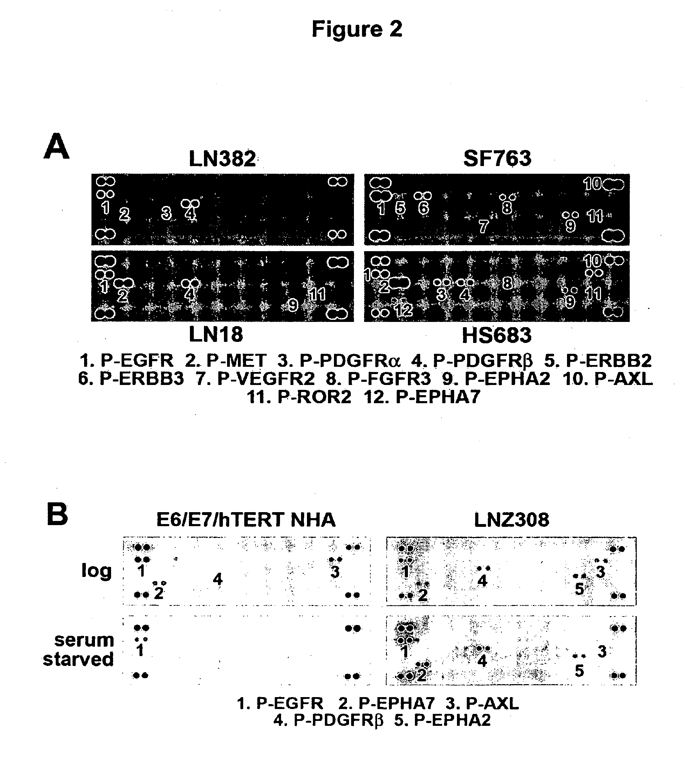

**Figures 2A-2D****:** Multiple RTKs are activated simultaneously in cancer cell lines. A. Whole cell extracts from the glioma cell lines LN382, SF763, LN18, and HS683 were incubated on RTK antibody arrays and phosphorylation status was determined by subsequent incubation with anti-phosphotyrosine-HRP. Each RTK is spotted in duplicate - the pairs of dots in each corner are positive controls. Each positive RTK dot pair is denoted by a red numeral with the corresponding RTKs listed below the arrays. These arrays are representative of various RTK co-expression patterns in the 20 total glioma cell lines examined. B. RTK antibody arrays were utilized as in A with whole cell extracts from an immortalized human astrocyte cell line (E6/E7/hTERT NHA) or the glioma line LNZ308 grown in 10% serum (log) or for 48 hrs in 0.05% serum (serum starved). C. RTK antibody arrays were used as in A to compare RTK activation in whole cell extracts from xenograft tumors derived from the glioma cell lines SF767 or LN340 or from the corresponding *in vitro* cultured cells. D. Antibody arrays were used as in A to examine RTK activation patterns in the lung carcinoma cell lines A549 and H1299 and pancreatic ductal adenocarcinoma lines 8988S and 8988T.

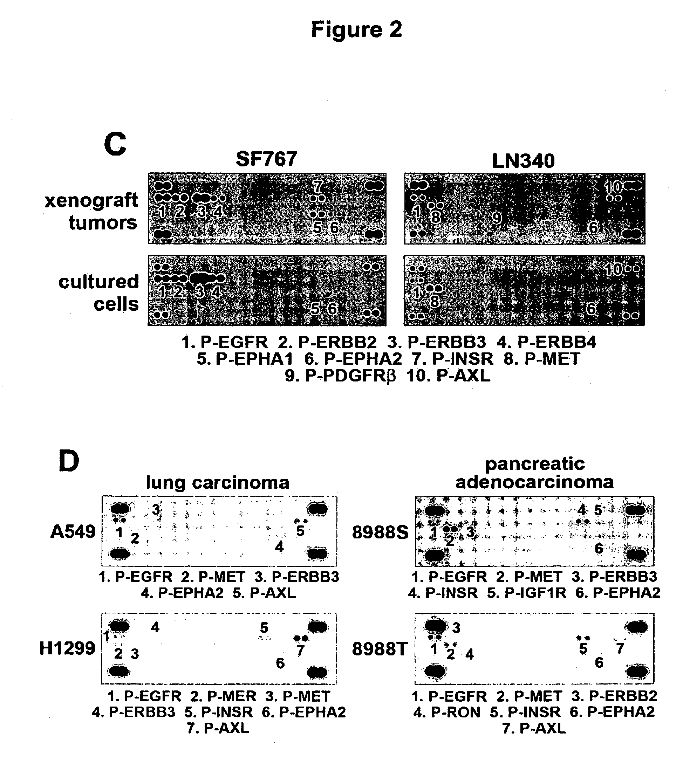

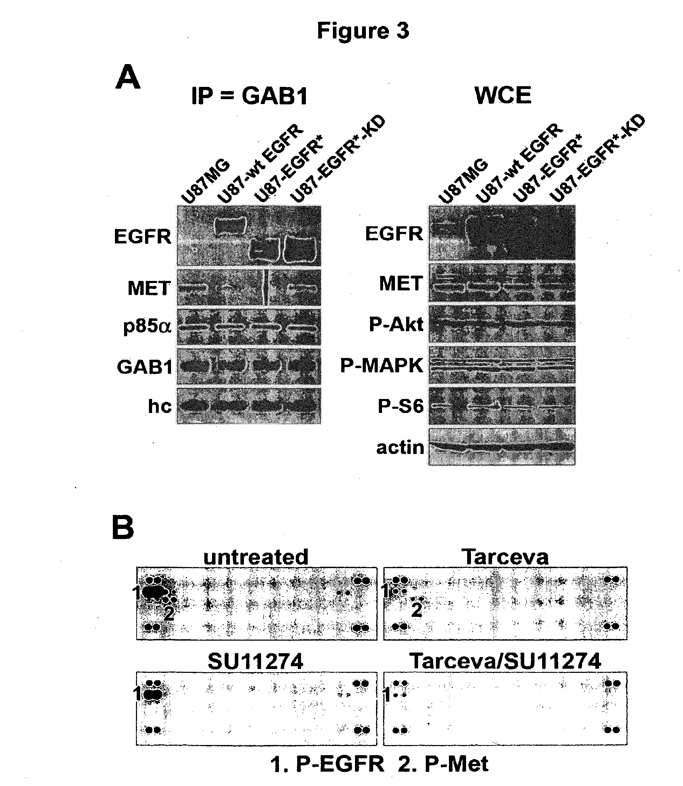

**Figures 3A-3G****:** The inhibition of multiple RTKs is necessary to abrogate PI 3-kinase/RTK complex formation and consequent downstream signaling & cell survival. A. The PI 3-kinase/Gab1 adaptor complex can readily switch between MET and EGFR binding with little discernable effect on downstream signaling. U87MG parental cells or cells constitutively expressing wt EGFR, the activating vIII deletion mutant (EGFR*) or the vIII mutant with an inactivating mutation in its kinase domain (EGFR*-KD) were immunoprecipitated with an antibody to the RTK/PI-3K adaptor protein, GAB1 (left panel), then immunoblots were probed with the indicated antibodies. Heavy chain (hc) is shown to demonstrate equal immunoprecipitation efficiency. Whole cell extracts (WCE) from the same cells were immunoblotted with the indicated antibodies. B. U87MG-EGFR* cells were treated with 10 µM of Tarceva^{™}, the MET inhibitor SU11274 (Calbiochem), both, or vehicle, then whole cell extracts were incubated on RTK antibody arrays as in Figure 2. C. Top panel: U87MG-EGFR* cells were treated with the 10 µM each of the RTK inhibitors Tarceva^{™} (T), SU11274 (S), and/or Gleevec^{™} (G), then whole cell extracts were immunoprecipitated with an antibody to GAB1, eluted, and immunoblotted with antibodies to p85 α or Gab1. Note the faster migration of GAB1 in RTK-inhibitor treated cells, consistent with a decrease in phosphorylation. Bottom panel: Un-immunoprecipitated whole cell extracts were immunoblotted with the indicated antibodies. D. Treatment with multiple RTK inhibitors increases cell death in U87MG-EGFR* cells. Cell were treated for 72 hr with combinations of 5 µM Tarceva (E: bottom panel), 1 µM SU11274 (S), and 1 µM Gleevec (I), or with 10 µM ActinomycinD (ActD) in 0.1% serum-containing medium and then cell viability was assayed by ATP quantitation. Error bars indicate SEM, n=4. E. Impact of single and combination RTK inhibitor treatments on soft-agar colony formation of U87MG-EGFR* cells. Cells were plated in 10% serum, 0.4% agarose containing growth medium with 10 µM of each of the indicated RTK inhibitors. Colonies were counted after 18 days. F. Representative images of U87MG-EGFR* soft agar colonies, as in E. G. RTK inhibition reduces cell viability in part through the PI3K pathway. U87MG-EGFR* cells were transfected with empty vector, HRASV12, myristoylated-AKT, or p110α-CAAX, then treated with 10 µM each of erlotinib (E), SU11274 (S), and imatinib (I) in 0.05% serum-containing medium for 72 hours prior to assaying cell viability. Error bars indicate SEM, n=3.

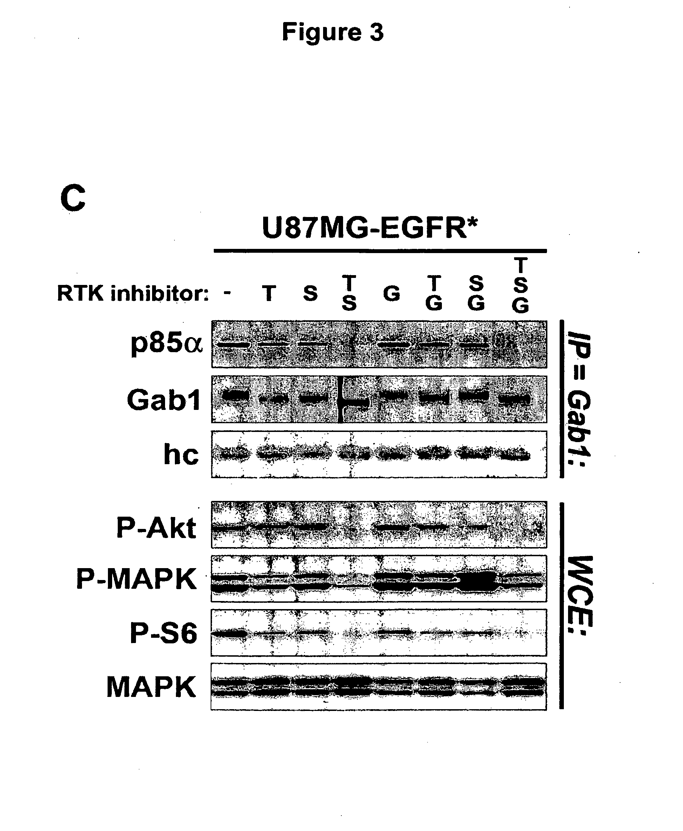

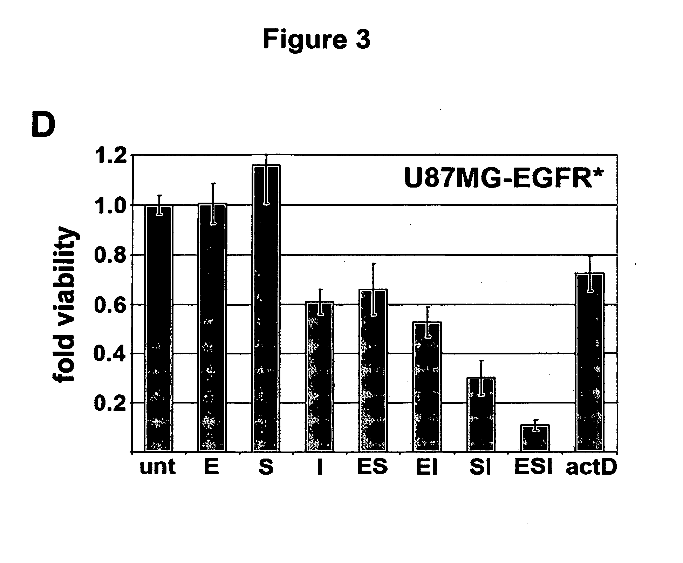

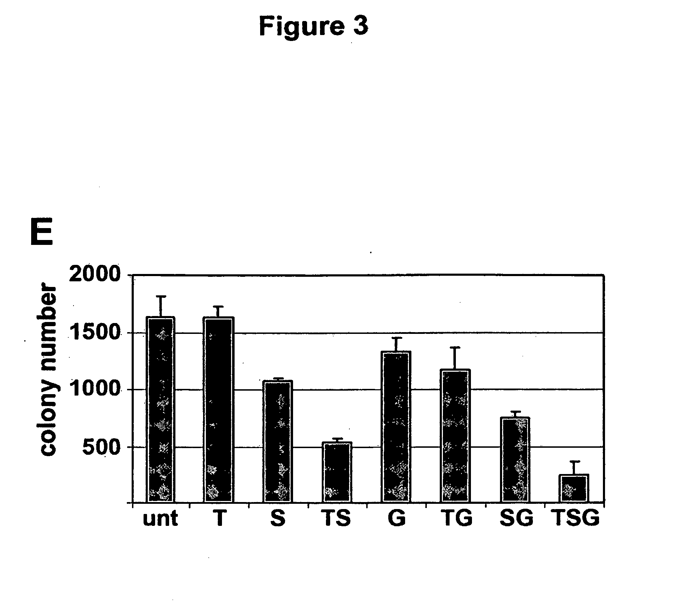

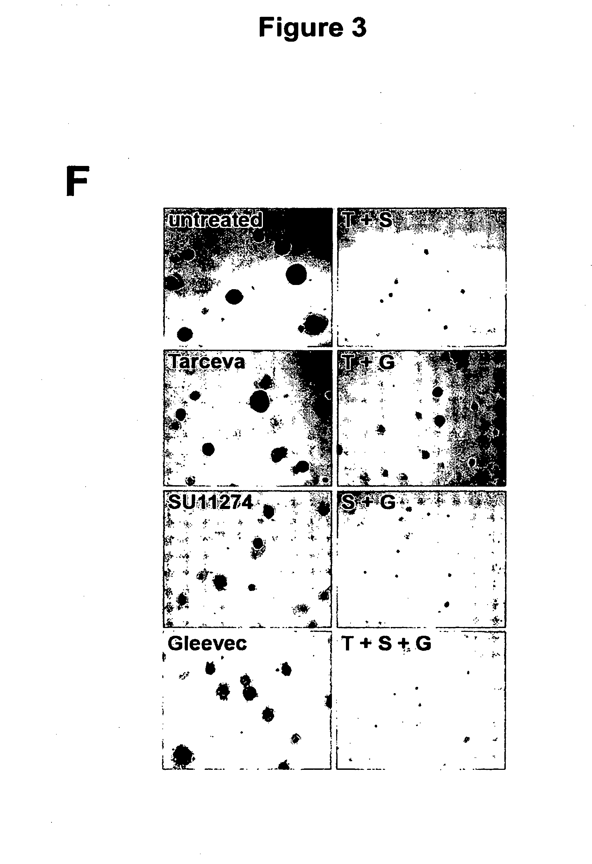

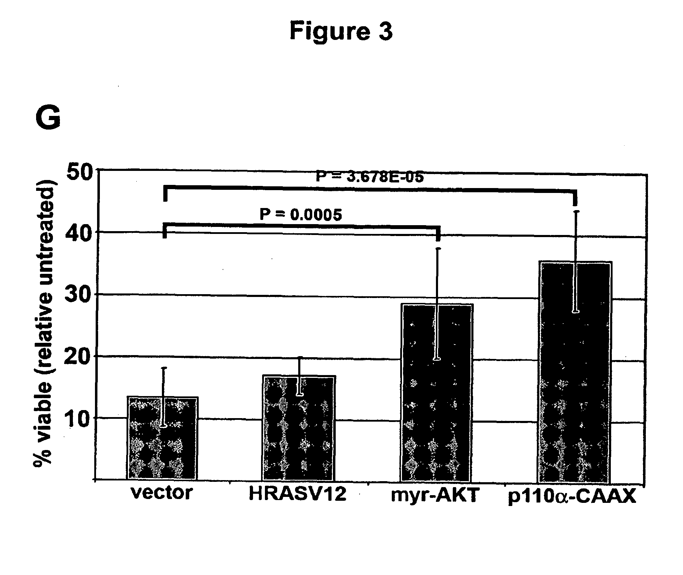

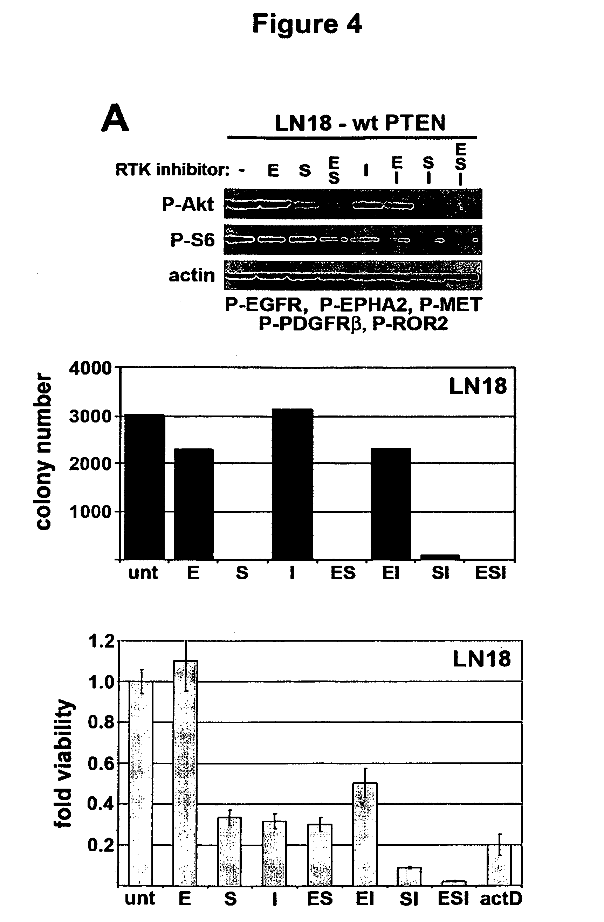

**Figures 4A-4I****:** Multiple RTKs are activated in primary GBM specimens, and targeting multiple activated RTKs in glioma cell lines results in greater cell death than any single treatment. A-E. The glioma cell lines LN18 (wt PTEN), SF767 (wt PTEN), LN382 (mut PTEN) and LNZ308 (mut PTEN) were incubated in 0.05% serum-containing medium cells and treated with 10 µM erlotinib (E or T), 10 µM SU11274 (S), and/or 10 µM imatinib (I or G) and immunoblotted as in Figure 3. The activated RTKs within these cells are indicated beneath the blots; erlotinib (E or T), SU11274 (S), imatinib (I or G) (top panel of 4A-D). Combined RTK inhibitors inhibit soft-agar colony formation in LN18, LN382 and LNZ308 glioma cells - cells were plated and treated as in Figure 3 (4A and 4C middle panel and 4D bottom panel). SU11274 and Gleevec^{™} enhance Tarceva^{™}-mediated cell death - cells were treated with 5 µM erlotinib (E), 2 µM SU11274 (S), and/or 2 µM imatinib (I) or 10 µM Actinomycin D, then cell viability was assayed as in Figure 3 (4A, 4C, and 4E bottom panel). F. Imatinib and SU11274 partially non-specifically inhibit other RTKs. LN382 and LN18 cells were treated with 1µM each RTK inhibitor for 24 hours, then immunoblotted with antibodies to P-PDGFRβ and P-MET. Note the decrease in phosphorylation of MET with imatinib treatment and a decrease in P-PDGFRβ with SU11274 in LN382 cells, but not LN18 cells. G. Antibody arrays were performed as in Figure 2 on protein lysates extracted from snap-frozen primary human gliomas or normal brain autopsy material. H. Antibody arrays were performed as in Figure 2 on protein lysates extracted from colorectal cancer cell lines (WiDr, HT29, LoVo, and SW1417) and primary tumors (CRC_32T and CRC_63T). I. Co-expression of phospho-RTKs in cells dissociated from primary GBM MSK199. Individual tumor-derived cells were immunofluorescently stained with the phospho-RTK antibodies P-EGFR, P-PDGFRα, P-InsR, or P-CSF1R. Each row depicts one field of cells from a slide simultaneously stained with the indicated antibodies. DNA is labeled with Hoechst 33342. Nestin is expressed in neural progenitor cells, tumor endothelial cells, and diffuse gliomas, including astrocytomas and GBMs (K. Sugawara *et al., Lab Invest* **82,** 345 (2002)), and olig2 is expressed in neural progenitors, normal oligodendroglia, and diffuse gliomas (K. L. Ligon *et al., J Neuropathol Exp Neurol* **63,** 499 (2004)). The bottom row depicts cells stained with secondary antibodies only.

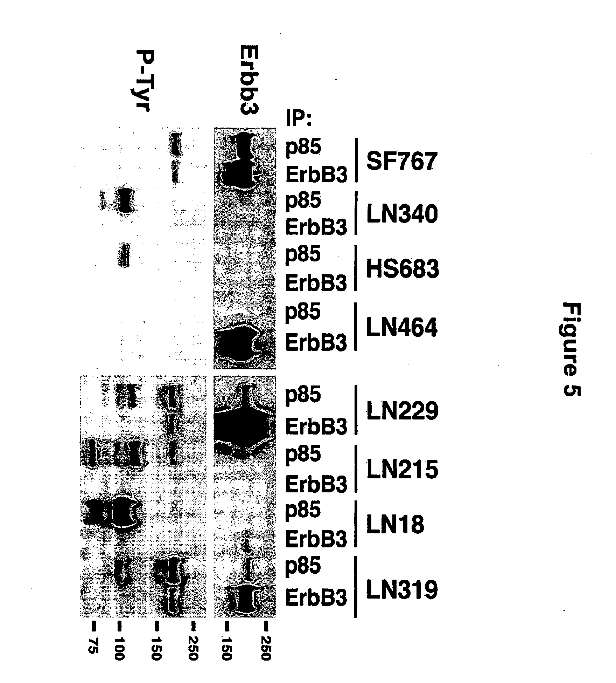

**Figure 5****:** PI 3-kinase/ErbB3 association in glioma cell lines. Whole cell extracts from the indicated cell lines were immunoprecipitated with antibodies to p85α (Upstate) or ErbB3 (Lab Vision), then immunoblotted with antibodies to ErbB3 and phospho-tyrosine (P-Tyr, Upstate). Both antibodies immunoprecipitated a 185 kD phosphoprotein that with immunoreactivity to anti-ErbB3 in 3 of the 8 cell lines depicted here.

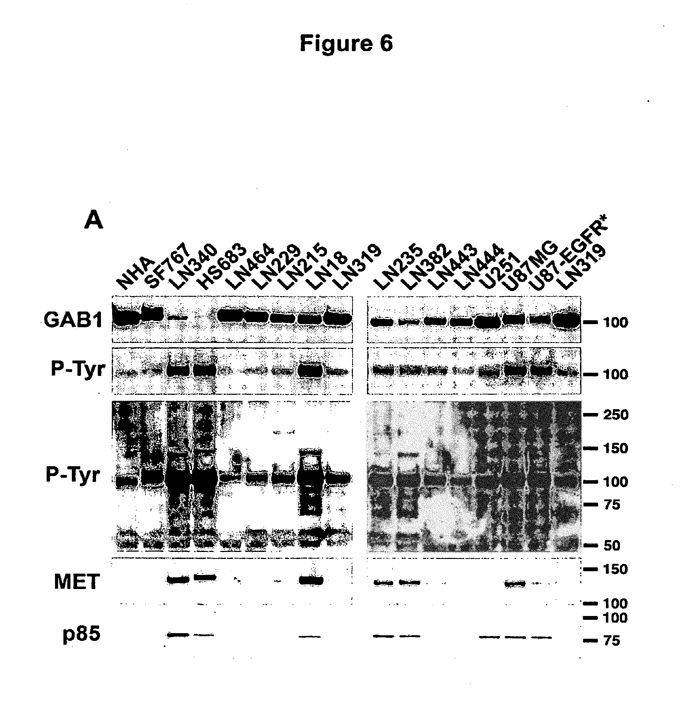

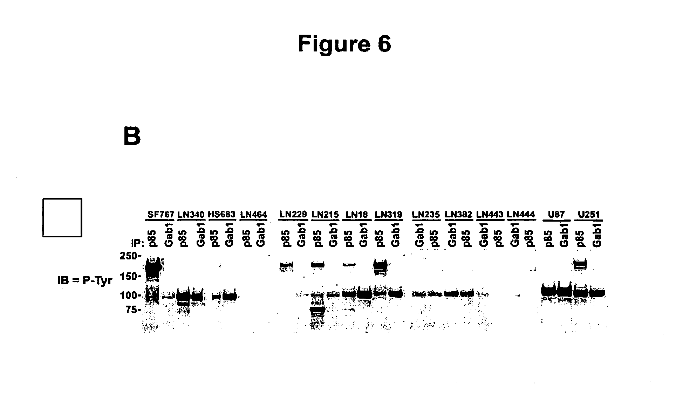

**Figure 6A-6B****:** Gab1/Met association in glioma cell lines. A. Whole cell extracts from the indicated cell lines (including E6/E7/hTERT immortalized normal human astrocytes, NHA) were immunoprecipitated with an antibody to Gab1 (Upstate) followed by immunoblotting with antibodies to Gab1, phosphotyrosine, Met and p85α. Note the presence of a co-precipitating 140 kD phosphoprotein that migrates identically with Met in 7 of the 16 cell lines shown. Met co-precipitation with Gab1 also coincides with p85α/Gab1 co-precipitation, suggesting the formation of a ternary complex. Gab1 is also extensively phosphorylated in the cells in which it is bound to Met. B. PI3K/Gabl association in glioma cell lines. Whole cell extracts from the indicated cell lines were immunoprecipitated with antibodies to p85α or Gab1, then immunoblotted with antibodies to phosphotyrosine (P-Tyr). Both antibodies immunoprecipitated a 110 kD phosphoprotein.

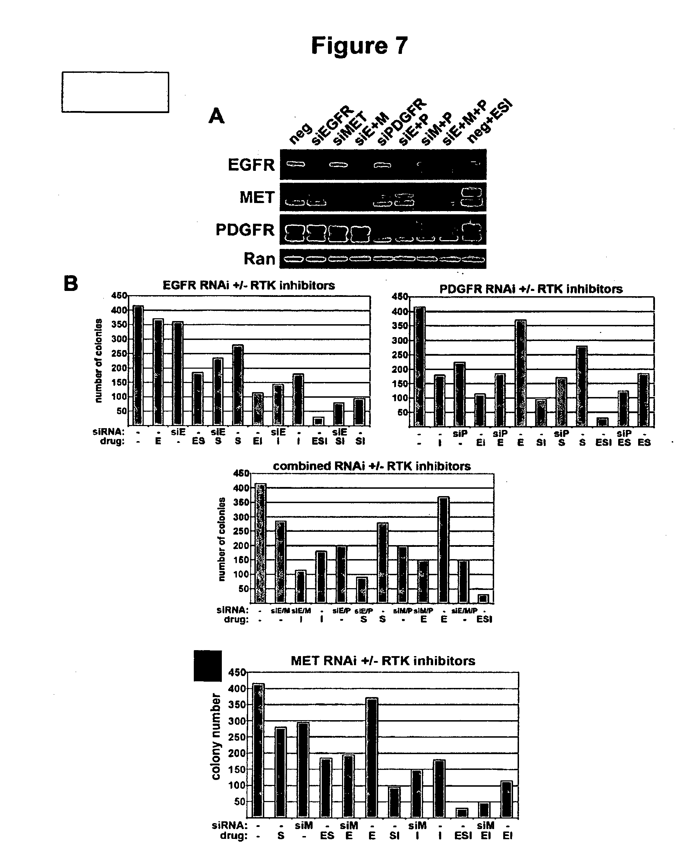

**Figure 7A-7B****:** RNAi can substitute for pharmacological inhibitors. A. LN382 cells were transfected with the indicated siRNAs or a scrambled negative control (neg) and lysates were immunoblotted with antibodies to EGFR, MET, a cocktail of PDGFRα and PDGFRβ, and the loading control Ran. Lysates from cells transfected with the negative control and treated with all 3 RTK inhibitors were run in lane 9. B Soft agar colony inhibition by RTK siRNAs combined with RTK inhibitors. LN382 cells were transfected with the indicated siRNAs or a scrambled negative control (-) and plated and treated with the indicated RTK inhibitors as in Figure 3E. Note the ability of the siRNAs to partially replace their corresponding pharmalogical RTK inhibitor in multiply-treated cells.

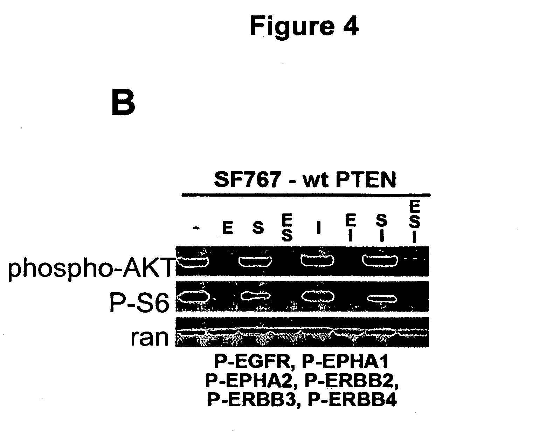

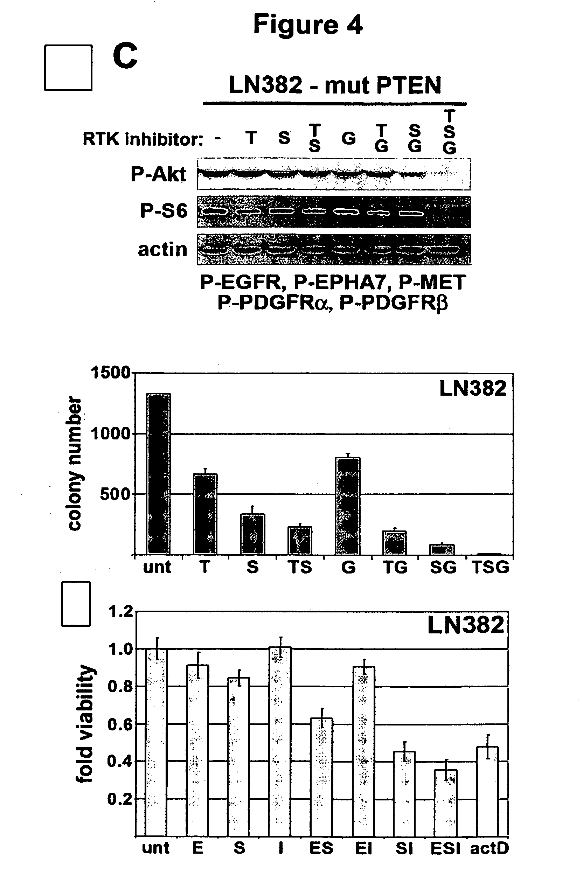

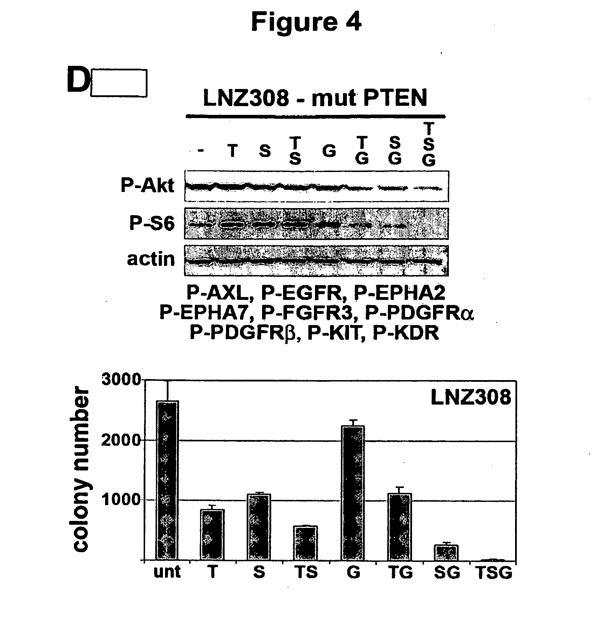

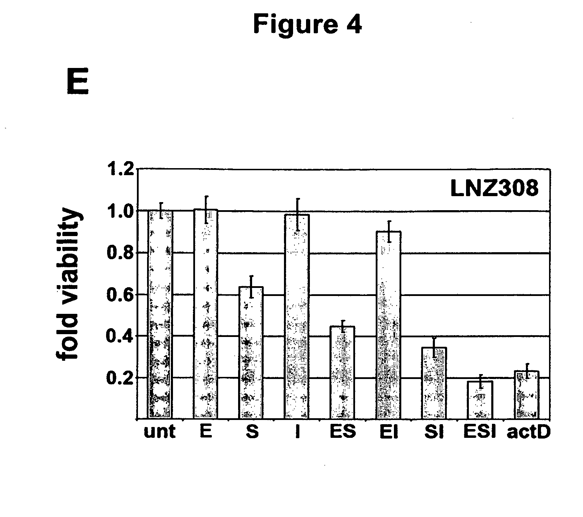

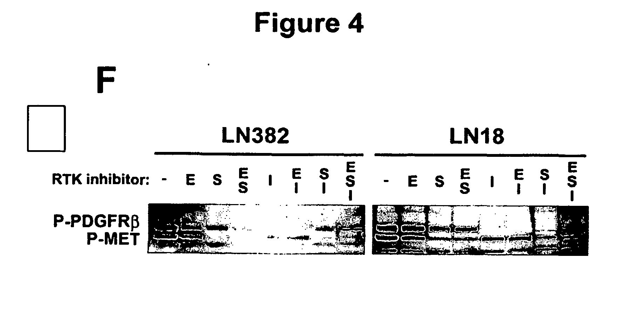

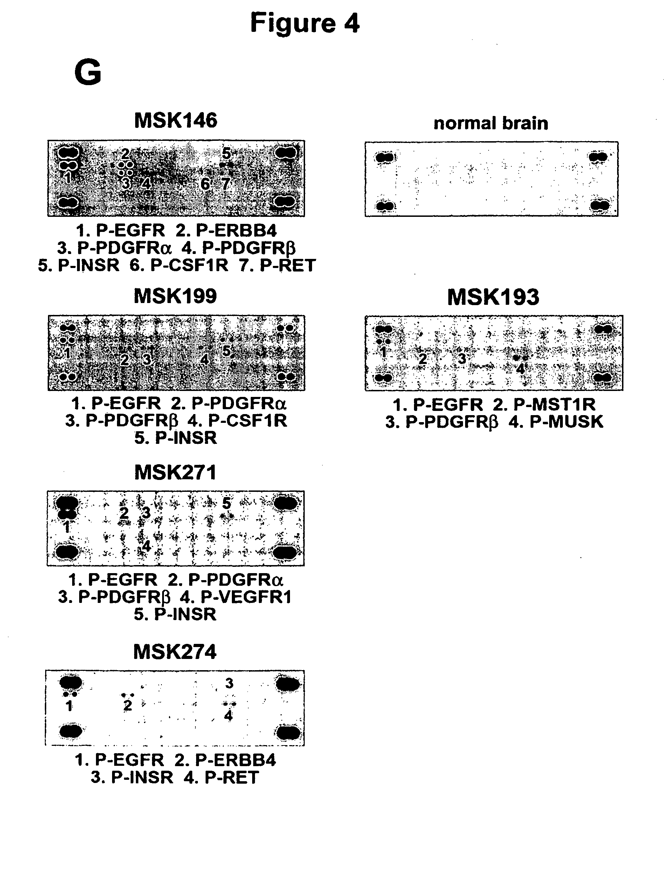

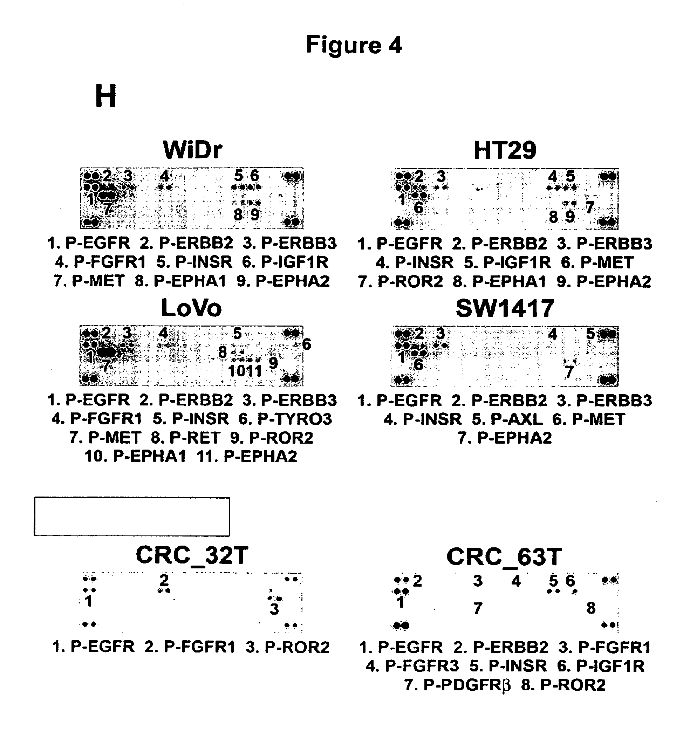

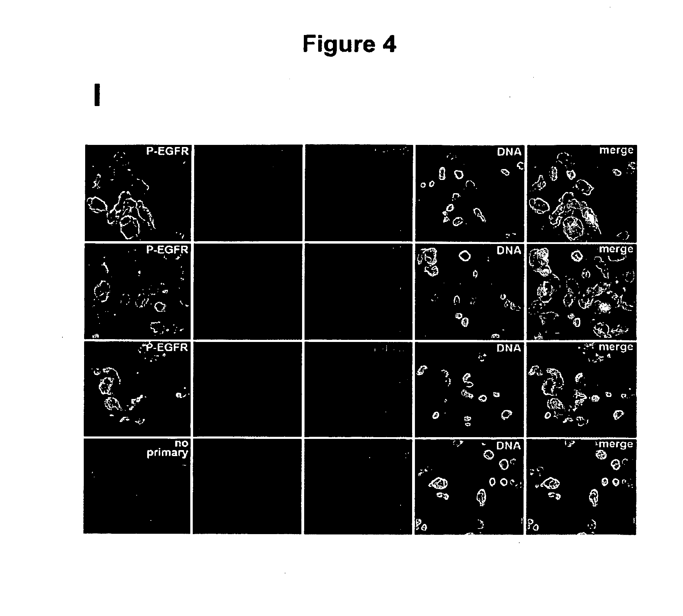

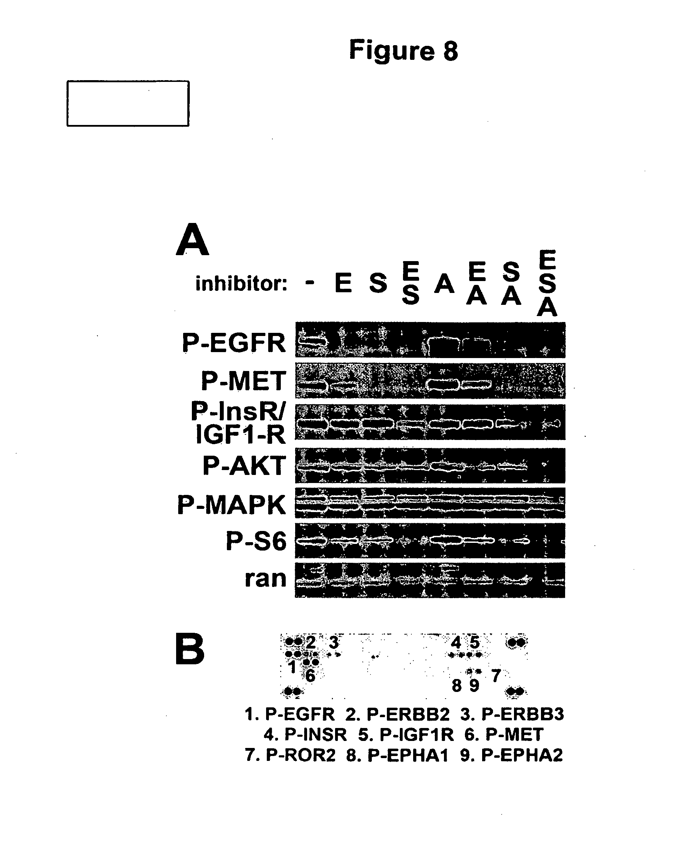

**Figure 8A-8B****:** Targeting multiple activated RTKs in human colorectal adenocarcinoma cell lines results in greater decreases in RTK phosphorylation than targeting individual RTKs. A. HT29 cells (human colorectal adenocarcinoma) were treated with combinations of 1 micromolar Erlotinib (E), 1 micromolar SU11274 (S, a Met inhibitor), and/or 1 micromolar AG538 (A, an IGF1R/InsR inhibitor) and immunoblotted as in Figure 4 and Example 5. B. Whole cell extracts from the drug-treated cells in Figure 8A were incubated on RTK antibody arrays and phosphorylation status was determined as in Figure 2A. The activated RTKs within these cells are indicated beneath the blots.

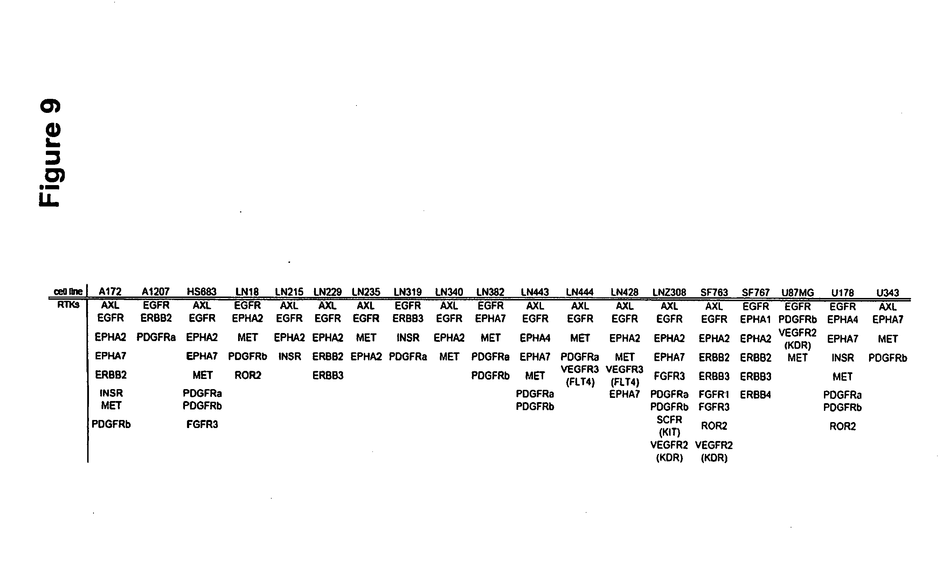

**Figure 9****:** Phosphorylated RTKs in glioma cell lines (Table 1).

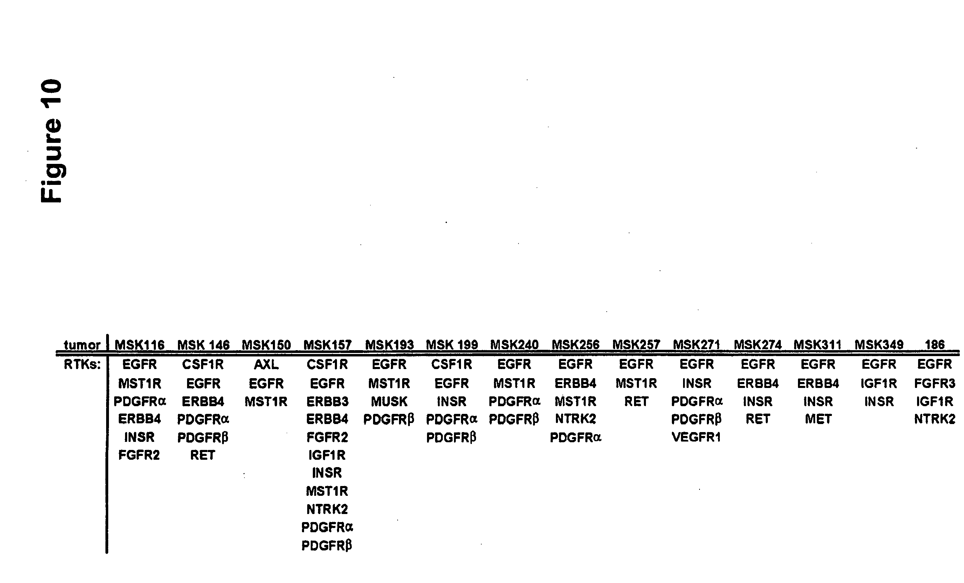

**Figure 10****:** Phosphorylated RTKs in primary gliomas (Table 2).

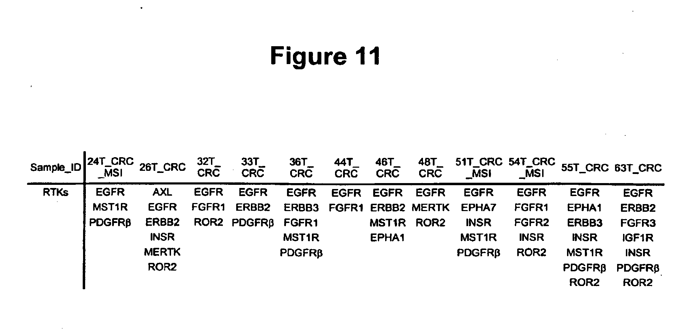

**Figure 11****:** Phosphorylated RTKs in colorectal cancer tumors (Table 3).

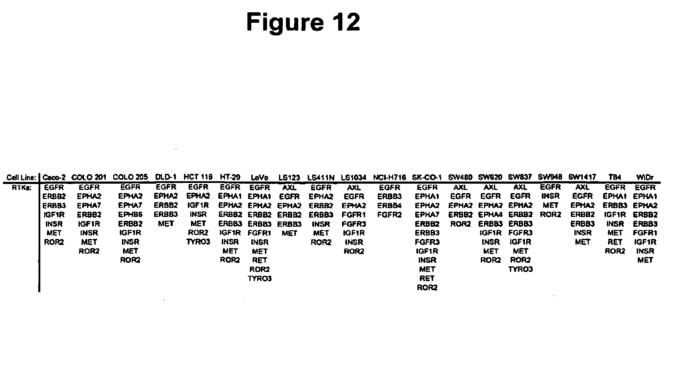

**Figure 12****:** Phosphorylated RTKs in colorectal cancer cell lines (Table 4).

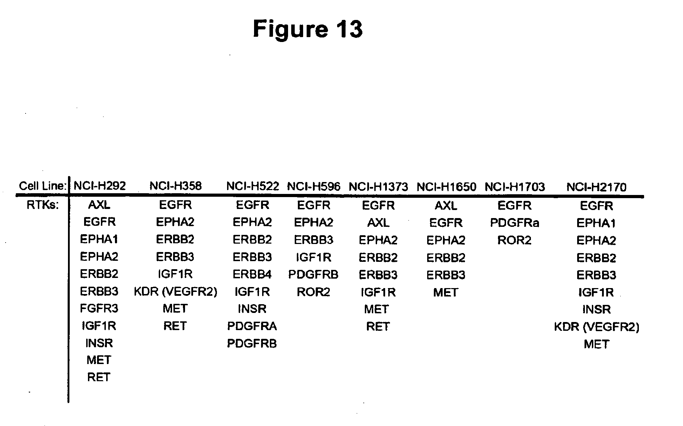

**Figure 13****:** Phosphorylated RTKs in lung cancer cell lines (Table 5).

### DETAILED DESCRIPTION OF THE INVENTION

### A. Definitions

As used herein, the following terms and phrases shall have the meanings set forth below. Unless defined otherwise, all technical and scientific terms used herein have the same meaning as commonly understood to one of ordinary skill in the art.

The singular forms "a," "an," and "the" include plural reference unless the context clearly dictates otherwise.

The terms "comprise" and "comprising" are used in the inclusive, open sense, meaning that additional elements may be included.

The term "including" is used to mean "including but not limited to". "Including" and "including but not limited to" are used interchangeably.

The term "mammal" is known in the art, and exemplary mammals include humans, primates, livestock animals (including bovines, porcines, etc.), companion animals (e.g., canines, felines, etc.) and rodents (e.g., mice and rats).

The terms "gene amplification" and "gene duplication" are used interchangeably and refer to a process by which multiple copies of a gene or gene fragment are formed in a particular cell or cell line. Usually, the amount of the messenger RNA (mRNA) produced, i.e., the level of gene expression, also increases in the proportion of the number of copies made of the particular gene expressed.

The term "copy number of a gene" refers to the number of DNA sequences in a cell encoding a particular gene product. Generally, for a given gene, an animal has two copies of each gene. The copy number can be increased, however, by gene amplification or duplication, or reduced by deletion.

"Cancer" refers to all neoplastic cell growth and proliferation, to all cancerous cells and tissues, and to all metastases. The terms "tumor" and "cancer" are used interchangeable herein. In certain embodiments, cancer is a malignant neoplasm.

Erlotinib hydrochloride (Tarceva^{™} from Genentech/OSIP) is an EGFR specific inhibitor. The terms "erlotinib" and " Tarceva^{™}" will be used interchangeably herein.

Imatinib mesylate (Gleevec^{™} from Novartis) is a tyrosine kinase inhibitor. The terms "imatinib" and " Gleevec^{™}" will be used interchangeably herein.

### B. Overview

The invention is based in part on the discovery by applicants that multiple RTKs are activated in cancer cells and that RTKs can substitute for each other in mediating PI3K signaling.

One aspect of the invention provides methods of profiling RTK activation in cancers that enables the rational design of selecting RTK inhibitors to achieve maximal response.

Another aspect of the invention provides methods of designing treatments, such as first-line treatments, for subjects afflicted with cancer.

Some methods for the invention comprise determining the presence of 10 or more indicators of RTK activation from multiple RTKs from a biological sample of the subject, such as from a tumor biopsy or other sample from a subject that contains cancerous or tumorigenic cells. The activation state may be determined by obtaining an activation profile of the subject generated from the identification of one or more indicators of RTK activation. Indicators of RTK activation may be measured by any number of biochemical, immunological or molecular biology assays of techniques. For example, activated receptor indicators may be identified using enzymatic tests that measure the kinase activity of the RTKs using a substrate that may be phosphorylated by the RTK. Other methods include immunological assays that can distinguish between the activated and inactivated forms of the RTKs. Antibodies able to distinguish phosphorylated forms of the receptors may be particularly useful e.g., anti-phosphotyrosine antibodies. Measurements of gene expression, such as of protein and mRNA levels, and the detection of gene duplication may be used as indicators. Yet another method of identifying indicators of RTK activation comprises detecting activation of downstream signaling components in the cells being assayed.

Some of the methods describe herein for first-line defense also comprise the step of selecting a treatment plan where the subject afflicted with cancer is treated by inhibiting two or more RTK pathways that display indicators of activation in the cancer cells. This may be achieved by selecting multiple agents, with each one inhibiting one of the pathways. It may also be achieved by administering a single inhibitor that inhibits multiple pathways. Or the two approaches may be combined, such that one agent inhibits multiple pathways, and a second agent inhibits one or more pathways, some or all of which may be the same. Some exemplary combinations using two agents are as follows:

| | Agent 1 | Agent 2 |
|---|---|---|
| Activated Pathways Inhibited | A | B |
| Activated Pathways Inhibited | A and B | A and B |
| Activated Pathways Inhibited | A and B | A or B |
| Activated Pathways Inhibited | A and B and C | A or B or C |
| Activated Pathways Inhibited | A and B and C | A and B |
| Activated Pathways Inhibited | A and B and C | A and B and C |

Some exemplary combinations, using three agents are as follows:

| | Agent 1 | Agent 2 | Agent 3 |
|---|---|---|---|
| Activated Pathways Inhibited | A | B | C |
| Activated Pathways Inhibited | A and B | C | A or B or C |
| Activated Pathways Inhibited | A and B | B and C | A or B or C |
| Activated Pathways Inhibited | A and B and C | A or B or C | A or B or C |
| Activated Pathways Inhibited | A and B and C | A and B and C | A or B or C |
| Activated Pathways Inhibited | A and B and C | A and B and C | A and B and C |
| Activated Pathways Inhibited | A and B | A and B | A and B and C |
| Activated Pathways Inhibited | A and B and C | A and B | A and B and C |

The selected treatment plan may further be transmitted to the subject, to an agent of the subject, to a caregiver of the subject, to a family member of the subject, or more preferably to a healthcare professional, such as to a doctor or nurse. The transmission may occur through physical means, such as by fax, mail or telephone, or by electronic means such as through the internet, email or through a computer readable medium.

Further, methods are provided comprising steps for treating the subject according to the treatment plan. Treatment may include a one-time treatment, sporadic treatment or continuous treatment. The agents may be administered simultaneously or at different times. They may also be staggered, coformulated or formulated separately. The dosage forms may be the same or they may be different, such as an oral dosage form for one agent and an intradermal dosage form for the other. Treatment may also include withholding administration of inhibitors of RTK pathways that are not activated in the sample from the subject. The treatment plan may include self-administration by the subject, or administration by another person, such as a health care professional, or combinations thereof. For example, a health care professional may administer chemotherapy while a subject administers oral formulations of an agent to himself or herself.

### C. Therapeutic Treatment

The application describes novel methods of treating cancer. The application also provides methods for designing, formulating or crafting therapeutic treatments and evaluating candidate RTK inhibitors.

In one aspect, the application provides a method of designing a therapeutic treatment for a subject afflicted with cancer, the method comprising determining the presence of 10 or more indicators of RTK activation in a biological sample from the subject and selecting a group of one or more RTK inhibitors, wherein the group inhibits the activity of at least two RTKs that display one or more of the indicators.

In certain embodiments, a biological sample from a subject comprises tissue biopsies, urine, stool, sputum, blood, cells, tissue scrapings, breast aspirates or other cellular materials. In certain embodiments, the biological sample is a tumor biopsy of a glioblastoma multiforme. In certain embodiments, a biological sample comprises cancer cells.

In some embodiments, RTK activation is reflected by an increase in RTK kinase domain autophosphorylation. In certain embodiments, an activated RTK has an increase in kinase domain autophosphorylation of at least 0.5 fold, at least 1 fold, at least 1.5 fold, at least 2 fold, at least 3 fold, at least 4 fold, at least 5 fold, at least 10 fold, at least 15 fold, at least 20 fold at least 50 fold, at least 100 fold or more over control.

A skilled person is capable of selecting the appropriate control depending on individual circumstances. Controls include, for example, non-cancerous cells from the subject, from other subjects, from a population of subjects, or even a reference table or tables derived from one or more individuals containing the relevant values for normal RTK activity.

In certain embodiments, a biological sample from a subject afflicted with cancer is compared to a biological sample from a healthy subject or a reference table derived from a population of healthy subjects. In certain embodiments, the biological samples are from the same type of tissue or cellular material. In certain embodiments, a biological sample comprising tumor or cancer cells from a subject afflicted with cancer is compared to a non-cancerous biological sample from the same subject. In certain embodiments, the non-cancerous biological sample is from the same tissue as the cancerous biological sample.

In some embodiments, RTK activation increases RTK kinase domain autophosphorylation in a subject's cancer cells by at least 0.5 fold over (i) the subject's non-cancerous cells from the same tissue, (ii) an average value for non-cancerous cells from the same tissue from the general population, (iii) an average value for non-cancerous cells from the same tissue from a population of subjects, optionally matched to the subject by any one or more of clinical factors such as age, gender and race.

In some embodiments, RTK activation increases AKT phosphorylation. In certain embodiments, RTK activation increases AKT phosphorylation by at least 0.5 fold, at least 1 fold, at least 1.5 fold, at least 2 fold, at least 3 fold, at least 4 fold, at least 5 fold, at least 10 fold, at least 15 fold, at least 20 fold at least 50 fold, at least 100 fold or more over control. In certain embodiments, the control is selected from (i) the subject's non-cancerous cells from the same tissue, (ii) an average value for non-cancerous cells from the same tissue from the general population, (iii) an average value for non-cancerous cells from the same tissue from a population of subjects, optionally matched to the subject by any one or more of clinical factors such as age, gender and race.

In some embodiments, RTK activation increases cancer cell growth or proliferation. In certain embodiments, RTK activation increases cell growth by at least 0.5 fold, at least 1 fold, at least 1.5 fold, at least 2 fold, at least 3 fold, at least 4 fold, at least 5 fold, at least 10 fold, at least 15 fold, at least 20 fold at least 50 fold, at least 100 fold or more over control. In certain embodiments, the control is selected from (i) the subject's non-cancerous cells from the same tissue, (ii) an average value for non-cancerous cells from the same tissue from the general population, (iii) an average value for non-cancerous cells from the same tissue from a population of subjects, optionally matched to the subject by any one or more of clinical factors such as age, gender and race.

In some embodiments, RTK activation inhibits apoptosis in a cancer cell. In certain embodiments, RTK activation decreases apoptosis in a group of cancer cells by at least 0.5 fold, at least 1 fold, at least 1.5 fold, at least 2 fold, at least 3 fold, at least 4 fold, at least 5 fold, at least 10 fold, at least 15 fold, at least 20 fold at least 50 fold, at least 100 fold or more over control. In certain embodiments, the control is selected from (i) the subject's non-cancerous cells from the same tissue, (ii) an average value for non-cancerous cells from the same tissue from the general population, (iii) an average value for non-cancerous cells from the same tissue from a population of subjects, optionally matched to the subject by any one or more of clinical factors such as age, gender and race.

In certain embodiments, the group of one or more RTK inhibitors can inhibit the activity of at least 3, 4, 5, 6, 7, 8, 9, 10, 15, 20, or more RTKs that display one or more indicators. In certain embodiments, an RTK displays at least 1, 2, 3, 4 or more indicators of activation. In certain embodiments, an RTK displays an indicator of RTK activity, such as, for example increased phosphorylation. In certain embodiments, an RTK displays an indicator of RTK amplification.

### Indicators of RTK activation

In some embodiments, an indicator of RTK activation used in the methods is the presence of one or more mutations in an RTK gene, an increase in RTK RNA expression levels, an increase in RTK protein expression levels, an increase of cell-surface expression of the RTK protein, or an increase in RTK biochemical activity. In some embodiments, an indicator of RTK activation is the presence of one or more mutations in an RTK ligand gene, an increase in RTK ligand RNA expression levels, an increase in RTK ligand protein expression levels, an increase in RTK ligand secretion, or an increase in RTK ligand activity.

In some embodiments, an indicator of RTK activation is an increase in RTK RNA expression level, RTK RNA stability, RTK protein expression, or cell-surface RTK expression. These indicators may result from one or more mutations in the RTK gene or from one or more mutations in other genes including, for example, transcription factors, chaperones, heat-shock proteins, (e.g., Hsp90), RNA binding proteins, proteasomal proteins, and ubiquitination proteins.

### RNA/Protein Expression

In certain embodiments, an indicator of RTK activation used in the methods is an increase in expression of RNA or protein in a cancer cell in a subject as compared to a control. As used herein expression level refers to the amount of an RNA transcript or protein product in a cell. An increase in expression level refers to an increase in the amount of an RNA transcript or protein product or both in a cell compared to a control cell. The term misexpression refers to the expression of an RNA transcript or protein product in a cell or tissue that normally does not express said RNA or protein and is encompassed by term "increase in expression level". The increase in expression is not limited to any particular molecular mechanism and includes an increase in gene copy number, an increase in gene transcription, an increase in RNA stability or reduction in degradation, an increase in translation, and an increase in protein stability or reduction in degradation.

In certain embodiments, a cancer cell expresses an RTK RNA, an RTK protein, or both at least 10%, 20%, 30%, 40%, 50%, 60%, 70%, 80%, 90%, 100%, 125%, 150%, 175%, 200%, 250%, 300%, 400%, 500%, 1000% or more than a control. In certain embodiments, the control is selected from (i) the subject's non-cancerous cells from the same tissue, (ii) an average value for non-cancerous cells from the same tissue from the general population, (iii) an average value for non-cancerous cells from the same tissue from a population of subjects, optionally matched to the subject by any one or more of clinical factors such as age, gender and race.

Any suitable means of measuring the expression of the RNA products of the RTKs or RTK ligands can be used to determine the presence of one or more indicators of RTK activation. For example, the methods may utilize a variety of polynucleotides that specifically hybridize to one or more of the RNA products of the RTKs or RTK ligands including, for example, oligonucleotides, cDNA, DNA, RNA, PCR products, synthetic DNA, synthetic RNA, or other combinations of naturally occurring of modified nucleotides which specifically hybridize to one or more of the RNA products of the RTKs or RTK ligands. Such polynucleotides may be used in combination with the methods to measure RNA expression described further herein including, for example, array hybridization, RT-PCR, nuclease protection and northern blots.

### i. Array Hybridization

In certain embodiments, the expression level of an RTK or RTK ligand may be determined using array hybridization to evaluate the level of RNA expression. Array hybridization utilizes nucleic acid members stably associated with a support that can hybridize with RTK or RTK ligand expression products. The length of a nucleic acid member attached to the array can range from 8 to 1000 nucleotides in length and are chosen so as to be specific for the RNA products of the RTKs. The array may comprise, for example, one or more nucleic acid members that are specific for RTKs or RTK ligands, or variants thereof (e.g., splice variants), including, for example, EGFR, ErbB2, ErbB3, ErbB4, FGFR1, FGFR2α, FGFR3, FGFR4, InsulinR, IGF-1R, Axl, Dtk, Mer, HGFR, MSPR, PDGFRα, PDGFRβ, SCFR, Flt-3, M-CSFR, c-Ret, ROR1, ROR2, Tie-1, Tie-2, TrkA, TrkB, TrkC, VEGFR1, VEGFR2, VEGFR3, MuSK, EphA1, EphA2, EphA3, EphA4, EphA6, EphA7, EphB1, EphB2, EphB4, and EphB6. The array may comprise, for example, one or more nucleic acid members that are specific for RTK ligands, or variants thereof (e.g., splice variants), including, for example, EGF, VEGF-A, VEGF-B, VEGF-C, VEGF-D, VEGF-E, placental growth factor (P1GF), VEGF-F, hepatocyte growth factor (HGF), TGFα, amphiregulin, betacellulin, heparin-binding EGF, epiregulin, FGF1, FGF2, FGF8, and neural cell adhesion molecules (CAMs). The nucleic acid members may be RNA or DNA, single or double stranded, and/or may be oligonucleotides or PCR fragments amplified from cDNA. Preferably oligonucleotides are approximately 10-100, 10-50, 20-50, or 20-30 nucleotides in length. Portions of the expressed regions of the RTKs or RTK ligands can be utilized as probes on the array. More particularly oligonucleotides complementary to the RTK or RTK ligand genes and or cDNAs derived from the genes are useful. For oligonucleotide based arrays, the selection of oligonucleotides corresponding to the gene of interest which are useful as probes is well understood in the art. More particularly it is important to choose regions which will permit hybridization to the target nucleic acids. Factors such as the Tm of the oligonucleotide, the percent GC content, the degree of secondary structure and the length of nucleic acid are important factors. See for example U.S. Pat. No. 6,551,784.

Arrays may be constructed, custom ordered, or purchased from a commercial vendor. Various methods for constructing arrays are well known in the art. For example, methods and techniques applicable to oligonucleotide synthesis on a solid support, e.g., in an array format have been described, for example, in WO 00/58516, U.S. Pat. Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752 and Zhou et al., Nucleic Acids Res. 32: 5409-5417 (2004).

In an exemplary embodiment, construction and/or selection oligonucleotides may be synthesized on a solid support using maskless array synthesizer (MAS). Maskless array synthesizers are described, for example, in PCT application No. WO 99/42813 and in corresponding U.S. Pat. No. 6,375,903. Other methods for constructing arrays include, for example, light-directed methods utilizing masks (e.g., VLSIPS^{™} methods described, for example, in U.S. Pat. Nos. 5,143,854, 5,510,270 and 5,527,681), flow channel methods (see e.g., U.S. Pat. No. 5,384,261), spotting methods (see e.g., U.S. Pat. No. 5,807,522), pin-based methods (see e.g., U.S. Pat. No. 5,288,514), and methods utilizing multiple supports (see e.g., .S. Pat. Nos. 5,770,358, 5,639,603, and 5,541,061).

In certain embodiments, an array of nucleic acid members stably associated with the surface of a support is contacted with a sample comprising target nucleic acids under hybridization conditions sufficient to produce a hybridization pattern of complementary nucleic acid members/target complexes in which one or more complementary nucleic acid members at unique positions on the array specifically hybridize to target nucleic acids. The identity of target nucleic acids which hybridize can be determined with reference to location of nucleic acid members on the array.

Control nucleic acid members may be present on the array including nucleic acid members comprising oligonucleotides or nucleic acids corresponding to genomic DNA, housekeeping genes, vector sequences, negative and positive control genes, and the like. Control nucleic acid members are calibrating or control genes whose function is not to tell whether a particular gene of interest is expressed, but rather to provide other useful information, such as background or basal level of expression.

Other control nucleic acids on the array may be used as target expression control nucleic acids and mismatch control nucleotides to monitor non-specific binding or cross-hybridization to a nucleic acid in the sample other than the target to which the probe is directed. Mismatch probes thus indicate whether a hybridization is specific or not. For example, if the target is present, the perfectly matched probes should be consistently brighter than the mismatched probes. In addition, if all control mismatches are present, the mismatch probes are used to detect a mutation.

An array provided herein may comprise a substrate sufficient to provide physical support and structure to the associated nucleic acids present thereon under the assay conditions in which the array is employed, particularly under high throughput handling conditions.

The substrate may be biological, non-biological, organic, inorganic, or a combination of any of these, existing as particles, strands, precipitates, gels, sheets, tubing, spheres, beads, containers, capillaries, pads, slices, films, plates, slides, chips, etc. The substrate may have any convenient shape, such as a disc, square, sphere, circle, etc. The substrate is preferably flat or planar but may take on a variety of alternative surface configurations. The substrate may be a polymerized Langmuir Blodgett film, functionalized glass, Si, Ge, GaAs, GaP, SiO₂, SIN₄, modified silicon, or any one of a wide variety of gels or polymers such as (poly)tetrafluoroethylene, (poly)vinylidenedifluoride, polystyrene, polycarbonate, or combinations thereof. Other substrate materials will be readily apparent to those of skill in the art in view of this disclosure.

In certain embodiments, a target nucleic acid sample may comprise total mRNA or a nucleic acid sample corresponding to mRNA (e.g., cDNA) isolated from a biological sample. Total mRNA may be isolated from a given sample using, for example, an acid guanidinium-phenol-chloroform extraction method and polyA+mRNA may be isolated using oligo dT column chromatography or using (dT)n magnetic beads (see, e.g., Sambrook et al., Molecular Cloning: A Laboratory Manual (2nd ed.), Vols. 1-3, Cold Spring Harbor Laboratory, (1989), or Current Protocols in Molecular Biology, F. Ausubel et al., ed. Greene Publishing and Wiley-Interscience, New York (1987). In certain embodiments, total RNA may be extracted using TRIzol^{™} reagent (GIBCO/BRL, Invitrogen Life Technologies, Cat. No. 15596). Purity and integrity of RNA may be assessed by absorbance at 260/280 nm and agarose gel electrophoresis followed by inspection under ultraviolet light.

In certain embodiments, it may be desirable to amplify the target nucleic acid sample prior to hybridization. One of skill in the art will appreciate that whatever amplification method is used, if a quantitative result is desired, care must be taken to use a method that maintains or controls for the relative frequencies of the amplified nucleic acids. Methods of quantitative amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified nucleic acid. Detailed protocols for quantitative PCR are provided in PCR Protocols, A Guide to Methods and Applications, Innis et al., Academic Press, Inc. N.Y., (1990).

In certain embodiments, the target nucleic acid sample mRNA is reverse transcribed with a reverse transcriptase and a primer consisting of oligo dT and a sequence encoding the phage T7 promoter to provide single-stranded DNA template. The second DNA strand is polymerized using a DNA polymerase. After synthesis of double-stranded cDNA, T7 RNA polymerase is added and RNA is transcribed from the cDNA template. Successive rounds of transcription from each single cDNA template results in amplified RNA. Methods of *in vitro* transcription are well known to those of skill in the art (see, e.g., Sambrook, supra.) and this particular method is described in detail by Van Gelder, et al., 1990, Proc. Natl. Acad. Sci. USA, 87: 1663-1667 who demonstrate that *in vitro* amplification according to this method preserves the relative frequencies of the various RNA transcripts. Moreover, Eberwine et al. Proc. Natl. Acad. Sci. USA, 89: 3010-3014 provide a protocol that uses two rounds of amplification via *in vitro* transcription to achieve greater than 106 fold amplification of the original starting material thereby permitting expression monitoring even where biological samples are limited.

Detectable labels suitable for use in accordance with the methods described herein include any composition detectable by spectroscopic, photochemical, biochemical, immunochemical, electrical, optical or chemical means. Useful labels include biotin for staining with labeled streptavidin conjugate, magnetic beads (e.g., Dynabeads^{™}), fluorescent dyes (e.g., fluorescein, texas red, rhodamine, green fluorescent protein, and the like), radiolabels (e.g., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,277,437; 4,275,149; and 4,366,241.

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and calorimetric labels are detected by simply visualizing the colored label.

The labels may be incorporated by any of a number of means well known to those of skill in the art. For example, the label may be simultaneously incorporated during the amplification step in the preparation of the sample nucleic acids. Thus, for example, polymerase chain reaction (PCR) with labeled primers or labeled nucleotides will provide a labeled amplification product. Additionally, transcription amplification, as described above, using a labeled nucleotide (e.g. fluorescein-labeled UTP and/or CTP) incorporates a label into the transcribed nucleic acids.

Alternatively, a label may be added directly to the original nucleic acid sample (e.g., mRNA, polyA mRNA, cDNA, etc.) or to the amplification product after the amplification is completed. Means of attaching labels to nucleic acids are well known to those of skill in the art and include, for example, nick translation or end-labeling (e.g. with a labeled RNA) by kinasing of the nucleic acid and subsequent attachment (ligation) of a nucleic acid linker joining the sample nucleic acid to a label (e.g., a fluorophore).

In certain embodiments, the fluorescent modifications are by cyanine dyes e.g. Cy-3/Cy-5 dUTP, Cy-3/Cy-5 dCTP (Amersham Pharmacia) or alexa dyes (Khan, et al., 1998, Cancer Res. 58:5009-5013).

In certain embodiments, it may be desirable to simultaneously hybridize two target nucleic acid samples to the array, including, for example, a target nucleic acid sample from a subject (e.g., a subject having or at risk of having cancer or another hyperproliferative disorder) and a control nucleic acid sample (e.g., a healthy individual). In a further embodiment, one target nucleic acid sample may be obtained from a tumor or other cancerous growth of a subject, while the second target nucleic acid sample may be obtained from healthy biological material from the same subject. The two target samples used for comparison are labeled with different fluorescent dyes which produce distinguishable detection signals, for example, targets from a control sample are labeled with Cy5 and targets from a subject to be monitored or diagnosed are labeled with Cy3. The differently labeled target samples are hybridized to the same microarray simultaneously. The labeled targets may be purified using methods known in the art, e.g., by ethanol purification or column purification.

In certain embodiments, the target nucleic acid samples will include one or more control molecules which hybridize to control probes on the microarray to normalize signals generated from the microarray. Labeled normalization targets may be, for example, nucleic acid sequences that are perfectly complementary to control oligonucleotides that are spotted onto the microarray as described above. The signals obtained from the normalization controls after hybridization provide a control for variations in hybridization conditions, label intensity, reading efficiency and other factors that may cause the signal of a perfect hybridization to vary between arrays. Signals (e.g., fluorescence intensity) read from all other probes in the array may be divided by the signal (e.g., fluorescence intensity) from the control probes, thereby normalizing the measurements.

Normalization targets may be selected to reflect the average length of the other targets present in the sample or they may be selected to cover a range of lengths. The normalization control(s) also can be selected to reflect the (average) base composition of the other probes in the array. In certain embodiments, only one or a few normalization probes are used and they are selected such that they hybridize well (i.e., have no secondary structure and do not self hybridize) and do not match any target molecules. Normalization probes may be localized at any position in the array or at multiple positions throughout the array to control for spatial variation in hybridization efficiency. For example, normalization controls may be located at the corners or edges of the array as well as in the middle.

Nucleic acid hybridization to an array involves incubating a denatured probe or target nucleic acid member on an array and a target nucleic acid sample under conditions wherein the probe or target nucleic acid member and its complementary target can form stable hybrid duplexes through complementary base pairing. The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. Under low stringency conditions (e.g., low temperature and/or high salt) hybrid duplexes (e.g., DNA:DNA, RNA:RNA, or RNA:DNA) will form even where the annealed sequences are not perfectly complementary. Thus specificity of hybridization is reduced at lower stringency. Conversely, at higher stringency (e.g., higher temperature or lower salt) successful hybridization requires fewer mismatches. Methods of optimizing hybridization conditions are well known to those of skill in the art (see, e.g., Laboratory Techniques in Biochemistry and Molecular Biology, Vol. 24: Hybridization With Nucleic acid Probes, P. Tijssen, ed. Elsevier, N.Y., (1993)).

Following hybridization, non-hybridized labeled or unlabeled nucleic acids are removed from the support surface by washing thereby generating a pattern of hybridized target nucleic acid on the substrate surface. A variety of wash solutions are known to those of skill in the art and may be used. The resultant hybridization patterns of labeled, hybridized oligonucleotides and/or nucleic acids may be visualized or detected in a variety of ways, with the particular manner of detection being chosen based on the particular label of the target nucleic acid sample, where representative detection means include scintillation counting, autoradiography, fluorescence measurement, calorimetric measurement, light emission measurement and the like.

Following hybridization, washing step and/or subsequent treatments, the resultant hybridization pattern is detected. In detecting or visualizing the hybridization pattern, the intensity or signal value of the label will be not only be detected but quantified, e.g., the signal from each spot on the hybridized array will be measured and compared to a unit value corresponding to the signal emitted by a known number of end labeled target nucleic acids to obtain a count or absolute value of the copy number of each end-labeled target that is hybridized to a particular spot on the array in the hybridization pattern.

Methods for analyzing the data collected from array hybridizations are well known in the art. For example, where detection of hybridization involves a fluorescent label, data analysis can include the steps of determining fluorescent intensity as a function of substrate position from the data collected, removing outliers, i.e., data deviating from a predetermined statistical distribution, and calculating the relative binding affinity of the test nucleic acids from the remaining data. The resulting data is displayed as an image with the intensity in each region varying according to the binding affinity between associated oligonucleotides and/or nucleic acids and the test nucleic acids.

### ii. RT-PCR

In certain embodiments, the level of expression of RNA products of the RTKs and RTK ligands can be measured by amplifying the RNA products from a sample using reverse transcription (RT) in combination with the polymerase chain reaction (PCR). In certain embodiments, the RT can be quantitative as would be understood to a person skilled in the art.

Total RNA, or mRNA from a sample may be used as a template and a primer specific to the transcribed portion of an RTK or RTK ligand is used to initiate reverse transcription. Methods of reverse transcribing RNA into cDNA are well known and are described, for example, in Sambrook et al., 1989, supra. Primer design can be accomplished utilizing commercially available software (e.g., Primer Designer 1.0, Scientific Software etc.) or methods that are standard and well known in the art. Primer Software programs can be used to aid in the design and selection of primers include, for example, The Primer Quest software which is available through the following web site link: biotools.idtdna.com/primerquest/. Additionally, the following website links are useful when searching and updating sequence information from the Human Genome Database for use in RTK primer design: 1) the NCBI LocusLink Homepage: world wide web at ncbi.nlm.nih.gov/LocusLink/, and 2) Ensemble Human Genome Browser: world wide web at ensembl.org/Homo_sapiens, preferably using pertinent RTK or RTK ligand information such as Gene or Sequence Description, Accession or Sequence ID, Gene Symbol, RefSeq #, and/or UniGene #.

General guidelines for designing primers that may be used in accordance with the methods described herein include the following: the product or amplicon length may be ~100-150 bases, the optimum Tm may be ~60° C, or about 58-62° C, and the GC content may be ~50%, or about 45-55%. Additionally, it may be desirable to avoid certain sequences such as one or more of the following: (i) strings of three or more bases at the 3'-end of each primer that are complementary to another part of the same primer or to another primer in order to reduce primer-dimer formation, (ii) sequences within a primer that are complementary to another primer sequence, (iii) runs of 3 or more G's or C's at the 3'-end, (iv) single base repeats greater than 3 bases, (v) unbalanced distributions of G/C- and A/T rich domains, and/or (vi) a T at the 3'-end.

The product of the reverse transcription is subsequently used as a template for PCR. PCR provides a method for rapidly amplifying a particular nucleic acid sequence by using multiple cycles of DNA replication catalyzed by a thermostable, DNA-dependent DNA polymerase to amplify the target sequence of interest. PCR requires the presence of a nucleic acid to be amplified, two single-stranded oligonucleotide primers flanking the sequence to be amplified, a DNA polymerase, deoxyribonucleoside triphosphates, a buffer and salts. The method of PCR is well known in the art. PCR, is performed as described in Mullis and Faloona, 1987, Methods Enzymol., 155: 335.

QRT-PCR, which is quantitative in nature, can also be performed to provide a quantitative measure of RTK gene expression levels. In QRT-PCR reverse transcription and PCR can be performed in two steps, or reverse transcription combined with PCR can be performed concurrently. One of these techniques, for which there are commercially available kits such as Taqman (Perkin Elmer, Foster City, Calif.), is performed with a transcript-specific antisense probe. This probe is specific for the PCR product (e.g. a nucleic acid fragment derived from a gene) and is prepared with a quencher and fluorescent reporter probe complexed to the 5' end of the oligonucleotide. Different fluorescent markers are attached to different reporters, allowing for measurement of two products in one reaction. When Taq DNA polymerase is activated, it cleaves off the fluorescent reporters of the probe bound to the template by virtue of its 5'-to-3' exonuclease activity. In the absence of the quenchers, the reporters now fluoresce. The color change in the reporters is proportional to the amount of each specific product and is measured by a fluorometer; therefore, the amount of each color is measured and the PCR product is quantified. The PCR reactions are performed in 96 well plates so that samples derived from many individuals are processed and measured simultaneously. The Taqman system has the additional advantage of not requiring gel electrophoresis and allows for quantification when used with a standard curve.

A second technique useful for detecting PCR products quantitatively is to use an intercalating dye such as the commercially available QuantiTect SYBR Green PCR (Qiagen, Valencia Calif.). RT-PCR is performed using SYBR green as a fluorescent label which is incorporated into the PCR product during the PCR stage and produces a fluorescence proportional to the amount of PCR product. Additionally, other systems to quantitatively measure mRNA expression products are known including Molecular Beacons^{™}.

Additional techniques to quantitatively measure RNA expression include, but are not limited to, polymerase chain reaction, ligase chain reaction, Qbeta replicase (see, e.g., International Application No. PCT/US87/00880), isothermal amplification method (see, e.g., Walker et al. (1992) PNAS 89:382-396), strand displacement amplification (SDA), repair chain reaction, Asymmetric Quantitative PCR (see, e.g., U.S. Publication No. US200330134307A1) and the multiplex microsphere bead assay described in Fuja et al., 2004, Journal of Biotechnology 108:193-205.

The level of gene expression can be measured by amplifying RNA from a sample using transcription based amplification systems (TAS), including nucleic acid sequence amplification (NASBA) and 3SR. See, e.g., Kwoh et al (1989) PNAS USA 86:1173; International Publication No. WO 88/10315; and U.S. Pat. No. 6,329,179. In NASBA, the nucleic acids may be prepared for amplification using conventional phenol/chloroform extraction, heat denaturation, treatment with lysis buffer and minispin columns for isolation of DNA and RNA or guanidinium chloride extraction of RNA. These amplification techniques involve annealing a primer that has target specific sequences. Following polymerization, DNA/RNA hybrids are digested with RNase H while double stranded DNA molecules are heat denatured again. In either case the single stranded DNA is made fully double stranded by addition of second target specific primer, followed by polymerization. The double-stranded DNA molecules are then multiply transcribed by a polymerase such as T7 or SP6. In an isothermal cyclic reaction, the RNA's are reverse transcribed into double stranded DNA, and transcribed once with a polymerase such as T7 or SP6. The resulting products, whether truncated or complete, indicate target specific sequences.

Several techniques may be used to separate amplification products. For example, amplification products may be separated by agarose, agarose-acrylamide or polyacrylamide gel electrophoresis using conventional methods. See Sambrook et al., 1989. Several techniques for detecting PCR products quantitatively without electrophoresis may also be used (see for example PCR Protocols, A Guide to Methods and Applications, Innis et al., Academic Press, Inc. N.Y., (1990)). For example, chromatographic techniques maybe employed to effect separation. There are many kinds of chromatography which may be used: adsorption, partition, ion-exchange and molecular sieve, HPLC, and many specialized techniques for using them including column, paper, thin-layer and gas chromatography (Freifelder, Physical Biochemistry Applications to Biochemistry and Molecular Biology, 2nd ed., Wm. Freeman and Co., New York, N.Y., 1982).

Amplification products must be visualized in order to confirm amplification of the nucleic acid sequences of interest. One typical visualization method involves staining of a gel with ethidium bromide and visualization under UV light. Alternatively, if the amplification products are integrally labeled with radio- or fluorometrically-labeled nucleotides, the amplification products may then be exposed to x-ray film or visualized under the appropriate stimulating spectra, following separation.

Alternatively, visualization may be achieved indirectly. Following separation of amplification products, a labeled, nucleic acid probe is brought into contact with the amplified nucleic acid sequence of interest. The probe may be conjugated to a chromophore, radiolabeled, or conjugated to a binding partner, such as an antibody or biotin, where the other member of the binding pair carries a detectable moiety.

Additionally, detection may be carried our using Southern blotting and hybridization with a labeled probe. The techniques involved in Southern blotting are well known to those of skill in the art and may be found in many standard books on molecular protocols. See Sambrook et al., 1989, supra. Briefly, amplification products are separated by gel electrophoresis. The gel is then contacted with a membrane, such as nitrocellulose, permitting transfer of the nucleic acid and non-covalent binding. Subsequently, the membrane is incubated with a chromophore-conjugated probe that is capable of hybridizing with a target amplification product. Detection is by exposure of the membrane to x-ray film or ion-emitting detection devices.

### iii. Nuclease Protection Assays

In certain embodiments, nuclease protection assays (including both ribonuclease protection assays and S1 nuclease assays) can be used to detect and quantitate RNA products of RTKs or RTK ligands. In nuclease protection assays, an antisense probe (e.g., radiolabeled or nonisotopic labeled) hybridizes in solution to an RNA sample. Following hybridization, single-stranded, unhybridized probe and RNA are degraded by nucleases. An acrylamide gel is used to separate the remaining protected fragments. Typically, solution hybridization can accommodate up to ~100 µg of sample RNA whereas blot hybridizations may only be able to accommodate ~20-30 µg of RNA sample.

The ribonuclease protection assay, which is the most common type of nuclease protection assay, requires the use of RNA probes. Oligonucleotides and other single-stranded DNA probes can only be used in assays containing S1 nuclease. The single-stranded, antisense probe must typically be completely homologous to target RNA to prevent cleavage of the probe:target hybrid by nuclease.

### iv. Northern Blots

A standard Northern blot assay can also be used to ascertain an RNA transcript size, identify alternatively spliced RNA transcripts, and the relative amounts of RNA products of RTKs or RTK ligands, in accordance with conventional Northern hybridization techniques known to those persons of ordinary skill in the art. In Northern blots, RNA samples are first separated by size via electrophoresis in an agarose gel under denaturing conditions. The RNA is then transferred to a membrane, crosslinked and hybridized with a labeled probe. Nonisotopic or high specific activity radiolabeled probes can be used including random-primed, nick-translated, or PCR-generated DNA probes, *in vitro* transcribed RNA probes, and oligonucleotides. Additionally, sequences with only partial homology (e.g., cDNA from a different species or genomic DNA fragments that might contain an exon) may be used as probes. The labeled probe, e.g., a radiolabeled cDNA, either containing the full-length, single stranded DNA or a fragment of that DNA sequence may be any length up to at least 20, at least 30, at least 50, or at least 100 consecutive nucleotides in length. The probe can be labeled by any of the many different methods known to those skilled in this art. The labels most commonly employed for these studies are radioactive elements, enzymes, chemicals that fluoresce when exposed to ultraviolet light, and others. A number of fluorescent materials are known and can be utilized as labels. These include, but are not limited to, fluorescein, rhodamine, auramine, Texas Red, AMCA blue and Lucifer Yellow. A particular detecting material is anti-rabbit antibody prepared in goats and conjugated with fluorescein through an isothiocyanate. Nonlimiting examples of isotopes include ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, and ¹⁸⁶Re. Enzyme labels are likewise useful, and can be detected by any of the presently utilized colorimetric, spectrophotometric, fluorospectrophotometric, amperometric or gasometric techniques. The enzyme may be conjugated to the selected probe by reaction with bridging molecules such as carbodiimides, diisocyanates, glutaraldehyde and the like. Any enzymes known to one of skill in the art can be utilized, including, for example, peroxidase, beta-D-galactosidase, urease, glucose oxidase plus peroxidase and alkaline phosphatase. U.S. Pat. Nos. 3,654,090, 3,850,752, and 4,016,043 are referred to by way of example for their disclosure of alternate labeling material and methods.

### Protein Products

The expression level of an RTK or RTK ligand may also be measured by determining protein levels using any art-known method. Traditional methodologies for protein quantification include 2-D gel electrophoresis, mass spectrometry and antibody binding. Exemplary methods for assaying protein levels in a biological sample include antibody-based techniques, such as immunoblotting (western blotting), immunohistological assay, enzyme linked immunosorbent assay (ELISA), radioimmunoassay (RIA), or protein chips. For example, RTK-specific monoclonal antibodies can be used both as an immunoadsorbent and as an enzyme-labeled probe to detect and quantify RTKs. The amount of RTK present in the sample can be calculated by reference to the amount present in a standard preparation using a linear regression computer algorithm. In another embodiment, RTKs or RTK ligands may be immunoprecipitated from a biological sample using an antibody specific for an RTK. The isolated proteins may then be run on an SDS-PAGE gel and blotted (e.g., to nitrocellulose or other suitable material) using standard procedures. The blot may then be probed with an anti-RTK or RTK ligand specific antibody to determine the expression level of the RTK or RTK ligand.

Gel electrophoresis, immunoprecipitation and mass spectrometry may be carried out using standard techniques, for example, such as those described in Molecular Cloning A Laboratory Manual, 2nd Ed., ed. by Sambrook, Fritsch and Maniatis (Cold Spring Harbor Laboratory Press: 1989), Harlow and Lane, Antibodies: A Laboratory Manual (1988 Cold Spring Harbor Laboratory), G. Suizdak, Mass Spectrometry for Biotechnology (Academic Press 1996), as well as other references cited herein.

As used herein, the term "antibody" (Ab) or "monoclonal antibody" (mAb) is meant to include intact molecules as well as antibody portions (such as, for example, Fab, Fab', F(ab')₂, Fv, single chain Fv, or Fd) which are capable of specifically binding to an RTK or RTK ligand.

Antibodies suitable for isolation and detection of RTKs or RTK ligands may be purchased commercially from a variety of sources and may also be produced using standard techniques. Generally applicable methods for producing antibodies are well known in the art and are described extensively in references cited herein, e.g., Current Protocols in Immunology and Using Antibodies: A Laboratory Manual. It is noted that antibodies can be generated by immunizing animals (or humans) either with a full length polypeptide, a partial polypeptide, fusion protein, or peptide (which may be conjugated with another moiety to enhance immunogenicity). The specificity of the antibody will vary depending upon the particular preparation used to immunize the animal and on whether the antibody is polyclonal or monoclonal. In general, preferred antibodies will possess high affinity, e.g., a K_{d} of <200 nM, and preferably, of <100 nM for a specific RTK or RTK ligand.

### Genetic Mutations

In some embodiments, an indicator of RTK activation used in the methods is the presence of one or more mutations in an RTK gene. In some embodiments, mutations are in the promoter region; the 5'UTR; the protein coding regions including the ligand binding domain, the transmembrane domain, or in the intracellular domain; an enhancer element or in the the 3'UTR. Mutations include, for example, a point mutation, a deletion, an insertion, inversions, gene amplification, or chromosomal translocations.

In some embodiments, one or more mutations in an RTK gene results in gene amplification, increasing RTK RNA expression level, increasing RTK protein expression, increasing RTK cell-surface expression, or increasing RTK activity.

In some embodiments, one or more mutations in the RTK gene confers upon an RTK protein kinase domain-autophosphorylation or receptor dimerization that is independent of, or highly sensitized to, ligand binding. In some embodiments, one or more mutations in the RTK gene result in binding between the RTK protein and a downstream target that is independent of RTK phosphorylation or ligand binding.

In some embodiments, an indicator of RTK activation is the presence of one or more mutations in an RTK ligand gene. In some embodiments, mutations are in the promoter region; an enhancer element; the 5'UTR; the protein coding regions; or the 3'UTR.

In some embodiments, one or more mutations in an RTK ligand gene result in gene amplification, increasing RTK ligand RNA expression level, increasing RTK ligand protein expression, increasing RTK ligand secretion and increasing RTK ligand activity.

In certain embodiments, the presence of one or more mutations in an RTK or RTK ligand is determined using a technique comprising contacting nucleic acid from said sample with a nucleic acid probe that is capable of specifically hybridizing to nucleic acid encoding a mutated RTK/RTK ligand protein, or fragment thereof incorporating a mutation, and detecting said hybridization. In a particular embodiment said probe is detectably labeled such as with a radioisotope (³H, ³²P, ³³P etc), a fluorescent agent (rhodamine, fluorescene etc.) or a chromogenic agent. In a particular embodiment the probe is an antisense oligomer, for example PNA, morpholino-phosphoramidates, LNA or 2'-alkoxyalkoxy. The probe may be from about 8 nucleotides to about 100 nucleotides, or about 10 to about 75, or about 15 to about 50, or about 20 to about 30.

In certain embodiments, the presence of one or more mutations in an RTK is determined using a technique comprising amplifying from said sample nucleic acid corresponding to the kinase domain of said RTK or a fragment thereof suspected of containing a mutation, and comparing the electrophoretic mobility of the amplified nucleic acid to the electrophoretic mobility of corresponding wild-type RTK gene or fragment thereof. A difference in the mobility indicates the presence of a mutation in the amplified nucleic acid sequence. Electrophoretic mobility may be determined on polyacrylamide gel.

In certain embodiments, the presence of one or more mutations in an RTK or RTK ligand is determined using a technique wherein amplified RTK or RTK ligand or a fragment nucleic acid may be analyzed for detection of mutations using Enzymatic Mutation Detection (EMD) (Del Tito et al, Clinical Chemistry 44:731-739, 1998). EMD uses the bacteriophage resolvase T4 endonuclease VII that scans along double-stranded DNA until it detects and cleaves structural distortions caused by base pair mismatches resulting from point mutations, insertions and deletions. Detection of two short fragments formed by resolvase cleavage, for example by gel eletrophoresis, indicates the presence of a mutation. Benefits of the EMD method are a single protocol to identify point mutations, deletions, and insertions assayed directly from PCR reactions eliminating the need for sample purification, shortening the hybridization time, and increasing the signal-to-noise ratio. Mixed samples containing up to a 20-fold excess of normal DNA and fragments up to 4 kb in size can been assayed. However, EMD scanning does not identify particular base changes that occur in mutation positive samples requiring additional sequencing procedures to identity of the mutation if necessary. CEL I enzyme can be used similarly to resolvase T4 endonuclease VII as demonstrated in U.S. Pat. No. 5,869,245.

Detection of point mutations in an RTK or RTK ligand may be accomplished by molecular cloning of the RTK or RTK ligand allele (or alleles) and sequencing that allele(s) using techniques well known in the art. Alternatively, the polymerase chain reaction (PCR) can be used to amplify gene sequences directly from a genomic DNA preparation from the tumor tissue. The DNA sequence of the amplified sequences can then be determined and mutations identified therefrom. The polymerase chain reaction is well known in the art and described in Saiki et al., Science 239:487, 1988; U.S. Pat. No. 4,683,203; and U.S. Pat. No. 4,683,195.

The ligase chain reaction, which is known in the art, can also be used to amplify RTK or RTK ligand sequences. See Wu et al., Genomics, Vol. 4, pp. 560-569 (1989). In addition, a technique known as allele specific PCR can be used. (See Ruano and Kidd, Nucleic Acids Research, Vol. 17, p. 8392, 1989.) According to this technique, primers are used which hybridize at their 3'ends to a particular RTK or RTK ligand mutation. If the particular RTK or RTK ligand mutation is not present, an amplification product is not observed. Amplification Refractory Mutation System (ARMS) can also be used as disclosed in European Patent Application Publication No. 0332435 and in Newton et al., Nucleic Acids Research, Vol. 17, p.7, 1989. Insertions and deletions of genes can also be detected by cloning, sequencing and amplification. In addition, restriction fragment length polymorphism, (RFLP) probes for the gene or surrounding marker genes can be used to score alteration of an allele or an insertion in a polymorphic fragment. Single stranded conformation polymorphism (SSCP) analysis can also be used to detect base change variants of an allele. (Orita et al., Proc. Natl. Acad. Sci. USA Vol. 86, pp. 2766-2770, 1989, and Genomics, Vol. 5, pp. 874-879, 1989.) Other techniques for detecting insertions and deletions as are known in the art can be used.

In certain embodiments, the presence of one or more mutations in an RTK or RTK ligand is determined using the technique of mismatch detection. Mismatches are hybridized nucleic acid duplexes which are not 100% complementary. The lack of total complementarity may be due to deletions, insertions, inversions, substitutions or frameshift mutations. Mismatch detection can be used to detect point mutations in the gene or its mRNA product. While these techniques are less sensitive than sequencing, they are simpler to perform on a large number of tumor samples. An example of a mismatch cleavage technique is the RNase protection method, which is described in detail in Winter et al., Proc. Natl. Acad. Sci. USA, Vol. 82, p. 7575, 1985 and Meyers et al., Science, Vol. 230, p. 1242, 1985. In the practice a the present invention the method involves the use of a labeled riboprobe which is complementary to the human wild-type RTK or RTK ligand gene coding sequence (or exons 18-21 or KDR thereof). The riboprobe and either mRNA or DNA isolated from the tumor tissue are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full-length duplex RNA for the riboprobe and the mRNA or DNA. The riboprobe need not be the full length of the RTK or RTK ligand mRNA or gene but can be segments thereof. If the riboprobe comprises only a segment of the RTK or RTK ligand mRNA or gene it will be desirable to use a number of these probes to screen the whole mRNA sequence for mismatches.

In a similar manner, DNA probes can be used to detect mismatches, through enzymatic or chemical cleavage. See, e.g., Cotton et al., Proc. Natl. Acad. Sci. USA, Vol. 85, 4397, 1988; and Shenk et al., Proc. Natl. Acad. Sci. USA, Vol. 72, p. 989, 1975. Alternatively, mismatches can be detected by shifts in the electrophoretic mobility of mismatched duplexes relative to matched duplexes. See, e.g., Cariello, Human Genetics, Vol. 42, p. 726, 1988. With either riboprobes or DNA probes, the cellular mRNA or DNA which might contain a mutation that can be amplified using PCR before hybridization. Changes in DNA of the RTK or RTK ligand gene can also be detected using Southern hybridization, especially if the changes are gross rearrangements, such as deletions and insertions.

DNA sequences of the RTK or RTK ligand which have been amplified by use of polymerase chain reaction may also be screened using allele-specific probes. These probes are nucleic acid oligomers, each of which contains a region of the RTK or RTK ligand sequence harboring a known mutation. For example, one oligomer may be about 30 nucleotides in length, corresponding to a portion of the RTK or RTK ligand gene sequence. By use of a battery of such allele-specific probes, PCR amplification products can be screened to identify the presence of a previously identified mutation in the RTK or RTK ligand gene. Hybridization of allele-specific probes with amplified RTK or RTK ligand sequences can be performed, for example, on a nylon filter. Hybridization to a particular probe under stringent hybridization conditions indicates the presence of the same mutation in the tumor tissue as in the allele-specific probe.

In some embodiments, one or more mutations in an RTK gene increase the DNA copy number of an RTK, also known as "amplification". In certain embodiments, gene amplification is at least 2-fold, at least 3-fold, at least 4-fold, or at least 5-fold.

In certain embodiments, the detection of RTK gene amplification is used as an indicator of RTK activation. Gene amplification is a quantitative modification, whereby the actual number of complete coding sequence, i.e. a gene, increases, leading to an increased number of available templates for transcription, an increased number of translatable transcripts, and, ultimately, to an increased abundance of the protein encoded by the amplified gene.

The presence of an RTK gene that has undergone amplification in tumors is evaluated by determining the copy number of the target genes, i.e., the number of DNA sequences in a cell encoding the target protein. Generally, a normal diploid cell has two copies of a given autosomal gene. The copy number can be increased, however, by gene amplification or duplication, for example, in cancer cells, or reduced by deletion. Methods of evaluating the copy number of a particular gene are well known in the art, and include, hybridization and amplification based assays. In some embodiments, the actual number of amplified copies of the gene is determined. In another embodiment, a qualitative measure of gene amplification is obtained. In certain embodiments, the RTK is said to be activated when a cell contains at least 1, 2, 3, 4, 5, 6, 7, 8, 9, 10, 11, 12, 13, 14 or 15 or more extra copies of an RTK gene, RTK transcriptional unit or RTK coding sequence.

### Amplification-Based Assays

In certain embodiments, amplification-based assays can be used to measure copy number of RTK genes. In such amplification-based assays, the corresponding RTK nucleic acid sequence acts as a template in an amplification reaction (for example, Polymerase Chain Reaction or PCR). In a quantitative amplification, the amount of amplification product will be proportional to the amount of template in the original sample. Comparison to appropriate controls provides a measure of the copy-number of the RTK gene, corresponding to the specific probe used. The presence of a higher level of amplification product, as compared to a control, is indicative of amplified RTK.

Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. Detailed protocols for quantitative PCR are provided, for example, in Innis et al. (1990) PCR Protocols, A Guide to Methods and Applications, Academic Press, Inc. N.Y. The known nucleic acid sequence for the RTKs is sufficient to enable one of skill to routinely select primers to amplify any portion of the gene. (e.g., GenBank Accession Nos. NM_005228 (EGFR), NM_001982 (ERBB3), NM_000245 (MET), NM_000141 (FGFR2). Further nucleic acid sequences for RTKs are available in Genbank, available on the world wide web at ncbi.nlm.nih.gov.)

Real time PCR is another amplification technique that can be used to determine gene copy levels or levels of mRNA expression. (See, e.g., Gibson et al., Genome Research 6:995-1001, 1996; Heid et al., Genome Research 6:986-994, 1996). Real-time PCR evaluates the level of PCR product accumulation during amplification. This technique permits quantitative evaluation of mRNA levels in multiple samples. For gene copy levels, total genomic DNA is isolated from a sample. For mRNA levels, mRNA is extracted from tumor and normal tissue and cDNA is prepared using standard techniques. Real-time PCR can be performed, for example, using a Perkin Elmer/Applied Biosystems (Foster City, Calif.) 7700 Prism instrument. Matching primers and fluorescent probes can be designed for genes of interest using, for example, the primer express program provided by Perkin Elmer/Applied Biosystems (Foster City, Calif.). Optimal concentrations of primers and probes can be initially determined by those of ordinary skill in the art, and control (for example, beta-actin) primers and probes may be obtained commercially from, for example, Perkin Eliner/Applied Biosystems (Foster City, Calif.). To quantitate the amount of the specific nucleic acid of interest in a sample, a standard curve is generated using a control. Standard curves may be generated using the Ct values determined in the real-time PCR, which are related to the initial concentration of the nucleic acid of interest used in the assay. Standard dilutions ranging from 10-10⁶ copies of the gene of interest are generally sufficient. In addition, a standard curve is generated for the control sequence. This permits standardization of initial content of the nucleic acid of interest in a tissue sample to the amount of control for comparison purposes.

Methods of real-time quantitative PCR using TaqMan probes are well known in the art. Detailed protocols for real-time quantitative PCR are provided, for example, for RNA in: Gibson et al., 1996, A novel method for real time quantitative RT-PCR. Genome Res., 10:995-1001; and for DNA in: Heid et al., 1996, Real time quantitative PCR. Genome Res., 10:986-994.

A TaqMan-based assay also can be used to quantify RTK polynucleotides. TaqMan based assays use a fluorogenic oligonucleotide probe that contains a 5' fluorescent dye and a 3' quenching agent. The probe hybridizes to a PCR product, but cannot itself be extended due to a blocking agent at the 3' end. When the PCR product is amplified in subsequent cycles, the 5' nuclease activity of the polymerase, for example, AmpliTaq, results in the cleavage of the TaqMan probe. This cleavage separates the 5' fluorescent dye and the 3' quenching agent, thereby resulting in an increase in fluorescence as a function of amplification (see, for example, http://www2.perkin-elmer.com).

Other suitable amplification methods include, but are not limited to ligase chain reaction (LCR) (see Wu and Wallace (1989) Genomics 4:560, Landegren et al. (1988) Science 241:1077, and Barringer et al. (1990) Gene 89:117), transcription amplification (Kwoh et al. (1989) Proc. Natl. Acad. Sci. USA 86:1173), self-sustained sequence replication (Guatelli et al. (1990) Proc. Nat. Acad. Sci. USA 87:1874), dot PCR, and linker adapter PCR, etc. *Hybridization-Based Assays*

Hybridization assays can be used to detect RTK copy number. Hybridization-based assays include, but are not limited to, traditional "direct probe" methods such as Southern blots or in situ hybridization (e.g., FISH), and "comparative probe" methods such as comparative genomic hybridization (CGH). The methods can be used in a wide variety of formats including, but not limited to substrate--(e.g. membrane or glass) bound methods or array-based approaches as described below.

### Southern Blot

One method for evaluating the copy number of RTK encoding nucleic acid in a sample involves a Southern transfer. Methods for doing Southern Blots are known to those of skill in the art (see Current Protocols in Molecular Biology, Chapter 19, Ausubel, et al., Eds., Greene Publishing and Wiley-Interscience, New York, 1995, or Sambrook et al., Molecular Cloning: A Laboratory Manual, 2d Ed. vol. 1-3, Cold Spring Harbor Press, NY, 1989). In such an assay, the genomic DNA (typically fragmented and separated on an electrophoretic gel) is hybridized to a probe specific for the target region. Comparison of the intensity of the hybridization signal from the probe for the target region with control probe signal from analysis of normal genomic DNA (e.g., a non-amplified portion of the same or related cell, tissue, organ, etc.) provides an estimate of the relative copy number of the target nucleic acid. An intensity level that is higher than the control is indicative of amplified RTK.

### Fluorescence In Situ Hybridization (FISH)

In another embodiment, FISH is used to determine the copy number of the RTK gene in a sample. Fluorescence in situ hybridization (FISH) is known to those of skill in the art (see Angerer, 1987 Meth. Enzymol., 152: 649). Generally, in situ hybridization comprises the following major steps: (1) fixation of tissue or biological structure to be analyzed; (2) prehybridization treatment of the biological structure to increase accessibility of target DNA, and to reduce nonspecific binding; (3) hybridization of the mixture of nucleic acids to the nucleic acid in the biological structure or tissue; (4) post-hybridization washes to remove nucleic acid fragments not bound in the hybridization, and (5) detection of the hybridized nucleic acid fragments.

In a typical in situ hybridization assay, cells or tissue sections are fixed to a solid support, typically a glass slide. If a nucleic acid is to be probed, the cells are typically denatured with heat or alkali. The cells are then contacted with a hybridization solution at a moderate temperature to permit annealing of labeled probes specific to the nucleic acid sequence encoding the protein. The targets (e.g., cells) are then typically washed at a predetermined stringency or at an increasing stringency until an appropriate signal to noise ratio is obtained.

The probes used in such applications are typically labeled, for example, with radioisotopes or fluorescent reporters. Preferred probes are sufficiently long, for example, from about 50, 100, or 200 nucleotides to about 1000 or more nucleotides, to enable specific hybridization with the target nucleic acid(s) under stringent conditions.

In some applications it is necessary to block the hybridization capacity of repetitive sequences. Thus, in some embodiments, tRNA, human genomic DNA, or Cot-1 DNA is used to block non-specific hybridization. Thus, In certain embodiments of the present invention, the presence or absence of RTK amplification is determined by FISH.

### Comparative Genomic Hybridization (CGH)

In comparative genomic hybridization methods, a "test" collection of nucleic acids (e.g. from a tumor or cancerous cells) is labeled with a first label, while a second collection (e.g. from a normal cell or tissue) is labeled with a second label. The ratio of hybridization of the nucleic acids is determined by the ratio of the first and second labels binding to each fiber in an array. Differences in the ratio of the signals from the two labels, for example, due to gene amplification in the test collection, is detected and the ratio provides a measure of the gene copy number, corresponding to the specific probe used. A cytogenetic representation of DNA copy-number variation can be generated by CGH, which provides fluorescence ratios along the length of chromosomes from differentially labeled test and reference genomic DNAs. In another embodiment of the present invention, comparative genomic hybridization may be used to detect the presence or absence of RTK amplification.

### Microarray based comparative genomic hybridization

In an alternative embodiment of the present invention, DNA copy numbers are analyzed via microarray-based platforms. Microarray technology offers high resolution. For example, the traditional CGH generally has a 20 Mb limited mapping resolution; whereas in microarray-based CGH, the fluorescence ratios of the differentially labeled test and reference genomic DNAs provide a locus-by-locus measure of DNA copy-number variation, thereby achieving increased mapping resolution. Details of various microarray methods can be found in the literature. See, for example, U.S. Pat. No. 6,232,068; Pollack et al., Nat. Genet., 23(1):41-6, (1999), Pastinen (1997) Genome Res. 7: 606-614; Jackson (1996) Nature Biotechnology 14:1685; Chee (1995) Science 274: 610; WO 96/17958, Pinkel et al. (1998) Nature Genetics 20: 207-211 and others.

The DNA used to prepare the arrays of the invention is not critical. For example, the arrays can include genomic DNA, e.g. overlapping clones that provide a high resolution scan of a portion of the genome containing the desired gene, or of the gene itself. Genomic nucleic acids can be obtained from, e.g., HACs, MACs, YACs, BACs, PACs, PIs, cosmids, plasmids, inter-Alu PCR products of genomic clones, restriction digests of genomic clones, cDNA clones, amplification (e.g., PCR) products, and the like. Arrays can also be produced using oligonucleotide synthesis technology. Thus, for example, U.S. Pat. No. 5,143,854 and PCT Patent Publication Nos. WO 90/15070 and WO 92/10092 teach the use of light-directed combinatorial synthesis of high density oligonucleotide arrays.

Hybridization protocols suitable for use with the methods of the invention are described, e.g., in Albertson (1984) EMBO J. 3: 1227-1234; Pinkel (1988) Proc. Natl. Acad. Sci. USA 85: 9138-9142; EPO Pub. No. 430,402; Methods in Molecular Biology, Vol. 33: In Situ Hybridization Protocols, Choo, ed., Humana Press, Totowa, N.J. (1994), Pinkel et al. (1998) Nature Genetics 20: 207-211, or of Kallioniemi (1992) Proc. Natl. Acad Sci USA 89:5321-5325 (1992), etc.

The sensitivity of the hybridization assays may be enhanced through use of a nucleic acid amplification system that multiplies the target nucleic acid being detected. Examples of such systems include the polymerase chain reaction (PCR) system and the ligase chain reaction (LCR) system. Other methods recently described in the art are the nucleic acid sequence based amplification (NASBAO, Cangene, Mississauga, Ontario) and Q Beta Replicase systems.

### Localization of RTK and RTK ligand

In some embodiments, an indicator of RTK activation is an increase in RTK cell-surface expression. RTK cell surface expression can be detected in a biological sample, for example, by staining with labeled antibodies that target the extracellular domain of an RTK. Labels can be detected by FACS analysis essentially as described by Picker et al., J. Immunol. 150:1105-1121 (1993). In certain embodiments, at least 5, 10, 20, 30, 50, 70, or 100% more RTK protein is expressed at the cell surface of a cancer cell compared to control cells. In certain embodiments the control cell is from the same tissue as the cancer cell and is from i) a non-cancerous cell from the same subject or ii) a non-cancerous cell from a different subject.

In some embodiments, an indicator of RTK activation is an increase in RTK ligand secretion. Ligand secretion can be measured by various techniques known in the art. In certain embodiments, an aliquot of tissue culture medium from a cancer cell culture is probed for the presence of RTK ligand using various techniques known in the art, such as enzyme linked immunosorbent assay (ELISA), immunoprecipitation, immunofluorescence, enzyme immunoassay (EIA), radioimmunoassay (RIA), and Western blot analysis.

### RTK activity

In some embodiments, an indicator of RTK activation is an increase in RTK activity, such as biochemical activity. RTK activity encompasses all enzymatic and signaling functions of an RTK, including kinase domain autophosphorylation, target binding, and target phosphorylation. An increase in RTK activity may result e.g., from one or more mutations in an RTK gene, from an increase in RTK RNA expression level, RTK protein expression, from RTK gene amplification, or from increased ligand interaction.

In certain embodiments, the presence of one or more indicators of RTK activation is determined by measuring RTK activity levels. RTK activity includes changes in the activation state of targets downstream of RTK activation, changes in binding to co-receptors as well as changes to binding downstream targets. Another method to quantify RTK activity is by determining the phosphorylation state of RTKs, in particular in the tyrosine kinase domain.

Various antibodies, including many that are commercially available, have been produced which specifically bind to phosphorylated, activated isoforms of RTKs and are referred to herein as kinase activation-state antibodies.

In certain embodiments, the presence of one or more indicators of RTK activation state is determined using a multiplicity of activation state antibodies that are immobilized. Antibodies may be non-diffusibly bound to an insoluble support having isolated sample receiving areas (e.g. a microtiter plate, an array, etc.). The insoluble supports may be made of any composition to which the compositions can be bound, is readily separated from soluble material, and is otherwise compatible with the overall method of screening. The surface of such supports may be solid or porous and of any convenient shape. Examples of suitable insoluble supports include microtiter plates, arrays, membranes, and beads. These are typically made of glass, plastic (e.g., polystyrene), polysaccharides, nylon or nitrocellulose, teflon^{™}, etc. Microtiter plates and arrays are especially convenient because a large number of assays can be carried out simultaneously, using small amounts of reagents and samples. In some cases magnetic beads and the like are included.

The particular manner of binding of the composition is not crucial so long as it is compatible with the reagents and overall methods of the invention, maintains the activity of the composition and is nondiffusable. Preferred methods of binding include the use of antibodies (which do not sterically block either the ligand binding site or activation sequence when the protein is bound to the support), direct binding to "sticky" or ionic supports, chemical crosslinking, the synthesis of the antibody on the surface, etc. Following binding of the antibody, excess unbound material is removed by washing. The sample receiving areas may then be blocked through incubation with bovine serum albumin (BSA), casein or other innocuous protein or other moiety.

In certain embodiments, an epitope-recognizing fragment of an activation state antibody rather than the whole antibody is used. In another preferred embodiment, the epitope-recognizing fragment is immobilized. In another preferred embodiment, the antibody light chain which recognizes an epitope is used. A recombinant nucleic acid encoding a light chain gene product which recognizes an epitope may be used to produce such an antibody fragment by recombinant means well known in the art.

A chip analogous to a DNA chip can be used in the methods of the present invention. Arrayers and methods for spotting nucleic acid to a chip in a prefigured array are known. In addition, protein chips and methods for synthesis are known. These methods and materials may be adapted for the purpose of affixing activation state antibodies to a chip in a prefigured array.

In certain embodiments, such a chip comprises a multiplicity of kinase activation state antibodies, and is used to determine a receptor tyrosine kinase activation state profile for a sample. In certain embodiments, detection of activated kinase is by "sandwich assay" as known in the art. Briefly, a sample is passed over the chip under conditions which allow the multiplicity of immobilized kinase activation state antibodies to simultaneously bind to a multiplicity of activated kinases if present in the sample. The immobilized antibody-kinase complex is optionally washed and contacted with a second plurality of antibodies comprising non-activation state antibodies that are capable of specifically binding to activated kinases while the kinases are specifically bound to kinase activation state specific antibodies. Such non-activation state specific antibodies specifically bind to activated kinases via an epitope that is accessible even when a kinase activation state specific antibody is bound. Binding of the non-activation state specific antibodies to the activation state antibody-activated kinase complex is determined and reveals the presence of activated kinase in sample.

The determination of binding of a second antibody in the sandwich assay can be accomplished in many different ways. In certain embodiments, the multiplicity of non-activation state specific antibodies are labeled to facilitate detection. By "label" is meant a molecule that can be directly (i.e., a primary label) or indirectly (i.e., a secondary label) detected; for example a label can be visualized and/or measured or otherwise identified so that its presence or absence can be known. A compound can be directly or indirectly conjugated to a label which provides a detectable signal, e.g. radioisotope, fluorescers, enzyme, antibodies, particles such as magnetic particles, chemiluminescers, or specific binding molecules, etc. Specific binding molecules include pairs, such as biotin and streptavidin, digoxin and antidigoxin etc.

In certain embodiments, the presence of one or more indicators of RTK activation is determined using an antibody array as described in U.S. Patent No. 6,197,599. Commercially available phosphorylated-RTK antibody arrays include the RayBio^{™} Phosphorylation Antibody Array and R&G System's Phospo-RTK Array.

In certain embodiments, the presence of one or more indicators of RTK activation is determined using an array, such as an anti-phospho-RTK antibody array or an anti-RTK antibody array wherein an anti-phosphotyrosine antibody is used to detect phosphorylation.

In some embodiments, the presence of indicators of RTK activation is determined for one or more of the following RTKs: ALK, Axl, ERBB receptors (e.g., EGFR, ERBB2, ERBB3, ERBB4), erythropoietin-producing hepatocellular (EPH) receptors (e.g., EphA1, EphA2, EphA3, EphA4, EphA5, EphA6, EphA7, EphA8, EphB1, EphB2, EphB3, EphB4, EphB5, EphB6), fibroblast growth factor (FGF) receptors (e.g., FGFR1, FGFR2, FGFR3, FGFR4, FGFR5), Fgr, IGFIR, Insulin R, LTK, M-CSFR, MUSK, platelet-derived growth factor (PDGF) receptors (e.g., PDGFR-A, PDGFR-B), RET, ROR1, ROR2, ROS, RYK, vascular endothelial growth factor (VEGF) receptors (e.g., VEGFR1/FLT1, VEGFR2/FLK1, VEGF3), tyrosine kinase with immunoglobulin-like and EGF-like domains (TIE) receptors (e.g., TIE-1, TIE-2/TEK), Tec, TYRO10, insulin-like growth factor (IGF) receptors (e.g., INS-R, IGF-1R, IR-R), Discoidin Domain (DD) receptors (e.g., DDR1, DDR2), receptor for c-Met (MET), recepteur d'origine nantais (RON); also known as macrophage stimulating 1 receptor, Flt3 fins-related tyrosine kinase 3 (Flt3), colony stimulating factor 1(CSF1) receptor, adhesion related kinase receptor (e.g., Axl), receptor for c-kit (KIT, or SCFR) and insulin receptor related (IRR) receptors.

In some embodiments of the methods of the invention, the presence of indicators of RTK activation is determined for at least 2, 3, 4, 5, 10, 15, 20, 25, 30, 40, 50 or more RTKs. In certain embodiments, the presence of indicators of RTK activation is determined for EGFR and MET. In certain embodiments, the presence of indicators of RTK activation is not determined for MET. In certain embodiments, the presence of indicators of RTK activation is determined for EGFR or MET (but not of both), together with other RTKs.

In certain embodiments, the activated RTK is EGFR. In some embodiments, the indicator of RTK activation is a mutation in the EGFR gene is located in or within exons 18-21 of EGFR. In certain embodiments, the indicator of RTK activation is a mutation located in the kinase domain of EGFR. In certain embodiments, the indicator of RTK activation is a mutation of G719A, E746K, L747S, E749Q, A750P, A755V, V765M, S768I, L858P, E746-R748 del, R748-P753 del, M766-A767 AI ins, S768-V769 SVA ins, P772-H773 NS ins. 2402G>C; 2482G>A; 2486T>C; 2491G>C; 2494G>C; 2510C>T; 2539G>A; 2549G>T; 2563C>T; 2819T>C; 2482-2490 del; 2486-2503 del; 2544-2545 ins GCCATA; 2554-2555 ins CCAGCGTGG; and 2562-2563 ins AACTCC. (The EGFR Genbank accession number is NP_005219).

In certain embodiments, the activated RTK is MET. In some embodiments, the indicator of RTK activation is a mutation in the MET gene kinase domain. In certain embodiments, the indicator of RTK activation is a mutation that results in an amino acid change at any of positions N375, I638, V13, V923, 316 and E168 relative to wild type MET. In some embodiments, the indicator of RTK activation is the S249C mutation in FGFR3 (Logie, A., Human Molecular Genetics 2005 14(9):1153-1160). In some embodiments, the indicator of RTK activation is a mutation in Tie2 as described in Vikkula M, et al., 1996 Cell 87: 1181 1190. In some embodiments, the indicator of RTK activation is a mutation in c-KIT as described in Longley, B. J., et al. Nature Genetics,12:312-314 (1996).

In one aspect, a method of designing a therapeutic treatment is provided, the method comprising i) obtaining a biological sample of a subject, such as one provided from a caregiver, determining the presence of one or more indicators or RTK activation in a biological sample from the subject, ii) selecting a group of one or more RTK inhibitors, wherein the group can inhibit the activity of two or more RTKs that display one or more of the indicators of RTK activation, and iii) transmitting a descriptor of the selected group of RTK inhibitors to e.g., a caregiver. The transmission may occur directly or by means of one or more third parties. In certain embodiments, the description is transmitted across a network.

The term caregiver encompasses any group, organization, or individual who is responsible (e.g. contractually, legally or morally) for the care of the subject, including medically-trained personnel, social workers, friends, guardians, and relatives of a subject, or the subject him/herself.

The application also provides methods for treating cancer. In some aspects, the methods comprise determining the presence of one or more indicators of receptor tyrosine kinase (RTK) activation in a biological sample from the subject and administering to the subject a group of one or more RTK inhibitors, wherein the group inhibits the activity of at least two RTKs that display one or more of the indicators.

In certain embodiments, the group of one or more RTK inhibitors can inhibit the activity of at least 3, 4, 5, 6, 7, 8, 9, 10, 15, 20, or more RTKs that display one or more indicators. In certain embodiments, an RTK displays at least 1, 2, 3, 4 or more indicators of activation. In certain embodiments, an RTK displays an indicator of RTK activity, such as, for example increased phosphorylation. In certain embodiments, an RTK displays an indicator of RTK amplification.

Combination therapies comprising one or more RTK inhibitors may refer to (1) pharmaceutical compositions that comprise a coformulation of at least two RKT inhibitors; and (2) co-administration of one or more RTK inhibitors wherein inhibitors have not been formulated in the same compositions (but may be present within the same kit or package, such as a blister pack or other multi-chamber package; connected, separately sealed containers (e.g., foil pouches) that can be separated by the user; or a kit where the therapeutic agents are in separate vessels). When using separate formulations, the RTK inhibitors may be administered at the same time (simultaneously), sequentially, intermittently, staggered, or various combinations of the foregoing.

The group of RTK inhibitors may be administered in a therapeutically effective amount to inhibit activity of at least two RTKs that display one or more of the indicators. The RTK inhibitors may be administered in a therapeutically effective amount sufficient to decrease tumor growth, to induce apoptosis of tumor cells or to alleviate one or more symptoms of cancer.

The methods described are for treating subjects with one or more indicators of EGFR activation, of MET activation, or both EGFR and MET activation, or neither EGFR nor MET activation or only one of EGFR or MET activation.

In some aspects of the invention, methods are provided for designing therapeutic treatment for subjects afflicted with cancer, including primary tumors, secondary tumors, metastases, acral lentiginous melanoma, actinic keratoses, adenocarcinoma, adenoid cycstic carcinoma, adenomas, adenosarcoma, adenosquamous carcinoma, adrenocortical carcinoma, AIDS-related lymphoma, anal cancer, anaplastic glioma, astrocytic tumors, astrocytomas, bartholin gland carcinoma, basal cell carcinoma, biliary tract cancer, bone cancer, bile duct cancer, bladder cancer, brain stem glioma, brain tumors, breast cancer, bronchial gland carcinomas, capillary carcinoma, carcinoids, carcinoma, carcinosarcoma, cavernous, central nervous system lymphoma, cerebral astrocytoma, cervical cancer, connective tissue cancer, cholangiocarcinoma, chondosarcoma, choriod plexus papilloma/carcinoma, clear cell carcinoma, colon cancer, colorectal cancer, cutaneous T-cell lymphoma, cystadenoma, endodermal sinus tumor, endometrial hyperplasia, endometrial stromal sarcoma, endometrioid adenocarcinoma, ependymal, ependymoma, epitheloid, esophageal cancer, Ewing's sarcoma, extragonadal germ cell tumor, eye cancer, fibrolamellar, focal nodular hyperplasia, gallbladder cancer, gangliogliomas , gastric cancer, gastrinoma, germ cell tumors, gestational trophoblastic tumor, glioblastoma multiforme, glioma, glucagonoma, head and neck cancer, hemangiblastomas, hemangioendothelioma, hemangiomas, hepatic adenoma, hepatic adenomatosis, hepatocellular carcinoma, Hodgkin's lymphoma, hypopharyngeal cancer, hypothalamic and visual pathway glioma, childhood, insulinoma, intaepithelial neoplasia, interepithelial squamous cell neoplasia, intraocular melanoma, intra-epithelial neoplasm, invasive squamous cell carcinoma, large cell carcinoma, islet cell carcinoma, Kaposi's sarcoma, kidney cancer, laryngeal cancer, leiomyosarcoma, lentigo maligna melanomas, leukemia-related disorders, lip and oral cavity cancer, liver cancer, lung cancer, lymphoma, malignant mesothelial tumors, malignant thymoma, medulloblastoma, medulloepithelioma, melanoma, meningeal, merkel cell carcinoma, mesothelial, metastatic carcinoma, mucoepidermoid carcinoma, multiple myeloma/plasma cell neoplasm, mycosis fungoides, myelodysplastic syndrome, myeloproliferative disorders, nasal cavity and paranasal sinus cancer, nasopharyngeal cancer, neuroblastoma, neurofibromatosis, neuroepithelial adenocarcinoma nodular melanoma, non-Hodgkin's lymphoma, non-small cell lung cancer, oat cell carcinoma, oligodendroglial, oligoastrocytomas, oral cancer, oropharyngeal cancer, osteosarcoma, pancreatic polypeptide, ovarian cancer, ovarian germ cell tumor, pancreatic cancer, papillary serous adenocarcinoma, pineal cell, pituitary tumors, plasmacytoma, pseudosarcoma, pulmonary blastoma, parathyroid cancer, penile cancer, pheochromocytoma, pineal and supratentorial primitive neuroectodermal tumors, pituitary tumor, plasma cell neoplasm, pleuropulmonary blastoma, prostate cancer, rectal cancer, renal cell carcinoma, cancer of the respiratory system, retinoblastoma, rhabdomyosarcoma, sarcoma, serous carcinoma, skin cancer, small cell carcinoma, small intestine cancer, soft tissue carcinomas, somatostatin-secreting tumor, squamous carcinoma, squamous cell carcinoma, stomach cancer, stromal tumors, submesothelial, superficial spreading melanoma, supratentorial primitive neuroectodermal tumors, testicular cancer, thyroid cancer, undifferentiatied carcinoma, urethral cancer, uterine sarcoma, uveal melanoma, verrucous carcinoma, vaginal cancer, vipoma, vulvar cancer, Waldenstrom's macroglobulinemia, well differentiated carcinoma, and Wilm's tumor.

In one aspect, the cancer is glioblastoma multiforme.

Methods are described comprising determining the presence of one or more indicators of RTK activation and selecting or administering a group of one or more RTK inhibitors, wherein the group can inhibit or inhibits the activity of at least two RTKs that display one or more of the indicators. One inhibitor may be selected that inhibits/can inhibit at least two RTKs. A first inhibitor may be selected that inhibits/can inhibit at least one RTK and a second inhibitor is selected that inhibits/can inhibit at least one RTK that is different from the first RTK. At least two, at least three, at least four, at least five, at least six, at least seven, at least eight, at least nine, at least ten or more inhibitors may be selected. Each RTK can be inhibited by more that one inhibitor and each inhibitor can inhibit more that one RTK, so long as at least two RTKs are inhibited by the selected group of inhibitors. In certain embodiments, each RTK that is targeted for treatment displays at least 1, 2, 3, 4, 5, 6, 7, or more indicators of activation.

The methods described herein in certain instances may be useful for replacing existing surgical procedures or drug therapies, although in most instances the method of the present invention is useful in improving the efficacy of existing therapies. Accordingly, combination therapy may be used to treat subjects that are undergoing or that will undergo a treatment for, inter alia, cancer. For example, the group of RTK inhibitors can be administered in conjunction with anti-proliferative agents. The inhibitors also can be administered in conjunction with nondrug treatments, such as surgery, radiation therapy, chemotherapy, immunotherapy and diet/exercise regimens. The other therapy may be administered before, concurrent with, or after treatment with the inhibitors. There may also be a delay of several hours, days and in some instances weeks between the administration of the different treatments, such that the inhibitors may be administered before or after the other treatment.

The selected group of RTK inhibitors may be administered in combination with (i.e., before, during or after) other anti-cancer therapy, and in particular other anti-cancer therapy that does not comprise the administration of RTK pathway inhibitors to the subject. For example, the agent may be administered together with any one or more of the chemotherapeutic drugs known to those of skill in the art of oncology, (Reference: Cancer, Principles & Practice of Oncology, DeVita, V. T., Hellman, S., Rosenberg, S. A., 6th edition, Lippincott-Raven, Philadelphia, 2001), such as, merely to illustrate: abarelix, adriamycin, aldesleukin, altretamine, aminoglutethimide, amsacrine, anastrozole, antide, arimidex, asimicin, asparaginase, AZD2171 (Recentin^{™}), Bacillus Calmette-Guerin/BCG (TheraCys^{™}, TICE^{™}), bevacizumab (Avastin^{™}), bicalutamide, bleomycin, bortezomib (Velcade^{™}), bullatacin, buserelin, busulfan, campothecin, capecitabine, carboplatin, carmustine, cetuximab (Erbitux^{™}), chlorambucil, chlorodeoxyadenosine cisplatin, cladribine, clodronate, colchicine, cyclophosphamide, cyproterone, cytarabine, dacarbazine, dactinomycin, dasatinib (Sprycel^{™}), daunorubicin, dienestrol, diethylstilbestrol, discodermolide, dexamethasone, docetaxel (Taxotere^{™}), doxorubicin, Abx-EGF, epothilones, epirubicin, erlonitib (Tarceva^{™}), estradiol, estramustine, etoposide, exemestane, floxuridine, 5-fluorouracil, filgrastim, flavopiridol, fludarabine, fludrocortisone, fluorouracil, fluoxymesterone, flutamide, fulvestrant, gefitinib (Iressa^{™}), gemcitabine (Gemzar^{™}), genistein, goserelin, guanacone, hydroxyurea, idarubicin, ifosfamide, imatinib mesylate (Gleevac^{™}), interferon, interleukins, irinotecan, ibritumomab (Zevalin^{™}), ironotecan, ixabepilone (BMS-247550), lapatinib (Tykreb^{™}), letrozole, leucovorin, leuprolide, levamisole, lomustine, mechlorethamine, medroxyprogesterone, megestrol, melphalan, mercaptopurine, mesna, methotrexate, mithramycin, mitomycin, mitotane, mitoxantrone, mitozolomide, nilutamide, nocodazole, octreotide, oxaliplatin, paclitaxel (Taxol^{™}), pamidronate, pegaspargase, pentostatin, plicamycin, porfimer, prednisone, procarbazine, raltitrexed (Tomudex^{™}), rapamycin, ramptothecin, rituximab (Rituxan^{™}), rolliniastatin, sorafenib (Nexavar^{™}/Bayer BAY43-9006), squamocin, squamotacin, streptozocin, suramin, sunitinib malate (Sutent^{™}), tamoxifen, temsirolimus (CC1-779), temozolomide (Temodar^{™}), teniposide, testosterone, thioguanine, thiotepa, titanocene dichloride, topotecan, toremifene, tositumomab (Bexxar^{™}), trastuzumab, tretinoin, VEGF Trap , vinblastine, vincristine, vindesine, and vinorelbine, zoledronate.

The methods of treatment may further comprise administering one or more DNA damaging agents. The DNA damaging agents may be administered before, concurrent with, or after treatment with the RTK inhibitors. The DNA damaging agents may be administered simultaneously or sequentially with the RTK inhibitors. In certain embodiments, the DNA damaging agents are selected from radiation, a chemotherapeutic, cyclophosphamide, melphalan, busulfan, chlorambucil, mitomycin, cisplatin, bleomycin, irinotecan, mitoxantrone, dactinomycin, temozolomide, gemcitibine, or a combination thereof. In certain embodiments of the methods of treatment, RTK inhibitors are administered conjointly with temozolimide and radiation therapy in the treatment of glioblastoma multiforme. In certain embodiments, RTK inhibitors are administered conjointly with temozolomide and gemcitibine in the treatment of pancreatic cancer.

A method of treating a subject afflicted with cancer may be provided as described herein, wherein the subject is Mer+, i.e., expresses elevated levels of methylguanine-DNA methyltransferase (O6MeGMT). In certain embodiments, the method further comprises the administration of temozolomide or other imidazotetrazinone mitozolomide related molecule.

Surgical methods for treating cancer include intra-abdominal surgeries such as right or left hemicolectomy, sigmoid, subtotal or total colectomy and gastrectomy, radical or partial mastectomy, prostatectomy and hysterectomy. A group of one or more RTK inhibitors may be administered locally to an area of cancerous mass after or during surgical removal of a tumor. The group of inhibitors may be administered systemically.

In addition, a group of one or more RTK inhibitors can be administered together with any form of radiation therapy including external beam radiation, intensity modulated radiation therapy (IMRT) and any form of radiosurgery including Gamma Knife, Cyberknife, Linac, and interstitial radiation (e.g. implanted radioactive seeds, GliaSite balloon). A group of one or more RTK inhibitors may be administered during a surgical procedure, such as, for example, during the removal of a tumor or a tumor biopsy.

The methods of treatment may reduce the growth of the cancer in the afflicted subject. A reduction in growth refers to any decrease in the size of a group of cancer cells or a tumor following administration of a group of one or more RTK inhibitors relative to the size of the group of cancer cells or tumor prior to administration. A group of cancer cells or tumor may be considered to be reduced in size or regressed if it is at least 5, 10, 15, 20, 30, 50, 75, or 99% smaller, or having no detectable cancer cells or tumor remaining. Tumor size is measured either directly or in vivo (i.e., by measurement of the group of cancer cells or a tumor which is directly accessible to physical measurement, such as by calipers) or by examination of the size of an image of the tumor produced, for example, by X-ray or magnetic resonance imaging or by computerized tomography, or from the assessment of other optical data (e.g., spectral data). For a group of cancer cells, such as a group of leukemia cells, a reduction in number can be determined by measuring the absolute number of leukemia cells in the circulation of a subject, or a reduction in the amount of a cancer cell-specific antigen.

The methods of treatment may reduce one or more of symptoms associated with cancer. Exemplary symptoms are anorexia, cognitive dysfunction, depression, dyspnea, fatigue, hormonal disturbances, neutropenia, pain, peripheral neuropathy, and sexual dysfunction. The symptoms associated with cancer may vary according to the type of cancer. For example, symptoms associated with -cervical cancer include, for example, abnormal bleeding, unusual heavy vaginal discharge, pelvic pain that is not related to the normal menstrual cycle, bladder pain or pain during urination, and -bleeding between regular menstrual periods, after sexual intercourse, douching, or pelvic exam. Symptoms associated with lung cancer, however, may include persistant cough, coughing up blood, shortness of breath, wheezing chest pain, loss of appetite, losing weight without trying and fatigue. Symptoms for liver cancer may include, for example, loss of appetite and weight, abdominal pain, especially in the upper right part of the abdomen that may extend into the back and shoulder, nausea and vomiting, general weakness and fatigue, an enlarged liver, abdominal swelling (ascites), and a yellow discoloration of the skin and the whites of eyes (jaundice). One skilled in oncology may easily determined which symptoms are associated with each cancer type.

Described herein is a method for evaluating a candidate RTK inhibitor, the method comprising determining the presence of one or more indicators of RTK activation in a population of subjects, selecting subjects having similar RTK activation profiles, and administering the candidate RTK inhibitor to one or more of the selected subjects in combination with at least one additional RTK inhibitor that targets a different RTK that displays one or more indications of activation in the selected population.

Subjects having similar RTK activation profiles may share at least 2, 3, 4, 5, 6, 7, 8, 9, 10, or more RTKs in common that display one or more indicators of activation. Subjects having similar RTK activation profiles may share in common at least one or more of the following RTKs displaying one or more indicators of activation: MET, EGFR, ERBB2, ERBB3, VEGFR2, PDGFRA. Subjects having similar RTK activation profiles may share in common at least 2, 3, 4, 5, 6, 7, 8, 9, 10, 15, 20, 25, 30, 40, 50, 60 or more RTKs that do not display one or more indicators of activation. Subjects having similar RTK activation profiles may not demonstrate one or more indicators of RTK activation for EGFR, MET, or both.

Also described is a method for evaluating a candidate RTK inhibitor, the method comprising determining the presence of one or more indicators of RTK activation in a population of subjects, selecting subjects that are more likely to be sensitive to the candidate RTK inhibitor based on the RTK activation profile, and administering the candidate RTK inhibitor to one or more of the selected subjects in combination with at least one additional RTK inhibitor that targets a different RTK that displays one or more indications of activation in the selected population. It is within the purview of a skilled person to predict likelihood of sensitivity based on the RTK activation profile. By way of example, a subject demonstrating one or more indicators of activation in the EGFR is likely to be sensitive to a candidate EGFR inhibitor. Assays to determine which RTKs a candidate RTK inhibitor is likely to inhibit are well known in the art.

Further, described is a method for evaluating a candidate RTK inhibitor, the method comprising determining the presence of one or more indicators of RTK activation in a population of subjects, administering the candidate RTK inhibitor to one or more of the selected subjects, and correlating the effectiveness of the candidate RTK inhibitor with an RTK activation profile from the one or more subjects. It is within the purview of a skilled person to correlate the effectiveness of treatment with a subject's activation profile. The method may further comprises the administration of at least one additional RTK inhibitor that targets a different RTK than the candidate RTK inhibitor and displays one or more indications of activation in the selected subjects.

At least one biological sample from a subject may be obtained before the treatment and at least one biological sample from the subject is obtained after initiation of the treatment. Several samples from the individual may be obtained at different time points after initiation of treatment. This enables monitoring the course of treatment during a prolonged period. This is, for instance, useful for establishing appropriate treatment schedules, dosage and type on a patient-per-patient basis. Furthermore, it can be determined whether continuation of treatment at a given time point is appropriate.

The population of subjects may be afflicted with cancer. The subjects in the population may be afflicted with the same cancer. The cancer may be glioblastoma multiforme.

The subject may be human. The human may be a male or a female, a newborn, toddler, child, teenager, adult or an elderly (>65 years) subject.

Also described is a method for reducing PI3K-mediated signaling in a cancer cell, the method comprising determining the presence of one or more indicators of RTK activation in the cancer cell and contacting the cancer cell with a group of one more RTK inhibitors, wherein the group inhibits the activity of at least two RTKs that display one ore more of the indicators.

Described herein is further a method for reducing AKT phosphorylation in a cancer cell, the method comprising determining the presence of one or more indicators of RTK activation in the cancer cell and contacting the cancer cell with a group of one more RTK inhibitors, wherein the group inhibits the activity of at least two RTKs that display one ore more of the indicators.

Assays for PI3-kinase activity are known in the art. For example, U.S. Pat. Pub. 2005/0170439 describes methods to determine binding complex between RTK and PI3 kinase. Alternatively, different phosphorylated forms of proteins that are substrates for PI3-kinase can be assayed using antibodies that are specific for those forms. Antibodies that are specific for the various phosphorylated forms of PKB/AKT are commercially available,(e.g., New England Biolabs (UK) Ltd of Hitchin, Hertfordshire, who supply the following antibodies: catalogue number 9272 can be used to measure total levels of phosphorylated and un-phosphorylated PKB/AKT, antibody catalogue number 9271 can be used to measure levels of PKB/AKT phosphorylated at Ser473, antibody catalogue number 9275 can be used to measure levels of PKB/AKT phosphorylated at Thr308. Other suitable antibodies will be known to those of skill in the art. Immunoassays to measure these proteins can be carried out in many different and convenient ways, as is well known to those skilled in the art. Alternatively, appropriately labeled antibody could be used to visualize unphosphorylated or phosphorylated forms of PKB/AKT in adherent cells in flat-bottomed wells or discrete regions of a slide that may have received micro-dispensed treatments scanned with a fluorescence microscope.

Described herein is also a method for reducing proliferation in a cancer cell, the method comprising determining the presence of one or more indicators of RTK activation in the cancer cell and contacting the cancer cell with a group of one more RTK inhibitors, wherein the group inhibits the activity of at least two RTKs that display one ore more of the indicators.. A reduction in the proliferation of a cancer cell can be established by monitoring the number of cancer cells and/or the size of a tumor. In certain embodiments, the number of cancer cells are reduced by at least 5, 10, 15, 20, 30, 50, 75, or 99%.

Also described is a method for increasing cell death in a cancer cell or group of cancer cells, the method comprising determining the presence of one or more indicators of RTK activation in the cancer cell and contacting the cancer cell with a group of one more RTK inhibitors, wherein the group inhibits the activity of at least two RTKs that display one ore more of the indicators. In some embodiments, an increase in cell death reduces the number of cancer cells in a subject or tumor size. In certain embodiments, the number of cancer cells are reduced by at least 5, 10, 15, 20, 30, 50, 75, or 99%.

Exemplary methods for determining cell growth and/or proliferation and/or apoptosis include, for example, Alamar Blue, Brd U, MTT, Trypan Blue exclusion, ³H-thymidine incorporation, and XTT assays. Kits for determining cell growth and/or proliferation and/or apoptosis are commercially available from a variety of sources. In certain embodiments, it may be desirable to compare the level of growth and/or proliferation and/or apoptosis in the cancer cell to a control, e.g., a cell that has not been contacted with a group of one or more RTK inhibitors, or a cell that has been contacted with a different amount of inhibitors, or a reference value, such as an expected value for a given assay, etc.

The application further describes a method for reducing tumor maintenance or progression, the method comprising determining the presence of one or more indicators of RTK activation in the cancer cell and contacting the cancer cell with a group of one more RTK inhibitors, wherein the group inhibits the activity of at least two RTKs that display one ore more of the indicators.

In certain embodiments of the methods, PTEN is disabled. In certain embodiments, no detectable PTEN protein is expressed in a cancer cell. In certain embodiments, PTEN activity is reduced at least 10, 20, 30, 40, 50, 60, 70, 80, 90% or more in a cancer cell as compared to a non-cancer cell.

### D. RTK inhibitors

The invention provides methods comprising the selection of a group of one or more RTK inhibitors. In some embodiments, RTK inhibitors useful for the methods include small molecules, polymers, sugars and other macromolecules, polypeptides (including antibodies), or nucleic acids (including antisense nucleic acids, ribozymes, and small interfering RNAs or siRNAs). An RTK inhibitor encompasses any composition that modulates, affects, alters, inhibits or reduces the activity of an RTK, including target binding, enzymatic activity or tyrosine phosphorylation action of a tyrosine kinase, preferably by at least 5, 10, 20, 30, 40, 50, 60, 70, 80, 90, 95, 97, 98, 99 or 100%. RTK inhibitors also encompass inhibitors of RTK ligand expression or activity. In certain embodiments, the inhibitor decreases by at least 5, 10, 20, 30, 40, 50, 60, 70, 80, 90, 95, 97, 98, 99 or 100% the binding between an RTK and its activating-ligand.

RTK inhibitors are well know in the art (e.g., Pinna, LA and Cohen, PTW (eds.) Inhibitors of Protein Kinases and Protein Phosphates, Springer (2004) andAbelson, JN, Simon, MI, Hunter, T, Sefton, BM (eds.) Methods in Enzymology, Volume 201: Protein Phosphorylation, Part B: Analysis of Protein Phosphorylation, Protein Kinase Inhibitors, and Protein Academic Press (2007)).

### Nucleic acid therapeutics

In certain embodiments, one or more of the RTK inhibitors is an antisense nucleic acid that targets the expression of an RTK, an RTK ligand, or another protein affecting the activation state of an RTK. By "antisense nucleic acid," it is meant a non-enzymatic nucleic acid compound that binds to a target nucleic acid by means of RNA-RNA, RNA-DNA or RNA-PNA (protein nucleic acid) interactions and alters the activity of the target nucleic acid (for a review, see Stein and Cheng, 1993 Science 261, 1004 and Woolf et al., U.S. Pat. No. 5,849,902). Typically, antisense molecules are complementary to a target sequence along a single contiguous sequence of the antisense molecule. However, in certain embodiments, an antisense molecule can form a loop and binds to a substrate nucleic acid which forms a loop. Thus, an antisense molecule can be complementary to two (or more) non-contiguous substrate sequences, or two (or more) non-contiguous sequence portions of an antisense molecule can be complementary to a target sequence, or both. For a review of current antisense strategies, see Schmajuk et al., 1999, J. Biol. Chem., 274, 21783-21789, Delihas et al., 1997, Nature, 15, 751-753, Stein et al., 1997, Antisense N. A. Drug Dev., 7, 151, Crooke, 2000, Methods Enzymol., 313, 3-45; Crooke, 1998, Biotech. Genet. Eng. Rev., 15, 121-157, Crooke, 1997, Ad. Pharmacol., 40, 1-49.

In other embodiments, the RTK-inhibiting compound may be an siRNA. The term "short interfering RNA," "siRNA," or "short interfering nucleic acid," refers to any nucleic acid compound capable of mediating RNAi or gene silencing when processed appropriately be a cell. For example, the siRNA can be a double-stranded polynucleotide molecule comprising self-complementary sense and antisense regions, wherein the antisense region comprises complementarity to a target nucleic acid compound (e.g., an RTK or RTK ligand). The siRNA can be a single-stranded hairpin polynucleotide having self-complementary sense and antisense regions, wherein the antisense region comprises complementarity to a target nucleic acid compound. The siRNA can be a circular single-stranded polynucleotide having two or more loop structures and a stem comprising self-complementary sense and antisense regions, wherein the antisense region comprises complementarity to a target nucleic acid compound, and wherein the circular polynucleotide can be processed either in vivo or in vitro to generate an active siRNA capable of mediating RNAi. The siRNA can also comprise a single stranded polynucleotide having complementarity to a target nucleic acid compound, wherein the single stranded polynucleotide can further comprise a terminal phosphate group, such as a 5'-phosphate (see for example Martinez et al., 2002, Cell., 110, 563-574), or 5',3'-diphosphate.

As described herein, the subject siRNAs are around 19-30 nucleotides in length, and even more preferably 21-23 nucleotides in length. The siRNAs are understood to recruit nuclease complexes and guide the complexes to the target mRNA by pairing to the specific sequences. As a result, the target mRNA is degraded by the nucleases in the protein complex. In a particular embodiment, the 21-23 nucleotides siRNA molecules comprise a 3' hydroxyl group. In certain embodiments, the siRNA constructs can be generated by processing of longer double-stranded RNAs, for example, in the presence of the enzyme dicer. In certain embodiments, the Drosophila in vitro system is used. In this embodiment, dsRNA is combined with a soluble extract derived from Drosophila embryo, thereby producing a combination. The combination is maintained under conditions in which the dsRNA is processed to RNA molecules of about 21 to about 23 nucleotides. The siRNA molecules can be purified using a number of techniques known to those of skill in the art. For example, gel electrophoresis can be used to purify siRNAs. Alternatively, non-denaturing methods, such as non-denaturing column chromatography, can be used to purify the siRNA. In addition, chromatography (e.g., size exclusion chromatography), glycerol gradient centrifugation, affinity purification with antibody can be used to purify siRNAs.

Production of the subject siRNAs can be carried out by chemical synthetic methods or by recombinant nucleic acid techniques. Endogenous RNA polymerase of the treated cell may mediate transcription in vivo, or cloned RNA polymerase can be used for transcription in vitro. As used herein, siRNA molecules of the disclosure need not be limited to those molecules containing only RNA, but further encompasses chemically-modified nucleotides and non-nucleotides. For example, the dsRNAs may include modifications to either the phosphate-sugar backbone or the nucleoside, e.g., to reduce susceptibility to cellular nucleases, improve bioavailability, improve formulation characteristics, and/or change other pharmacokinetic properties. To illustrate, the phosphodiester linkages of natural RNA may be modified to include at least one of a nitrogen or sulfur heteroatom. Modifications in RNA structure may be tailored to allow specific genetic inhibition while avoiding a general response to dsRNA. Likewise, bases may be modified to block the activity of adenosine deaminase. The dsRNAs may be produced enzymatically or by partial/total organic synthesis, any modified ribonucleotide can be introduced by in vitro enzymatic or organic synthesis. Methods of chemically modifying RNA molecules can be adapted for modifying dsRNAs (see, e.g., Heidenreich et al. (1997) Nucleic Acids Res, 25:776-780; Wilson et al. (1994) J Mol Recog 7:89-98; Chen et al. (1995) Nucleic Acids Res 23:2661-2668; Hirschbein et al. (1997) Antisense Nucleic Acid Drug Dev 7:55-61). Merely to illustrate, the backbone of an dsRNA can be modified with phosphorothioates, phosphoramidate, phosphodithioates, chimeric methylphosphonate-phosphodiesters, peptide nucleic acids, 5-propynyl-pyrimidine containing oligomers or sugar modifications (e.g., 2'-substituted ribonucleosides, a-configuration). In certain cases, the dsRNAs of the disclosure lack 2'-hydroxy(2'-OH) containing nucleotides.

In a specific embodiment, at least one strand of the siRNA molecules has a 3' overhang from about 1 to about 6 nucleotides in length, though may be from 2 to 4 nucleotides in length. More preferably, the 3' overhangs are 1-3 nucleotides in length. In certain embodiments, one strand having a 3' overhang and the other strand being blunt-ended or also having an overhang. The length of the overhangs may be the same or different for each strand. In order to further enhance the stability of the siRNA, the 3' overhangs can be stabilized against degradation. In certain embodiments, the RNA is stabilized by including purine nucleotides, such as adenosine or guanosine nucleotides. Alternatively, substitution of pyrimidine nucleotides by modified analogues, e.g., substitution of uridine nucleotide 3' overhangs by 2'-deoxythyinidine is tolerated and does not affect the efficiency of RNAi. The absence of a 2' hydroxyl significantly enhances the nuclease resistance of the overhang in tissue culture medium and may be beneficial in vivo.

In another specific embodiment, the subject dsRNA can also be in the form of a long double-stranded RNA. For example, the dsRNA is at least 25, 50, 100, 200, 300 or 400 bases. In some cases, the dsRNA is 400-800 bases in length. Optionally, the dsRNAs are digested intracellularly, e.g., to produce siRNA sequences in the cell. However, use of long double-stranded RNAs in vivo is not always practical, presumably because of deleterious effects which may be caused by the sequence-independent dsRNA response. In such embodiments, the use of local delivery systems and/or agents which reduce the effects of interferon or PKR are preferred.

In a further specific embodiment, the dsRNA is in the form of a hairpin structure (named as hairpin RNA or short hairpin RNA). The hairpin RNAs can be synthesized exogenously or can be formed by transcribing from RNA polymerase III promoters in vivo. Examples of making and using such hairpin RNAs for gene silencing in mammalian cells are described in, for example, Paddison et al., Genes Dev, 2002, 16:948-58; McCaffrey et al., Nature, 2002, 418:38-9; McManus et al., RNA, 2002, 8:842-50; Yu et al., Proc Natl Acad Sci USA, 2002, 99:6047-52). Preferably, such hairpin RNAs are engineered in cells or in an animal to ensure continuous and stable suppression of a desired gene. It is known in the art that siRNAs can be produced by processing a hairpin RNA in the cell.

In certain embodiments, antisense oligonucleotides comprise modification with Locked Nucleic Acids (LNAs) in which the 2'-hydroxyl group is linked to the 4' carbon atom of the sugar ring thereby forming a 2'-C,4'-C-oxymethylene linkage thereby forming a bicyclic sugar moiety. The linkage is preferably a methylene (--CH₂--), group bridging the 2' oxygen atom and the 4' carbon atom wherein n is 1 or 2 (Singh et al., Chem. Commun., 1998, 4, 455-456). LNA and LNA analogs display very high duplex thermal stabilities with complementary DNA and RNA (Tm=+3 to +10 C), stability towards 3'-exonucleolytic degradation and good solubility properties. Potent and nontoxic antisense oligonucleotides containing LNAs have been described (Wahlestedt et al., Proc. Natl. Acad. Sci. U.S.A., 2000, 97, 5633-5638.)

The synthesis and preparation of the LNA monomers adenine, cytosine, guanine, 5-methylcytosine, thymine and uracil, along with their oligomerization, and nucleic acid recognition properties have been described (Koshkin et al., Tetrahedron, 1998, 54, 3607-3630). LNAs and preparation thereof are also described in WO 98/39352 and WO 99/14226.

In certain embodiments, an siRNA molecule of the invention comprises one or more (e.g., about 1, 2, 3, 4, 5, 6, 7, 8, 9, 10, or more) locked nucleic acid (LNA) nucleotides, for example, at the 5'-end, the 3'-end, both of the 5' and 3'-ends, or any combination thereof, of the siRNA molecule

PCT application WO 01/77350 describes an exemplary vector for bi-directional transcription of a transgene to yield both sense and antisense RNA transcripts of the same transgene in a eukaryotic cell. Accordingly, in certain embodiments, the present disclosure provides a recombinant vector having the following unique characteristics: it comprises a viral replicon having two overlapping transcription units arranged in an opposing orientation and flanking a transgene for a dsRNA of interest, wherein the two overlapping transcription units yield both sense and antisense RNA transcripts from the same transgene fragment in a host cell.

In another embodiment, one or more RTK inhibitors may be an enzymatic nucleic acid. By "enzymatic nucleic acid," it is meant a nucleic acid which has complementarity in a substrate binding region to a specified target gene, and also has an enzymatic activity which is active to specifically cleave a target nucleic acid. It is understood that the enzymatic nucleic acid is able to intermolecularly cleave a nucleic acid and thereby inactivate a target nucleic acid. These complementary regions allow sufficient hybridization of the enzymatic nucleic acid to the target nucleic acid and thus permit cleavage. One hundred percent complementarity (identity) is preferred, but complementarity as low as 50-75% can also be useful (see for example Werner and Uhlenbeck, 1995, Nucleic Acids Research, 23, 2092-2096; Hammann et al., 1999, Antisense and Nucleic Acid Drug Dev., 9, 25-31). The enzymatic nucleic acids can be modified at the base, sugar, and/or phosphate groups. As described herein, the term "enzymatic nucleic acid" is used interchangeably with phrases such as ribozymes, catalytic RNA, enzymatic RNA, catalytic DNA, aptazyme or aptamer-binding ribozyme, regulatable ribozyme, catalytic oligonucleotides, nucleozyme, DNAzyme, RNA enzyme, endoribonuclease, endonuclease, minizyme, leadzyme, oligozyme or DNA enzyme. All of these terminologies describe nucleic acids with enzymatic activity. The specific enzymatic nucleic acids described herein are not meant to be limiting and those skilled in the art will recognize that all that is important in an enzymatic nucleic acid is that it has a specific substrate binding site which is complementary to one or more of the target nucleic acid regions, and that it have nucleotide sequences within or surrounding that substrate binding site which imparts a nucleic acid cleaving and/or ligation activity to the molecule (Cech et al., U.S. Pat. No. 4,987,071; Cech et al., 1988, 260 JAMA 3030). In certain embodiments, an enzymatic nucleic acid is a ribozyme designed to catalytically cleave an mRNA transcripts to prevent translation of mRNA (see, e.g., PCT International Publication WO90/11364, published Oct. 4, 1990; Sarver et al., 1990, Science 247:1222-1225; and U.S. Pat. No. 5,093,246). In another embodiment, an enzymatic nucleic acid is a DNA enzyme. Methods of making and administering DNA enzymes can be found, for example, in U.S. Pat. No. 6,110,462.

One or more RTK inhibitors may be administered by using a virus. The virus may encodes an inhibitor of RTK. The virus may be a virus that uses an RTK as a co-receptor. The virus may be an adeno-associated virus (AAV). AAV2 uses FGFR1 and MET as its co-receptors and AAV5 uses PDGFRa & PDGFRb. One or more coat proteins on the AAV may bind to an RTK. AAVs can be used to deliver hairpin RNA directed against the RTKs or RTK ligands. The AAV may further deliver a cytotoxic agent or a tumor suppressor to the cell.

Adeno-associated virus is a naturally occurring defective virus that requires another virus, such as an adenovirus or a herpes virus, as a helper virus for efficient replication and a productive life cycle. (For a review see Muzyczka et al. Curr. Topics in Micro. and Immunol. (1992) 158:97-129). It is also one of the few viruses that may integrate its DNA into non-dividing cells, and exhibits a high frequency of stable integration (see for example Flotte et al. (1992) Am. J. Respir. Cell. Mol. Biol. 7:349-356; Samulski et al. (1989) J. Virol. 63:3822-3828; and McLaughlin et al. (1989) J. Virol. 62:1963-1973). Vectors containing as little as 300 base pairs of AAV can be packaged and can integrate. Space for exogenous DNA is limited to about 4.5 kb. An AAV vector such as that described in Tratschin et al. (1985) Mol. Cell. Biol. 5:3251-3260 can be used to introduce DNA into cells. A variety of nucleic acids have been introduced into different cell types using AAV vectors (see for example Hermonat et al. (1984) Proc. Natl. Acad. Sci. USA 81:6466-6470; Tratschin et al. (1985) Mol. Cell. Biol. 4:2072-2081; Wondisford et al. (1988) Mol. Endocrinol. 2:32-39; Tratschin et al. (1984) J. Virol. 51:611-619; and Flotte et al. (1993) J. Biol. Chem. 268:3781-3790).

### Protein therapeutics

In certain embodiments, one or more RTK inhibitors are a protein display scaffold as described in Hosse, R.J., et al., Protein Science, 15:14-27 (2006). In certain embodiments, the protein display scaffold is a fibronectin based "addressable" therapeutic binding molecule. The fibronectin domain III (FnIII) loops comprise regions that may be subjected to random mutation and directed evolutionary schemes of iterative rounds of target binding, selection, and further mutation in order to develop useful therapeutic tools. An exemplary embodiment of fibronectin based protein therapeutics are Adnectins as described in PCT publications WO00/34784, WO01/64942, and WO02/032925. In certain embodiments, one or more RTK inhibitors is an RTK ligand binding Adnectin.

In another embodiment, one or more of the RTK inhibitors comprises an antibody or antigen binding fragment that binds to an RTK or to an RTK ligand protein. The term "antibody" as used herein is intended to include fragments thereof which are also specifically reactive with a polypeptide of the invention. Antibodies can be fragmented using conventional techniques and the fragments screened for utility in the same manner as is suitable for whole antibodies. For example, F(ab')₂ fragments can be generated by treating antibody with pepsin. The resulting F(ab')₂ fragment can be treated to reduce disulfide bridges to produce Fab' fragments. The antibody of the present invention is further intended to include bispecific and chimeric molecules, as well as single chain (scFv) antibodies. Also included are trimeric antibodies, humanized antibodies, human antibodies, and single chain antibodies. All of these modified forms of antibodies as well as fragments of antibodies are intended to be included in the term "antibody".

Antibodies may be elicited by methods known in the art. For example, a mammal such as a mouse, a hamster or rabbit may be immunized with an immunogenic form of an RTK or RTK ligand protein (e.g., an antigenic fragment which is capable of eliciting an antibody response). Alternatively, immunization may occur by using a nucleic acid that in vivo expresses an RTK or RTK ligand protein giving rise to the immunogenic response observed. Techniques for conferring immunogenicity on a protein or peptide include conjugation to carriers or other techniques well known in the art. For instance, a peptidyl portion of a polypeptide of the invention may be administered in the presence of adjuvant. The progress of immunization may be monitored by detection of antibody titers in plasma or serum. Standard ELISA or other immunoassays may be used with the immunogen as antigen to assess the levels of antibodies.

Following immunization, antisera reactive with an RTK or RTK ligand may be obtained and, if desired, polyclonal antibodies isolated from the serum. To produce monoclonal antibodies, antibody producing cells (lymphocytes) may be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), as the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with the polypeptides of the invention and the monoclonal antibodies isolated.

Antibodies directed against polypeptide antigens can further comprise humanized antibodies or human antibodies. These, antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al. (1986) Nature, 321:522-525;Riechmann et al. (1988) Nature, 332:323-327; Verhoeyen et al. (1988) Science, 239:1534-1536), by substituting rodent CDRs or CDR sequences for the corresponding sequences of a human antibody. (See also U.S. Pat. No. 5,225,539.)

Antibody fragments that contain the idiotypes to a the RTK or RTK ligand may be produced by techniques known in the art including, but not limited to: (i) an F(ab')₂ fragment produced by pepsin digestion of an antibody molecule; (ii) an Fab fragment generated by reducing the disulfide bridges of an F(ab')₂ fragment; (iii) an Fab fragment generated by the treatment of the antibody molecule with papain and a reducing agent and (iv) Fv fragments.

In certain embodiments, one or more RTK inhibitors is an RTK ligand-binding polypeptide that reduces binding between RTK and its corresponding ligand. In certain embodiments, the RTK ligand binding polypeptide is a soluble RTK polypeptide, in particular a polypeptide comprising the extracellular domain of an RTK. The term "soluble RTK polypeptide," as used herein, includes any naturally occurring extracellular domain of an RTK protein as well as any variants thereof (including mutants, fragments and peptidomimetic forms) that retain ligand binding.

In certain embodiments, the RTK ligand-binding polypeptides include peptidomimetics. As used herein, the term "peptidomimetic" includes chemically modified peptides and peptide-like molecules that contain non-naturally occurring amino acids, peptoids, and the like. Peptidomimetics provide various advantages over a peptide, including enhanced stability when administered to a subject. Methods for identifying a peptidomimetic are well known in the art and include the screening of databases that contain libraries of potential peptidomimetics. For example, the Cambridge Structural Database contains a collection of greater than 300,000 compounds that have known crystal structures (Allen et al., Acta Crystallogr. Section B, 35:2331 (1979)). Where no crystal structure of a target molecule is available, a structure can be generated using, for example, the program CONCORD (Rusinko et al., J. Chem. Inf. Comput. Sci. 29:251 (1989)). Another database, the Available Chemicals Directory (Molecular Design Limited, Informations Systems; San Leandro Calif.), contains about 100,000 compounds that are commercially available and also can be searched to identify potential peptidomimetics of the RTK ligand-binding polypeptides.

In certain aspects, functional variants or modified forms of the RTK ligand-binding polypeptides include fusion proteins having at least a portion of the RTK polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt-conjugated resins are used. Many of such matrices are available in "kit" form, such as the Pharmacia GST purification system and the QIAexpress^{™}. system (Qiagen) useful with (HIS₆) fusion partners. As another example, a fusion domain may be selected so as to facilitate detection of the RTK polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus haemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins therefrom. The liberated proteins can then be isolated from the fusion domain by subsequent chromatographic separation. In certain preferred embodiments, an RTK polypeptide is fused with a domain that stabilizes the RTK polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (e.g., dimerizing, tetramerizing) domains and functional domains (that confer an additional biological function, such as further stimulation of muscle growth).

In certain embodiments, an RTK inhibitor is a soluble fusion protein of the ligand binding domain of VEGFR1 bound to the human Ig constant region as described in Holash et al., PNAS 99(17)11393-11398 (2002).

In certain embodiments, one or more RTK inhibitors are RTK ligands modified to retain RTK binding while losing their endogenous activating activity. In certain embodiments, one or more RTK inhibitors is a modified hepatocyte growth factor (HGF) that competes with endogenous HGF for MET binding without activating the receptor (Date et al., FEBS Letters 420:1-6, (1997)).

### Small molecule inhibitors

In certain embodiments, the RTK inhibitor is a small molecule. In certain embodiments, the small molecule inhibitor is less than 100, 80, 50, 40, 30, 20, 10, 5, 4, 2, or 1 kD.

Examples of VEGFR inhibitors include, but are not limited to, Avastin^{™} (bevacizumab), CP-547,632, axitinib (AG13736), AEE788, AZD-2171, VEGF trap, Macugen, nM862, Pazopanib (GW786034), ABT-869 and angiozyme. Additional VEGF inhibitors include CP-547,632 (Pfizer Inc., NY, USA), AG13736 (Pfizer Inc.), ZD-6474 (AstraZeneca), AEE788 (Novartis), VEGF Trap (Regeneron,/Aventis), Vatalanib (also known as RTK-787, ZK-222584: Novartis & Schering AG), Macugen (pegaptanib octasodium, NX-1838, EYE-001, Pfizer Inc./Gilead/Eyetech), IM862 (Cytran Inc. of Kirkland, Wash., USA); and angiozyme, a synthetic ribozyme from Ribozyme (Boulder, Colo.) and Chiron (Emeryville, Calif.) and combinations thereof. VEGF inhibitors useful in the practice of the present invention are disclosed in U.S. Pat. Nos. 6,534,524 and 6,235,764. Additional VEGF inhibitors are described in, for example in WO 99/24440 (published May 20,1999), PCT International Application PCT/IB99/00797 (filed May 3, 1999), in WO 95/21613 (published Aug. 17, 1995), WO 99/61422 (published Dec. 2, 1999), U.S. Pat. No. 6,534,524 (discloses AG13736), U.S. Pat. No. 5,834,504 (issued Nov. 10, 1998), WO 98/50356 (published Nov. 12, 1998), U.S. Pat. No. 5,883,113 (issued Mar. 16, 1999), U.S. Pat. No. 5,886,020 (issued Mar. 23, 1999), U.S. Pat. No. 5,792,783 (issued Aug. 11, 1998), U.S. Pat. No. 6,653,308 (issued Nov. 25, 2003), WO 99/10349 (published Mar. 4, 1999), WO 97/32856 (published Sep. 12, 1997), WO 97/22596 (published Jun. 26, 1997), WO 98/54093 (published Dec. 3, 1998), WO 98/02438 (published Jan. 22, 1998), WO 99/16755 (published Apr. 8, 1999), and WO 98/02437 (published Jan. 22, 1998).

Examples of MEK inhibitors include, but are not limited to, PD325901, ARRY-142886, ARRY-438162 and PD98059.

EGFR inhibitors include, but are not limited to, Iressa^{™} (gefitinib, AstraZeneca), Tarceva^{™} (erlotinib or OSI-774, OSI Pharmaceuticals Inc.), Erbitux^{™} (cetuximab, Imclone Pharmaceuticals, Inc.), EMD-7200 (Merck AG), ABX-EGF (Amgen Inc. and Abgenix Inc.), HR3 (Cuban Government), IgA antibodies (University of Erlangen-Nuremberg), TP-38 (IVAX), EGFR fusion protein, EGF-vaccine, anti-EGFr immunoliposomes (Hermes Biosciences Inc.) and combinations thereof.

Examples of ErbB2 receptor inhibitors include, but are not limited to, CP-724-714, CI-1033 (canertinib), Herceptin^{™} (trastuzumab), Omnitarg^{™} (2C4, petuzumab), TAK-165, GW-572016 (Ionafarnib), GW-282974, EKB-569, PI-166, dHER2 (HER2 Vaccine), APC8024 (HER2 Vaccine), anti-HER/2neu bispecific antibody, B7.her2IgG3, AS HER2 trifunctional bispecfic antibodies, mAB AR-209 and mAB 2B-1. Additional erbB2 inhibitors include those described in WO 98/02434 (published Jan. 22, 1998), WO 99/35146 (published Jul. 15, 1999), WO 99/35132 (published Jul. 15, 1999), WO 98/02437 (published Jan. 22, 1998), WO 97/13760 (published Apr. 17, 1997), WO 95/19970 (published Jul. 27, 1995), U.S. Pat. No. 5,587,458 (issued Dec. 24, 1996), and U.S. Pat. No. 5,877,305 (issued Mar. 2, 1999). ErbB2 receptor inhibitors useful in the present invention are also described in U.S. Pat. Nos. 6,465,449, and 6,284,764, and International Application No. WO 2001/98277.

Specific IGF1R antibodies that can be used in the present invention include those described in International Patent Application No. WO 2002/053596.

Examples of PDGFR inhibitors include, but are not limited to, SU9518, CP-673,451 and CP-868596.

Examples of AXL inhibitors include, but are not limited to, SGI-AXL-277 (SuperGen) as well as inhibitors disclosed in U.S. Pat. Pub. 20050186571.

Examples of FGFR inhibitors include, but are not limited to, PD17034, PD166866, and SU5402.

Examples of TIE2 inhibitors include, but are not limited to, those described in Kissau, L. et. al., J Med Chem, 46:2917-2931 (2003).

RTK inhibitors also encompass inhibitors with multiple targets. Pan ERBB receptor inhibitors include, but are not limited to, GW572016, CI-1033, EKB-569, and Omnitarg. MP371 (SuperGen) is an inhibitor of c-Kit, Ret, PDGFR, and Lck, as well as the non-receptor tyrosine kinase c-src. MP470 (SuperGen) is an inhibitor of c-Kit, PDGFR, and c-Met. Imatinib (Gleevec^{™}) is an inhibitor of c-kit, PDGFR, and ROR, as well as the non-receptor tyrosine kinase bcl/abl. Lapatinib (Tykerb^{™}) is an epidermal growth factor receptor (EGFR) and ERBB2 (Her2/neu) dual tyrosine kinase inhibitor. Inhibitors of PDGFR and VEGFR include, but are not limited to, Nexavar^{™} (sorafenib, BAY43-9006), Sutent^{™} (sunitinib, SU11248), and ABT-869. Zactima^{™} (vandetanib, ZD-6474) is an inhibitor of VEGFR and EGFR.

### E. Pharmaceutical Compositions

The methods described herein may involve administration of one or more RTK inhibitors to a subject. The RTK inhibitors may be formulated in a conventional manner using one or more physiologically acceptable carriers or excipients. For example, RTK inhibitors and their physiologically acceptable salts and solvates may be formulated for administration by, for example, injection (e.g. SubQ, IM, IP), inhalation or insufflation (either through the mouth or the nose) or oral, buccal, sublingual, transdermal, nasal, parenteral or rectal administration. A RTK inhibitor may be administered locally, at the site where the target cells are present, i.e., in a specific tissue, organ, or fluid (e.g., blood, cerebrospinal fluid, tumor mass, etc.).

RTK inhibitors can be formulated for a variety of modes of administration, including systemic and topical or localized administration. Techniques and formulations generally may be found in Remington's Pharmaceutical Sciences, Meade Publishing Co., Easton, PA. For parenteral administration, injection is preferred, including intramuscular, intravenous, intraperitoneal, and subcutaneous. For injection, the compounds can be formulated in liquid solutions, preferably in physiologically compatible buffers such as Hank's solution or Ringer's solution. In addition, the compounds may be formulated in solid form and redissolved or suspended immediately prior to use. Lyophilized forms are also included.

For oral administration, the pharmaceutical compositions may take the form of, for example, tablets, lozanges, or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavouring, coloring and sweetening agents as appropriate. Preparations for oral administration may be suitably formulated to give controlled release of the active compound.

For administration by inhalation (e.g., pulmonary delivery), RTK inhibitors may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin, for use in an inhaler or insufflator may be formulated containing a powder mix of the compound and a suitable powder base such as lactose or starch.

RTK inhibitors may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

In addition, RTK inhibitors may also be formulated as a depot preparation. Such long acting formulations may be administered by implantation (for example subcutaneously or intramuscularly) or by intramuscular injection. Thus, for example, RTK inhibitors may be formulated with suitable polymeric or hydrophobic materials (for example as an emulsion in an acceptable oil) or ion exchange resins, or as sparingly soluble derivatives, for example, as a sparingly soluble salt. Controlled release formula also includes patches.

The compounds described herein may be formulated for delivery to the central nervous system (CNS) (reviewed in Begley, Pharmacology & Therapeutics 104: 29-45 (2004)). Conventional approaches for drug delivery to the CNS include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide).

An RTK inhibitor may be incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like.

Pharmaceutical compositions (including cosmetic preparations) may comprise from about 0.00001 to 100% such as from 0.001 to 10% or from 0.1% to 5% by weight of one or more RTK inhibitors described herein. In certain topical formulations, the active agent is present in an amount in the range of approximately 0.25 wt. % to 75 wt. % of the formulation, preferably in the range of approximately 0.25 wt. % to 30 wt. % of the formulation, more preferably in the range of approximately 0.5 wt. % to 15 wt. % of the formulation, and most preferably in the range of approximately 1.0 wt. % to 10 wt. % of the formulation.

Conditions of the eye can be treated or prevented by, e.g., systemic, topical, intraocular injection of a RTK inhibitor, or by insertion of a sustained release device that releases a RTK inhibitor. A RTK inhibitor may be delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humour, cornea, iris/ciliary, lens, choroid/retina and sclera. The pharmaceutically-acceptable ophthalmic vehicle may, for example, be an ointment, vegetable oil or an encapsulating material. Alternatively, the compounds may be injected directly into the vitreous and aqueous humour. In a further alternative, the compounds may be administered systemically, such as by intravenous infusion or injection, for treatment of the eye.

RTK inhibitors described herein may be stored in oxygen free environment according to methods in the art.

Toxicity and therapeutic efficacy of RTK inhibitors can be determined by standard pharmaceutical procedures in cell cultures or experimental animals. The LDso is the dose lethal to 50% of the population. The ED₅₀ is the dose therapeutically effective in 50% of the population. The dose ratio between toxic and therapeutic effects (LD₅₀/ED₅₀) is the therapeutic index. RTK inhibitors that exhibit large therapeutic indexes are preferred. While RTK inhibitors that exhibit toxic side effects may be used, care should be taken to design a delivery system that targets such compounds to the site of affected tissue in order to minimize potential damage to uninfected cells and, thereby, reduce side effects.

The data obtained from cell culture assays and animal studies can be used in formulating a range of dosage for use in humans. The dosage of such compounds may lie within a range of circulating concentrations that include the ED₅₀ with little or no toxicity. The dosage may vary within this range depending upon the dosage form employed and the route of administration utilized. For any compound, the therapeutically effective dose can be estimated initially from cell culture assays. A dose may be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

Methods for delivering nucleic acid compounds are known in the art (see, e.g., Akhtar et al., 1992, Trends Cell Bio., 2, 139; and Delivery Strategies for Antisense Oligonucleotide Therapeutics, ed. Akhtar, 1995; Sullivan et al., PCT Publication No. WO 94/02595). These protocols can be utilized for the delivery of virtually any nucleic acid compound. Nucleic acid compounds can be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. Alternatively, the nucleic acid/vehicle combination is locally delivered by direct injection or by use of an infusion pump. Other routes of delivery include, but are not limited to, oral (tablet or pill form) and/or intrathecal delivery (Gold, 1997, Neuroscience, 76, 1153-1158). Other approaches include the use of various transport and carrier systems, for example though the use of conjugates and biodegradable polymers. For a comprehensive review on drug delivery strategies, see Ho et al., 1999, Curr. Opin. Mol. Ther., 1, 336-343 and Jain, Drug Delivery Systems: Technologies and Commercial Opportunities, Decision Resources, 1998 and Groothuis et al., 1997, J. NeuroVirol., 3, 387-400. More detailed descriptions of nucleic acid delivery and administration are provided in Sullivan et al., supra, Draper et al., PCT WO93/23569, Beigelman et al., PCT Publication No. WO99/05094, and Klimuk et al., PCT Publication No. WO99/04819.

Antisense nucleotides, such as siRNA, may be delivered to cancer cells using a variety of methods. Cell-penetrating peptides (CPPs) having the ability to convey linked "cargo" molecules into the cytosol may be used (see Juliano, Ann N Y Acad Sci. 2006 Oct;1082:18-26). In certain embodiments, an atelocollagen-mediated oligonucleotide delivery system is used (Hanai et la. Ann N Y Acad Sci. 2006 Oct;1082:9-17). An LPD formulation (liposome-polycation-DNA complex) may be used to deliver siRNA to tumor cells. (Li et al. Ann N Y Acad Sci. 2006 Oct;1082:1-8). Complexation of siRNAs with the polyethylenimine (PEI) may also be sued to deliver siRNA into cells (Aigner, J Biomed Biotechnol. 2006;2006(4):71659). siRNA may also be complexed with chitosan-coated polyisohexylcyanoacrylate (PIHCA) nanoparticles for invivo delivery. (Pille et al., Hum Gene Ther. 2006 Oct;17(10):1019-26)

### EXEMPLIFICATION

The invention now being generally described, it will be more readily understood by reference to the following examples which are included merely for purposes of illustration of certain aspects and embodiments of the present invention, and are not intended to limit the invention in any way.

### Example 1: Identification of PI3-kinase bound phosphoproteins

To investigate phophoproteins that mediate PI3K signaling in human glioma cell lines, we immunoprecipitated the p85α subunit of PI3K using an anti-p85α antibody. Specifically, we immunoprecipitated whole cell extracts from 14 different glioma cell lines with an antibody to the p85α subunit of PI3-kinase and separated eluted bound proteins on Tris-Acetate gradient gels. We then probed immunoblots with anti-phosphotyrosine (P-Tyr), revealing the presence of multiple p85α-associated phosphoproteins as compared to whole cell extract immunoprecipitated with IgG, or with immunoprecipitations using an immortalized normal human astrocyte control (NHA). As shown in Figure 1A, multiple tyrosine-phosphorylated proteins were found to be in the PI3K complex.

In order to determine the identity of the eluted phosphoproteins, we separated whole cell extracts on Bis-Tris gradient gels and probed immunoblots with antibodies against EGFR, ERBB2, ERBB3, PDGFRA, and MET. We demonstrate that multiple RTKs, such as EGFR, ErbB2, ErbB3, PDGFR α, and MET, are simultaneously expressed in the majority of the cell lines (Figure 1B).

In order to further confirm the identity of the p85α-interacting phosphoproteins, we compared the molecular weights of the tyrosine-phosphorylated bands from Figure 1A with a list of possible RTKs and adaptors by Scansite, found on the world wide web at scansite.mit.edu (J. C. Obenauer, L. C. Cantley, M. B. Yaffe, Nucleic Acids Res 31, 3635 (2003)). We verified possible matches by immunoprecipitating with candidate-specific antibodies and immunoblotting for endogenous interactions with p85α. In this manner, we determined that the 185 kD phosphotyrosine band in 5 of the 14 cell lines tested belonged to the EGFR family member, ERBB3, known to mediate binding of EGFR and ERBB2 to PI3K (N. E. Hynes, H. A. Lane, Nat Rev Cancer 5, 341 (2005)). Figure 5 shows the results of immunoprecipitations we performed with antibodies to p85α or ERBB3. We immunoblotted with antibodies to ERBB3 and P-Tyr and confirmed that in 5 of the cell lines, p85α was associated with ERBB3 (3 of the cell lines are depicted in Figure 5).

To investigate the state of PI3K activity in the various glioma cell lines, we further probed the whole cell extract immunoblots with anti-PTEN and with phosphorylation specific antibodies agains AKT and MAPK. As shown in Figure 1B, AKT phosphorylation is increased in every cell line relative to (NHA) irrespective of PTEN status, indicating enhanced PI 3-kinase activity in every cell line examined.

### Example 2: Identification of GAB1 bound phosphoproteins

In order to investigate activated RTKs involved in PI3K signaling, we immunoprecipitated GAB1, a docking protein that binds activated RTKs directly or through association with Grb2 (H. Gu, B. G. Neel, Trends Cell Biol 13, 122 (2003)). Specifically, we performed immunoprecipitations of whole cell extracts from 14 glioma cell lines with an antibody to GAB1, followed by immunoblotting with antibodies to ERBB3 and phosphotyrosine (P-Tyr, Upstate). We demonstrate in Figure 6A that in 7 different GBM cell lines, GAB1 was highly tyrosine-phosphorylated and co-immunoprecipitated with a 140-kDa phosphorylated protein that we demonstrated to be activated MET. Importantly, all 7 of these cell lines also harbor robust activation ofp-EGFR (Table 1).

### Example 3: Identification of activated RTKs

In order to more broadly define the compendium of co-activated RTKs, we utilized an RTK antibody array that enables simultaneous assessment of the phosphorylation status of 45 RTKs. Specifically, we incubated whole cell extracts from the glioma cell lines on RTK antibody arrays and determined phosphorylation status by subsequent incubation with anti-phosphotyrosine-HRP. Each RTK is spotted in duplicate on the RTK antibody arrays - the pairs of dots in each comer are positive controls. Each positive RTK dot pair is denoted by a red numeral with the corresponding RTKs listed below the arrays. Figure 2A demonstrates the results of an array experiment from four different cell lines. These arrays are representative of various RTK co-expression patterns in the 20 total glioma cell lines examined. We summarized the results of the antibody array experiments in Table 1 (Figure 9). We found that a minimum of 3 activated RTKs could be detected in 19 of 20 glioma cell lines (Figure 2A and Table 1 shown in Figure 9), including the known glioma-relevant RTKs EGFR and PDGFRα as well as MET and ERBB3, consistent with the above p85α and GAB1 IP analyses. We also observed by immunoflorescence using activation specific antibodies to EGFR, PDGFR, and MET, that each of these RTKs were activated in virtually 100% of the cells indicating that the observed RTK co-activation pattern is not the result of single RTK activation in heterogeneous cell subpopulations (data not shown).

We repeated the antibody array experiments using glioma cell lines that had been grown for 48 hours in 10% serum (log) or in 0.05% serum (i.e., serum starved). We observed that most RTKs activated in GBM cells remained phosphorylated under serum deprivation (Figure 2B). We also utilized RTK antibody arrays to compare RTK activation in whole cell extracts from xenograft tumors derived from the glioma cell lines SF767 or LN340 or from the corresponding *in vitro* cultured cells (Figure 2C). We demonstrate in Figures 2B and 2C that these RTK activation patterns are derived from intrinsic (epi)genetic aberrations rather than ligands in serum-containing culture media. Indeed, some GBM cell lines and specimens show genomic gains at multiple RTK loci (CB, unpublished data).

In order to determine if the co-activation of multiple RTKs was a unique feature of glioma cells, we performed antibody arrays in lung carcinoma and pancreatic adenocarcinoma cell lines (Figure 2D; and Table 5 shown in Figure 13) as well as colorectal cancer cell lines and primary tumors (Figure 4H, antibody array results summarized in Table 3 shown in Figure 11; and Table 4 shown in Figure 12). We demonstrate that similar patterns of RTK co-activation can be seen in other solid tumor lineages, indicating that co-activation of multiple RTKs may be a common feature in tumors.

### Example 3: RTKs are able to functionally replace each other

To address the potential treatment implications of RTK co-activation, we utilized the established U87MG model system with constitutive expression of wild-type EGFR (wt EGFR), EGFRvIII (EGFR*), or a kinase-dead mutant of EGFRvIII (EGFR*-KD) at levels comparable to those observed in primary GBM tumors (R. Nishikawa et al., Proc Natl Acad Sci U S A 91, 7727 (1994)). EGFRvIII lacks amino acids 6-273 in the extracellular domain and is constitutively active independent of ligand binding.

We immunoprecipitated U87MG parental cells or cells constitutively expressing wt EGFR, the activating vIII deletion mutant (EGFR*) or the vIII mutant with an inactivating mutation in its kinase domain (EGFR*-KD) with an antibody to GAB1 and probed the immunoblot with antibodies against MET, p85α, GAB1, and heavy chain (hc, to demonstrate equal immunoprecipitation efficiency) as shown in Figure 3A, left panel. We also immunoblotted whole cell extract (WCE) from the same cells with antibodies against EGFR, MET, actin, and phosphorylated forms of AKT, MAPK, and S6.

We demonstrate that whereas MET is phosphorylated and bound to GAB1 in parental U87MG cells (Figure 6A), when wt EGFR and EGFR* were expressed in these cells, activated MET was significantly displaced by EGFR in the GAB1/PI3K complex (Figure 3A). The constituency of this complex required the catalytic activity of EGFR, since EGFR*-KD was significantly less capable of displacing MET than its catalytically-active counterpart (Figure 3A, lane 4). The fact that EGFR*-KD was expressed at similar levels as both wt EGFR and EGFR* makes it unlikely that this displacement is simply a consequence of ectopic expression. Importantly, this apparent "swapping" of RTKs within the PI3K complex did not lead to an obvious alteration in downstream signaling (Figure 3A, right panel), indicating that MET and EGFR can indeed functionally substitute for one another in the PI3K complex to maintain signaling downstream, acting as multiple redundant but independent inputs to this signaling network. By extension, co-activated MET would be expected to render anti-EGFR inhibition ineffective in extinguishing downstream signaling by replacing activated EGFR in the PI3K complex, prompting speculation that tumors cells with co-activated EGFR and MET (or other RTKs) might be less sensitive to anti-EGFR inhibition.

### Example 4: Inhibition of multiple RTKs is necessary to abrogate PI 3-kinase/RTK complex formation and consequent downstream signaling & cell survival

We next examined the consequences of single and combination inhibition of EGFR and MET in the U87MG-EGFR* cells, employing p-AKT and p-S6 Ribosomal Protein as molecular surrogates of the efficacy of PI3K inhibition. We treated U87MG-EGFR* cells with 10 µM of Tarceva^{™}, the MET inhibitor SU11274 (Calbiochem), both, or vehicle, then we incubated whole cell extracts on RTK antibody arrays. We confirmed that administration of either an EGFR inhibitor, Tarceva^{™} (erlotinib), or a MET Inhibitor, SU11274, effectively blocked phosphorylation of their intended target RTKs in U87MG-EGFR* cells (Figure 3B; P-EGFR=1, P-MET=2).

We next treated U87MG-EGFR* cells with 10 µM each of the RTK inhibitors Tarceva^{™} (T), SU11274 (S), and/or Gleevec^{™} (G), then immunoprecipitated whole cell extracts with an antibody to GAB1, eluted, and immunoblotted with antibodies to p85 α or Gab1 (Figure 3C, top panel). Note the faster migration of GAB1 in RTK-inhibitor treated cells, consistent with a decrease in phosphorylation. We also immunoblotted whole cell extracts with antibodies against MAPK and phosphorylated versions of AKT, MAPK, and S6 (Figure 3C, bottom panel):

Consistent with our hypothesis, we observed that while treatment with either inhibitor alone had no discernable effect on PI3K association with GAB1 or downstream activation of AKT and S6, combined inhibition with both Tarceva^{™} and SU11274 resulted in the release of p85α from the RTK/GAB1 complex and a reduction in P-AKT and P-S6 (Figure 3C). Moreover, the migration of GAB1 was fastest in cells treated with combination therapies and intermediate in cells treated with single inhibitor, consistent with GAB1 being less phosphorylated upon inhibition of both EGFR and MET. To demonstrate specificity, we added treatment with PDGFR inhibitor Gleevec^{™} (imatinib) alone or in combination with Tarceva^{™} or SU11274 and found that, in U87MG-EGFR* cells with no detectable PDGFR activation (Figure 3B), Gleevec^{™} did not significantly effect PI3K activation (Figure 3C). In other words, RTK co-activation patterns predicted the effective combination of PI3K pathway inhibitors in this particular GBM cell line. However, it should be noted that addition of Gleevec^{™} to Tarceva^{™} and SU11274 combination did eliminate residual p-AKT activity (Figure 3C, compare lane 4 and lane 8), likely reflecting activities of Gleevec^{™} on other kinases which may be active in these cells (M. A. Fabian et al., Nat Biotechnol 23, 329 (2005)).

In order to investigate the biological response of inhibiting PI3K signaling, we treated U87MG-EGFR* cells for 72 hr with combinations of 5 µM Tarceva, 1 µM SU 11274, and 1 µM Gleevec, or with 10 µM ActinomycinD in 0.1% serum-containing medium, then stained with Annexin V-FITC & propidium iodide (PI). As shown in Figure 3D, significant apoptosis of U87-EGFR* cells was evident only in cells treated with both Tarceva^{™} and the MET inhibitor or with all three RTK inhibitors. This correlates well with a left-shift of the IC50 for Tarceva^{™} from 9.9µM to 3.9µM when combined with Gleevec^{™} and SU11274 (data not shown). The decrease in cell viability was mediated in part by inhibition of PI3K signaling, as transient transfection of either myristoylated AKT or p110α-CAAX increased cell viability in drug treated cells (Figure 3G, p<0.001).

We performed soft-agar colony formation assays by plating U87MG-EGFR* cells in 10% serum, 0.4% agarose containing growth medium with 10 µM of each RTK inhibitor. Colonies were counted after 18 days (Figure 3E) and representative images of U87MG-EGFR* soft agar colonies are shown in Figure 3F. We demonstrate that in a soft agar assay for anchorage independent growth, concurrent EGFR and MET inhibition dramatically reduced both the number and size of colonies formed, while single inhibitor treatment and combined PDGFR inhibition had only minor effects.

### Example 5: Blockade of PI3K pathway activity in PTEN mutants by inhibiting multiple RTKs

It has been reported that PTEN mutational status is a major determinant in the response of GBM to EGFR inhibition. However, we observed significant inhibition of P-AKT and P-S6 in PTEN mutant U87MG-EGFR* cells upon combination treatments (Figure 3C), suggesting that even in the context of PTEN deficiency, adequate signaling from upstream RTKs may still be required to maintain PI3K activation. In other words, inhibition of co-activated RTKs via combination therapy could prove effective in decreasing PI3K activity even in PTEN deficient cells.

In order to further investigate the effect of decreasing PI3K activity in PTEN mutant cells, we treated the PTEN mutant glioma cell lines LN382 (Figure 4C) and LNZ308 (Figure4D-E), as well as the PTEN wild-type gliomas cell lines LN18 (Figure 4A) and SF767 (Figure 4B) with RTK inhibitors singly and in combination in 0.1% serum-containing medium and immunoblotted against actin and the phophorylated forms of AKT and S6. The activated RTKs within these cells, as we determined from antibody arrays, are indicated beneath the blots. Using P-AKT and P-S6 as biomarkers, we consistently observed that signaling through the PI3K pathway was significantly more attenuated or in many cases completely abrogated with combination treatment compared to single agent treatment as predicted a priori from the RTK-antibody arrays, and this effect was independent of PTEN status.

We then correlated inhibition of PI3K activity with biological effects of various combinations of RTK inhibitor treatment regimens. We examined apoptosis and soft agar colony growth of LN382 (Figure 4C, which has activated EGFR, MET, PDGFRα, PDGFRβ and EPHA7 on antibody array profiling, Table 1 shown in Figure 9), and LNZ308 (Figure 4D-E, which has activated AXL, EGFR, EPHA2, EPHA7, FGFR3, PDGFRα, PDGFRβ, KIT and KDR, Table 1 shown in Figure 9) using Tarceva^{™}, SU11274 and Gleevec^{™}. Although LNZ308 does not show activated MET, we included the MET inhibitor SU11274 in these studies for two reasons. First, it is well recognized that many kinase inhibitors exhibit activities against multiple RTKs and other kinases in addition to their prime targets (M. A. Fabian et al., Nat Biotechnol 23, 329 (2005)). For example, SU11274 diminishes p-PDGFR in LNZ308 cells. Second, we are mindful of the limitation of detection sensitivity by current antibody array technology and that a particular RTK may not be represented or be activated at an undetectable but biologically-relevant level. As shown in Figure 4C-E, combination inhibition of RTKs in LN382 and LNZ308, both PTEN mutant GBM lines, effectively extinguished PI3K activity. Correspondingly, we demonstrate that colony-formation in both cell lines was partially inhibited by single and dual treatment with RTK inhibitors, but most profoundly impacted by combined treatment with all 3 inhibitors.

We performed cell death and/or cell viability assays with LN18, LN382 and LNZ308 glioma cells treated with 5 µM Tarceva^{™} (T), 2 µM SU11274 (S), and/or 2 µM Gleevec^{™} (G) or 10 µM Actinomycin D. Figures 4A and 4C bottom panel and Figure 4E show that SU11274 and Gleevec^{™} enhance Tarceva^{™}-mediated cell death. These results suggest that, even in cells with mutant PTEN, PI3K pathway activity can be blocked with combined inhibition of relevant upstream signaling inputs which translates into robust biological effects.

Given the potential of non-specific actions of pharmaceutical agents, RNAi against MET, EGFR, and PDGFR was used to verify that inhibition of specific pathways can enhance the anti-oncogenic activity of erlotinib and imatinib (Figure 7).

### Example 6: Multiple RTKs are activated in primary GBM specimens

To assess the clinical translational potential of the above *in vitro* experimental findings, we assayed RTK co-activation patterns in a collection of newly diagnosed untreated primary human GBM tumors. Protein extracts were harvested from snap-frozen, pathologically-verified primary GBM specimens and assayed for RTK activation status by antibody array profiling. Similar to the GBM cell lines, we observed multiple activated RTKs in each of the samples examined (Figure 4G and Table 2 shown in Figure 10). These included RTKs commonly associated with GBM, such as EGFR, PDGFRα and MET, as well as RTKs not previously linked to gliomagenesis such as RET, RON, and CSF1R. This profile of co-activated RTKs in primary tumor specimens, coupled with our experimental findings in tumor cell lines above, provides a rationale for testing up-front first-line combination therapy against multiple RTKs in cancer patients particularly those deficient for PTEN.

We also performed immunofluorescence staining with phospho-specific antibodies against multiple RTKs and observed co-expression of activated RTKs in individual dissociated cells from a primary GBM (Fig. 4I), providing further evidence of *in vivo* RTK co-activation.

### Materials and Methods

**Cell lines.** All glioma cell lines were provided by Drs. Webster Cavenee and Frank Furnari (Ludwig Institutue, UCSD) and the immortalized normal human astrocyte line (E6/E7/hTERT NHA) was provided by Dr. Russell Pieper (UCSF). Cells were propagated in Dulbecco's MEM supplemented with 10% heat-inactivated fetal bovine serum.

**Tyrosine kinase inhibitors.** Gleevec^{™} (Imatinib) and Tarceva^{™} (Erlotinib) were purified from patient-discarded tablets recovered at the Dana-Farber Cancer Institute. Tablets were crushed, dissolved in water, extracted in ethyl acetate. Crude compound was purified on silica gel in 10% methanol in DCM, dried and dissolved in dimethyl sulfoxide. Purity was determined by high-performance liquid chromatography (240 nm).

**Immunoprecipitations and immunoblots.** Cells were harvested in lysis buffer consisting of 20 mM Tris pH 7.4, 150 mM NaCl, 1% NP-40, 10% glycerol, 1 mM EGTA, 1 mM EDTA, 5 mM Sodium Pyrophosphate, 50 mM Sodium Fluoride, 10 mM β-glycerophosphate, 1 mM Sodium Vanadate, 0.5 mM DTT, 1 mM PMSF, 2 mM Imidazole, 1.15 mM Sodium Molybdate, 4 mM Sodium Tartrate Dihydrate, and 1x Protease Inhibitor Cocktail (Sigma). Following 30 min incubation in lysis buffer at 4°C, lysates were cleared by centrifugation at 10k 10 min 4°C, then protein concentrations were determined by BioRad DC Protein Assay. GAB1 and p85α complexes were immunoprecipitated with antibodies from Upstate (#06-579 and #06-496) and Protein A agarose (RepliGen) and eluted by boiling in 1x Laemmli IP buffer with 0.1 M DTT. Tyrosine-phosphorylated proteins were visualized by separation on NuPAGE 3-8% Tris-Acetate gels (Invitrogen), blotted onto nitrocellulose, blocked with 3% Immunoblot Blocking Reagent (Upstate), then incubated with anti-phosphotyrosine antibody (4G10, Upstate). Whole cell extracts were separated on NuPAGE 3-8% Tris-Acetate or 4-12% Bis-Tris gels (Invitrogen). The following antibodies were used for immunoblotting: EGFR (Santa Cruz #SC-03), ErbB2 (Cell Signaling #2242), ErbB3 (Lab Vision #MS-201), PDGFRα (Lab Vision #RB-9027), MET (Santa Cruz #SC-161), PTEN (Santa Cruz #SC-7974), P-AKT (Cell Signaling #9271), P-MAPK (Cell Signaling #9101), GAB1 (Upstate #06-579), P-S6 Ribosomal Protein (Cell Signaling #2215), actin (Sigma #A2066), MAPK (Cell Signaling #9102), tubulin (developed by Michael Klymkowsky and obtained from the Developmental Studies Hybridoma Bank, University of Iowa). Co-immunoprecipitated proteins were detected with either mouse or rabbit TrueBlot HRP conjugated secondaries (eBioscience).

**Antibody arrays.** Cells were harvested as for immunoprecipitations above. Snap-frozen tumors were thawed in immunoprecipitation lysis buffer, homogenized by hand 50x with disposable 1.5 mL pestles, incubated 30 min 4°C with inversion, homogenized 50x, then cleared by centrifugation at 10k 10min 4°C. Proteins were quantitated as above. RTK antibody arrays were purchased from R&D Systems (#ARY-001) and performed as recommended but with 2 mg protein lysate per array.

**Soft agar colony formation.** The soft-agar assay was performed on 6-well plates in duplicate. For each well, 5,000 cells were mixed thoroughly in cell growth medium containing 0.4% agarose (Cat.A9045, Sigma) and corresponding mixture of RTK inhibitors. Cells were then plated onto bottom layers prepared with 1% agarose in regular medium. Medium containing different combination of RTK inhibitors were added to each well every five days.

**Cell viability assays.** Cells were plated in 96-well plates at 2.0 x10⁴ cells/well in 100 µl of RPMI-1640 with 10 % FBS. After 24 hr incubation, the medium was replaced with fresh RPMI-1640 media containing 0.1% FBS. Cells growth was measured using CellTiter-Glo Luminescent Cell Viability Kit (Promega) determining the number of viable cells based on quantity of the ATP. Assay was run according the manufacturers protocol.

**Transient transfection viability assays.** U87MG-EGFR* cells were plated at 8000 cells/well in 96-well plates at a final volume of 100 µl in 10% FBS-containing medium. After 24 hr incubation, cells were transfected using FuGENE HD Transfection Reagent (Roche, Indianapolis, IN) using a 5:2 ratio of transfection reagent to DNA. 48 hrs later, the medium was changed to 0.05% FBS-containing medium with 10 µM of erlotinib, SU11274, and imatinib. Cell viability was determined as above after a 72-hr incubation in drug.

**RNAi.** Cells were transfected using HiPerFect (Qiagen, Valencia, CA) according to the manufacturer's protocol. Two siRNAs for each RTK (EGFR, MET, PDGFRα, and PDGFRβ (IDT, Coralville, IA, sequences can be provided upon request) were combined and each used at a final concentration of 10nM. Scrambled siRNA (IDT) was used to keep the total molarity of siRNA in each experiment constant. Cells were incubated 96 hrs prior to harvest for verification of knockdown by immunoblotting, or plated for soft agar colony formation 24 hrs after transfection.

**Tumor dissociation and immunofluorescent staining.** Approximately 8 mm³ chunks were cut from frozen tumors on dry ice and immediately placed into Bambanker (Wako, Richmond, VA) on ice, then resuspended with a wide-bore P-200 pipet tip and vortexing, followed by pipetting with a standard-bore P-200 tip. Single-cell suspensions were obtained by filtering over a 40 micron nylon filter then cytospun onto positively charged slides at 800 rpm for 3 min. Slides were immediately fixed for 15 min at RT in 4% paraformaldehyde in PBS+ with 5 mM Sodium Pyrophosphate, 50 mM Sodium Fluoride, 10 mM β-glycerophosphate, 1 mM Sodium Vanadate, 2 mM Imidazole, 1.15 mM Sodium Molybdate, 4 mM Sodium Tartrate Dihydrate, washed in PBS+, permeabilized 10 min RT in 0.2% Triton X-100/PBS+, blocked 30 min RT in Image-iT FX signal enhancer, followed by blocking for 30 min RT in 10% donkey serum/PBS+. Slides were incubated overnight at 4°C with the following antibodies: 1:400 goat-anti-phospho-EGFR (Santa Cruz, SC-1235), 1:400 rabbit-anti-phospho-PDGFRα (Santa Cruz, SC-12911), 1:50 AF647-mouse-anti-phospho-InsR/IGF1R (BD Biosciences, #558588), 1:100 rabbit-anti-phospho-CSF1R (Cell Signaling,#3155), 1:200 mouse-anti-nestin (Chemicon, MAB5326, Temecula, CA), and 1:1000 rabbit-anti-olig2 (K. Ligon). Slides were then stained for 1 hour RT with AF647-donkey-anti-mouse, AF555-donkey-anti-rabbit, and AF488-donkey-anti-goat secondary antibodies (Invitrogen) and 1:2000 10 mg/mL Hoechst 33342 (Invitrogen). Coverslips were mounted with ProLong Anti-Fade Gold (Invitrogen). Microscopic images were obtained with a Nikon Eclipse E800 using a 60x objective and a Roper Scientific (Duluth, GA) CCD camera. Images were captured with Metavue Software (Molecular Devices, Downingtown, PA), using 0.75s exposures for the green channel, 8.25s exposures for the red channel, and 10s exposures for the far red channel. Images were compiled and false-colored with Adobe Photoshop (San Jose, CA), using identical settings for each color.

## Claims

1. A method of designing a first-line therapeutic treatment for a subject afflicted with cancer, comprising:
i) determining the presence of 10 or more indicators of receptor tyrosine kinase (RTK) activation in a biological sample from the subject; and
ii) selecting a group of one or more RTK inhibitors, wherein the group can inhibit the activity of at least two RTKs that display one or more of the indicators.

2. The method of claim 1, further comprising:
iii) transmitting a descriptor of the group of one or more RTK inhibitors, thereby designing a therapeutic treatment for the subject.

3. The method of claim 1 or 2, wherein the presence of indicators of RTK activation is determined for at least 15 RTKs.

4. The method of any of claims 1-3, wherein the presence of indicators of RTK activation is determined for at least 20 RTKs.

5. The method of any of claims 1-4, wherein the group of one or more RTK inhibitors can inhibit the activity of at least three RTKs that display one or more of the indicators.

6. The method of claim 5, wherein the group of one or more RTK inhibitors can inhibit the activity of at least four RTKs that display one or more of the indicators.

7. The method of any of claims 1-6, wherein the presence of one or more indicators of RTK activation is determined using an array, real-time PCR, fluorescent in situ hybridization, fluorescence activated cell sorting (FACS), mass spectrometry, RT- PCR, nuclease protection assay, northern blot, nucleotide sequencing, immunohistochemistry or immunocytochemistry with phosphorylation state-specific or total-protein detection antibodies, or a combination thereof.

8. The method of claim 7, wherein the array is an anti-phospho-RTK antibody array.

9. The method of claim 8, wherein the array is an anti-RTK array and wherein an anti-phosphotyrosine antibody is used to detect RTK phosphorylation.

10. The method of claim 7, wherein the one or more indicators of RTK activation are protein levels of an RTK or an RTK ligand and wherein the protein levels are determined by antibody binding.

11. The method of any of claims 1-10, wherein at least one inhibitor is a small molecule or an anti-RTK antibody.

## Patentansprüche

1. Verfahren zur Entwicklung einer Erstlinien ("first line")-therapeutischen Behandlung eines Individuums, das von Krebs befallen ist, umfassend:
i) Bestimmen der Anwesenheit von 10 oder mehr Anzeichen einer Rezeptortyrosinkinase (RTK)-Aktivierung in einer biologischen Probe des Individuums; und
ii) Auswählen einer Gruppe eines oder mehrerer RTK-Inhibitoren, wobei die Gruppe die Aktivität von mindestens zwei RTKs inhibieren kann, die ein oder mehrere der Anzeichen anzeigen.

2. Verfahren nach Anspruch 1, das weiterhin umfasst:
iii) Übermitteln eines Deskriptors der Gruppe eines oder mehrerer RTK-Inhibitoren, wodurch eine therapeutische Behandlung des Individuums entwickelt wird.

3. Verfahren nach Anspruch 1 oder 2, wobei die Anwesenheit von Anzeichen von RTK-Aktivierung für mindestens 15 RTKs bestimmt wird.

4. Verfahren nach einem der Ansprüche 1 bis 3, wobei die Anwesenheit von Anzeichen von RTK-Aktivierung für mindestens 20 RTKs bestimmt wird.

5. Verfahren nach einem der Ansprüche 1 bis 4, wobei die Gruppe eines oder mehrerer RTK-Inhibitoren die Aktivität von mindestens drei RTKs, die ein oder mehrere der Anzeichen anzeigen, inhibieren kann.

6. Verfahren nach Anspruch 5, wobei die Gruppe eines oder mehrerer RTK-Inhibitoren die Aktivität von mindestens vier RTKs, die ein oder mehrere der Anzeichen anzeigen, inhibieren kann.

7. Verfahren nach einem der Ansprüche 1 bis 6, wobei die Anwesenheit eines oder mehrerer Anzeichen von RTK-Aktivierung mittels Verwendung eines Arrays, von Realtime-PCR, von fluoreszierender in-situ-Hybridisierung, von fluoreszenzaktivierter Zellsortierung ("fluorescence activated cell sorting") (FACS), von Massenspektrometrie, von RT-PCR, eines Nuklease-Protektions-Assays ("nuclease protection assay"), eines Northern Blots, einer Nukleotidsequenzierung, von Immunhistochemie oder Immuncytochemie mit phosphorylationszustandsspezifischen Antikörpern oder Gesamtprotein-Nachweisantikörpern, oder einer Kombination davon, bestimmt wird.

8. Verfahren nach Anspruch 7, wobei das Array ein Anti-Phospho-RTK-Antikörper-Array ist.

9. Verfahren nach Anspruch 8, wobei das Array ein Anti-RTK-Array ist und wobei ein Anti-Phosphotyrosin-Antikörper für den Nachweis von RTK-Phosphorylierung verwendet wird.

10. Verfahren nach Anspruch 7, wobei das eine oder die mehreren Anzeichen der RTK-Aktivierung Proteinlevel einer RTK oder eines RTK-Liganden sind und wobei die Proteinlevel durch Antikörperbindung bestimmt werden.

11. Verfahren nach einem der Ansprüche 1 bis 10, wobei mindestens ein Inhibitor eine niedermolekulare Verbindung oder ein Anti-RTK-Antikörper ist.

## Revendications

1. Méthode de conception d'un traitement thérapeutique de première intention pour un sujet souffrant d'un cancer, comprenant :
i) la détermination de la présence de 10 indicateurs ou plus de l'activation des récepteurs tyrosine kinases (RTK) dans un échantillon biologique provenant du sujet ; et
ii) la sélection d'un groupe d'un ou de plusieurs inhibiteurs de RTK, où le groupe peut inhiber l'activité d'au moins deux RTK qui présentent un ou plusieurs des indicateurs.

2. Méthode selon la revendication 1, comprenant en outre :
iii) la transmission d'un descripteur du groupe d'un ou de plusieurs inhibiteurs de RTK, concevant de cette façon un traitement thérapeutique pour le sujet.

3. Méthode selon la revendication 1 ou 2, dans laquelle la présence d'indicateurs de l'activation de RTK est déterminée pour au moins 15 RTKs.

4. Méthode selon l'une quelconque des revendications 1 à 3, dans laquelle la présence d'indicateurs de l'activation de RTK est déterminée pour au moins 20 RTKs.

5. Méthode selon l'une quelconque des revendications 1 à 4, dans laquelle le groupe d'un ou de plusieurs inhibiteurs de RTK peut inhiber l'activité d'au moins trois RTKs qui présentent un ou plusieurs des indicateurs.

6. Méthode selon la revendication 5, dans laquelle le groupe d'un ou de plusieurs inhibiteurs de RTK peut inhiber l'activité d'au moins quatre RTKs qui présentent un ou plusieurs des indicateurs.

7. Méthode selon l'une quelconque des revendications 1 à 6, dans laquelle la présence d'un ou de plusieurs indicateurs de l'activation de RTK est déterminée en utilisant une puce, une PCR en temps réel, une hybridation fluorescente *in situ,* le tri des cellules activées par fluorescence (FACS), la spectrométrie de masse, la RT-PCR, le test de protection à la nucléase, l'analyse Northern blot, le séquençage des nucléotides, l'immunohistochimie ou l'immunocytochimie avec des anticorps spécifiques de l'état de phosphorylation ou de détection des protéines totales, ou l'une de leurs combinaisons.

8. Méthode selon la revendication 7, dans laquelle la puce est une puce d'anticorps anti-pho spho-RTK.

9. Méthode selon la revendication 8, dans laquelle la puce est une puce anti-RTK et où un anticorps anti-phosphotyrosine est utilisé pour détecter la phosphorylation des RTK.

10. Méthode selon la revendication 7, dans laquelle les un ou plusieurs indicateurs de l'activation de RTK sont des taux protéiques d'une RTK ou d'un ligand de RTK et où les taux protéiques sont déterminés par liaison d'anticorps.

11. Méthode selon l'une quelconque des revendications 1 à 10, dans laquelle au moins un inhibiteur est une petite molécule ou un anticorps anti-RTK.
